(19)

Europäisches
Patentamt

European
Patent Office

Office européen
des brevets

(11) **EP 4 752 228 A2**

(12) **EUROPEAN PATENT APPLICATION**

(43) Date of publication:
**03.06.2026 Bulletin 2026/23**

(21) Application number: **26157663.1**

(22) Date of filing: **15.04.2022**

(51) International Patent Classification (IPC):
***C12N 15/86*** (2006.01)

(52) Cooperative Patent Classification (CPC):
**C12N 9/22; A61P 7/00; C07K 14/4705;**
**C12N 15/63; C12N 15/86;** A61K 38/00;
C07K 14/805; C12N 2740/16043

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **15.04.2021 US 202163175467 P**

(62) Document number(s) of the earlier application(s) in
accordance with Art. 76 EPC:
**22789036.5 / 4 323 515**

(71) Applicants:
- **Altius Institute For Biomedical Sciences**
  **Seattle, WA 98121 (US)**
- **Cherone, Jennifer M.**
  **Seattle, WA 98121 (US)**
- **Funnell, Alister PW**
  **Seattle, WA 98121 (US)**
- **Stamatoyannopoulos, John A.**
  **Seattle, WA 98121 (US)**
- **Haugen, Eric David**
  **Seattle, WA 98121 (US)**

(72) Inventors:
- **Cherone, Jennifer M.**
  **Seattle, WA 98121 (US)**

- **Funnell, Alister PW**
  **Seattle, WA 98121 (US)**
- **Stamatoyannopoulos, John A.**
  **Seattle, WA 98121 (US)**
- **Haugen, Eric David**
  **Seattle, WA 98121 (US)**

(74) Representative: **Boult Wade Tennant LLP**
**Salisbury Square House**
**8 Salisbury Square**
**London EC4Y 8AP (GB)**

Remarks:
•The complete document including Reference
Table(s) and the Sequence Listing(s) can be
downloaded from the EPO website
•This application was filed on 10-02-2026 as a
divisional application to the application mentioned
under INID code 62.
•Claims filed after the date of filing of the application/
after the date of receipt of the divisional application
(Rule 68(4) EPC).

(54) **DNA BINDING PROTEINS FOR REGULATING GENE EXPRESSION**

(57)    The present disclosure provides methods and polypeptides for opening closed chromatin. The present disclosure provides methods and polypeptides for increasing expression of fetal hemoglobin G (HBG) in a cell by, for example, reducing binding of an endogenous transcription repressor to a regulatory sequence and/or potentiating binding of a transcriptional activator to a sequence in the fetal $\gamma$-globin gene promoter. The present disclosure also provides methods and DNA binding polypeptides (DBPs) for modulating expression of a gene in a cell which gene includes a regulatory region bound by a transcription factor (TF), wherein the DBP competes with the TF for binding to a regulatory sequence comprising CACC box or GATA site.

EP 4 752 228 A2

**Description**

**CROSS-REFERENCE TO RELATED APPLICATIONS**

This application claims priority to United States Provisional Application Serial No. 62/175,467 filed April 15, 2021, the disclosure of which is herein incorporated by reference in its entirety.

**INCORPORATION BY REFERENCE OF SEQUENCE LISTING PROVIDED AS A TEXT FILE**

A Sequence Listing is provided herewith as a text file, "ALTI-732WO Seq List_ST25.txt," created on April 15, 2022 and having a size of 111,616 bytes. The contents of the text file are incorporated by reference herein in their entirety.

**INTRODUCTION**

Transcription factors are frequently targeted for modulating gene expression. Modulation of gene expression is useful in studying protein function as well as in treating diseases.

Anemia, a red blood cell disorder, can be defined as a reduction in the ability of blood to transport oxygen. The majority of red blood cell disorders are caused by genetic defects that result in abnormal hemoglobin, such as, sickle cell syndromes; low hemoglobin, such as, thalassemia syndromes; or both, e.g., syndromes associated with unstable hemoglobin.

Fetal globin (also known as hemoglobin gamma "HBG" or gamma globin) normally combines with alpha globin chains prenatally to form fetal hemoglobin (HbF). Fetal globin is replaced by beta globin or hemoglobin beta (HBB) after birth, which then combines with alpha globin to form adult hemoglobin A. Fetal globin performs the same function as beta globin, and can combine with the alpha chains to generate a healthy form of hemoglobin in adults.

The various types of beta thalassemia are syndromes resulting from mutations, which produce a deficiency of beta globin chains. In beta thalassemia, the unmatched alpha globin chains aggregate inside red blood cells (RBCs) and their progenitors, causing the premature destruction of RBCs and RBC progenitors, which results in anemia, transfusion-dependence, iron overload, organ failure, and early death.

In sickle cell disease (SCD), one amino acid substitution in the beta globin chain results in the generation of sickling hemoglobin (HbS), which allows polymerization with repeated cycles of deoxygenation resulting in "sickling" of RBCs. The sickled RBCs undergo hemolysis, while adhesive sickled RBCs occlude the microcirculation, provoking widespread tissue ischemia and organ infarction. The natural history of SCD is marked by painful crises, acute chest syndrome, and eventual potentially life-threatening sequelae, including renal insufficiency, retinitis, osteonecrosis, osteomyelitis, aplastic crises, functional asplenism, stroke, priapism, and severe pulmonary hypertension.

The disclosure herein provides novel methods and compositions for modulating expression of target genes. The modulation may involve increasing expression of fetal hemoglobin in a patient with a blood disorder, including beta thalassemia and sickle cell disease.

**SUMMARY**

The present disclosure provides methods for remodeling chromatin architecture in a regulatory region of a gene of interest in a cell by providing in the cell a DNA-binding polypeptide (DBP) that binds to a sequence in the regulatory region of the gene. In certain embodiments, the method may be used to modulate expression of the gene. In certain embodiments, the DBP may have no functional domain, e.g., an enzyme, a transcription factor, etc. In certain embodiments, the remodeling comprises decreasing chromatin condensation and/or removing nucleosomes bound to the chromatin. Thus, the methods and DBPs disclosed herein may be used for localized opening of a closed region of the chromatin resulting in expression of gene(s) present in the closed region. In certain embodiments, the opening of a closed chromatin may be assayed by measuring sensitivity of the chromatin to cleavage by a DNase, e.g., detecting DNase I hypersensitive sites (DHSs) in the region upon exposure to the DBP and comparing to the number and/or pattern of DHSs in the same region in a cell not exposed to the DBP.

In certain embodiments, the DBP may be used to open a transcriptionally inactive region of the chromatin and enhance binding of TFs to sequences adjacent to the region at which the DBP binds. In certain embodiments, the DBP may be designed to bind to a site bound by a transcription factor (TF) and compete with the TF for binding to the site. In certain embodiments, the DBP may not only compete with the TF but may also enhance binding of TFs to sequences adjacent to the region at which the DBP binds. In certain embodiments, the DBP may not compete with a TF but may enhance binding of TFs to sequences adjacent to the region at which the DBP binds. In certain embodiments, the DBP may bind to the same site as a transcriptional repressor and in addition to decreasing the activity of the repressor may also enhance binding of transcriptional activator(s) to site(s) adjacent the site to which the transcriptional repressor binds. In certain embodiments, the DBP may bind to the same site as a transcriptional activator and in addition to decreasing the activity of the activator may also enhance binding of transcriptional activator(s) to site(s) adjacent the site to which the transcriptional activator binds.

In certain embodiments, the DBP may bind to a sequence in a regulatory region of the gene of interest which sequence is present at a position at least 100 nucleotides upstream of the transcription start site (TSS) (i.e., at least position -100). In certain embodiments, the DBP may bind to a sequence in a regulatory region of the gene of interest which sequence is present at a position up to 400 nucleotides upstream of the transcription start site (i.e., up to position - 400). In certain embodiments, the DBP may bind to a sequence in a regulatory region of the gene of interest which sequence is present at a position between -350 to -150; -300 to -175; - 250 to -150; -230 to -180; or -230 to -190 with reference to the TSS.

In certain embodiments, the gene of interest may be γ-globin gene (*HBG1* and/or *HBG2)* encoding fetal hemoglobin G (HBG) and the method may include increasing expression of fetal hemoglobin G (HBG) in a cell by binding of a DBP to a regulatory sequence in the fetal γ-globin gene promoter (*HBG1* and/or *HBG2).* In certain embodiments, the DBP may bind to a nucleotide sequence present between -350 to -150; -300 to -175; -250 to -150; -230 to -180; or - 230 to -190 with reference to the TSS in the fetal γ-globin gene promoter of *HBG1* and/or *HBG2* gene. In certain embodiments, the DBP may bind to a nucleotide sequence present between positions -222 to -193 with reference to the TSS in the *HBG1* and/or *HBG2* gene. In certain embodiments, the DBP may bind to a nucleotide sequence present between positions -222 to - 193 with reference to the TSS in the *HBG1* gene. In certain embodiments, the DBP may bind to a nucleotide sequence present between positions -227 to -193 with reference to the TSS in the *HBG2* gene.

In certain embodiments, a method for increasing expression of fetal hemoglobin G (HBG) in a cell, e.g., an adult erythrocyte, may include expressing in the cell a DBP that binds to a sub-sequence present in the sequence AGCAG TATCCTCTTGGGGGCCCCTTCCCCA (SEQ ID NO:3) in the regulatory region of the HBG1 and/or the HBG2 gene or a complement thereof. In certain embodiments, the sub-sequence may include the nucleotide sequence TCTT or a complement thereof. In certain embodiments, the DBP may bind to at least the nucleotide sequence TCTT and up to 16 nt present upstream or downstream of the sequence TCTT. In certain embodiments, the DBP may bind to at least the nucleotide sequence TCTT and up to 16 nt present downstream of the sequence TCTT. In certain embodiments, the DBP may bind to at least the nucleotide sequence TCTT and up to 11 nt present upstream of the sequence TCTT.

In certain embodiments, a method for increasing expression of fetal hemoglobin G (HBG) in a cell, e.g., an adult erythrocyte, may include expressing in the cell a DBP that binds to a sub-sequence present in the sequence TAAGC AGCAGTATCCTCTTGGGGGCCCCTTCCCCA (SEQ ID NO:8) in the regulatory region of the HBG2 gene or a complement thereof. In certain embodiments, the sub-sequence may include the nucleotide sequence TCTT or a complement thereof. In certain embodiments, the DBP may bind to at least the nucleotide sequence TCTT and up to 16 nt present upstream or downstream of the sequence TCTT. In certain embodiments, the DBP may bind to at least the nucleotide sequence TCTT and up to 16 nt present downstream of the sequence TCTT. In certain embodiments, the DBP may bind to at least the nucleotide sequence TCTT and up to 15 nt present upstream of the sequence TCTT.

The DBP disclosed herein and used in the methods provided herein may include a plurality of repeat units (RUs) that are arranged from N-terminus to C-terminus to bind to a sequence provided herein. The RUs may be derived from a TALE protein and the DBP may include at least 11.5 RUs, which include 11 RUs and a terminal half-repeat unit (0.5 RU).

The present disclosure also provides methods and DNA binding polypeptides (DBPs) for modulating expression of a gene in a cell which gene includes a regulatory region bound by a transcription factor (TF), wherein the DBP competes with the TF for binding to a regulatory sequence comprising CACC box or GATA site. The DBP may be designed to bind a sequence comprising the binding site for the TF or a complement thereof and additional nucleotides present on one or both sides of the sequence. Accordingly, the DBP specifically binds to binding site for the TF in the target gene but not in other genes that are also regulated by binding of the TF but do not include the nucleotides present on one or both sides of the sequence.

In certain aspects, recombinant DBPs that include a plurality of RUs that are arranged from N-terminus to C-terminus to bind to a sequence bound by the transcriptional repressor, ZBTB7A in the fetal γ-globin gene promoter is provided. The RUs may be derived from a TALE protein and the DBP may include at least 11.5 RUs, which include 11 RUs and a terminal half-repeat unit (0.5 RU). The DBP displaces ZBTB7A from the fetal γ-globin gene promoter and relieves suppression of the fetal γ-globin gene. DBPs that decrease expression of adult hemoglobin B (HBB) are also disclosed.

In certain aspects, a cell line comprising a stable expression of the recombinant DBP as provided herein is disclosed. The cell line may be produced by stable integration into the genome of the cells of a nucleic acid encoding the DBP. The cell line may be administered to a subject in need thereof.

## BRIEF DESCRIPTION OF THE DRAWINGS

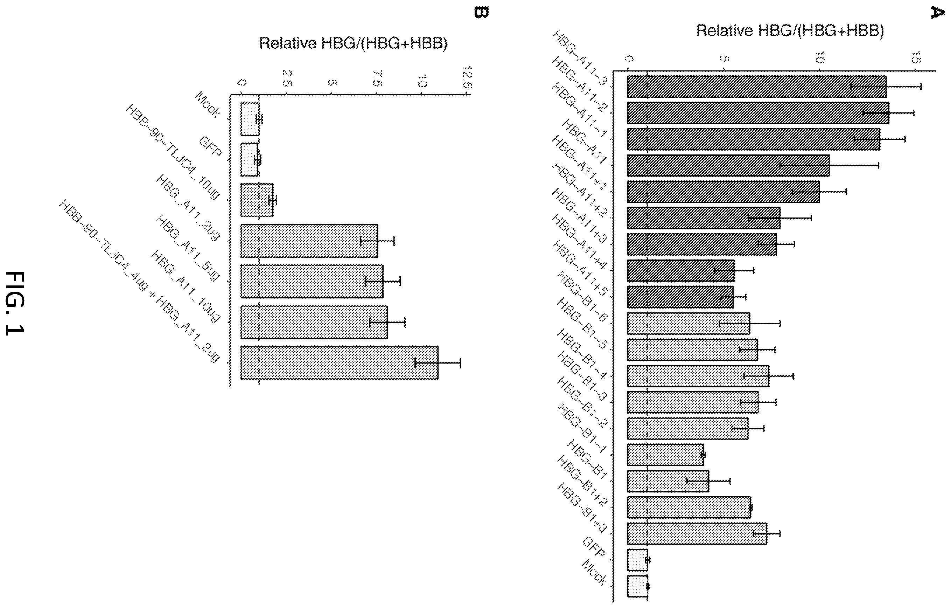

**FIG. 1.** TALE proteins yield globin switching. **A.** Relative expression of HBG out of total globin (HBG + HBB), normalized to Mock transfection, in HUDEP-2 cells 48 hours post-transfection of mRNA of A11 and B1 TALEs of varying lengths with no effector domain and measured by TaqMan qPCR. **B.** Varying amounts of A11 TALE, or a TALE targeting the HBB CACC box at -90, or a combination of these two TALEs.

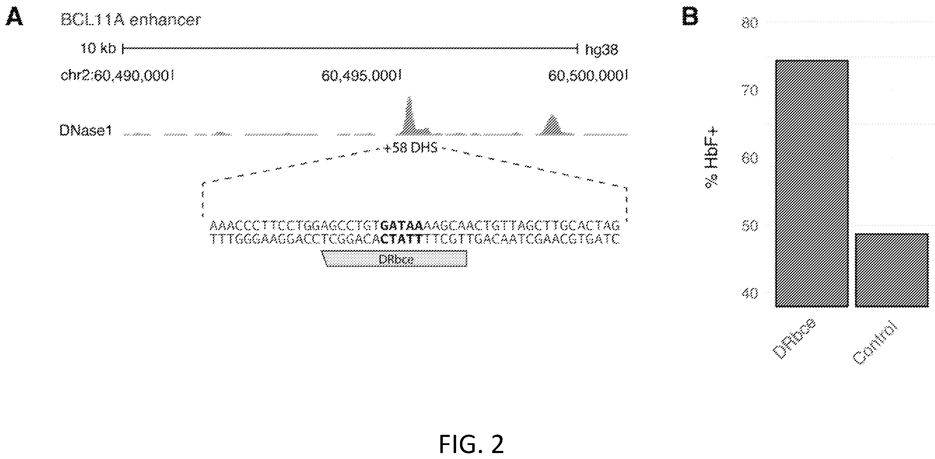

**FIG. 2.** TALE binding over activating GATA site in +58 DHS of the BCL11A enhancer activates HbF expression. **A.** Diagram of DRbce TALE binding position over GATA site (from top to bottom SEQ ID NOs:212-213). **B.** %HbF positive cells in CD34+ cells transduced with lentivirus carrying the DRbce TALE or non-transduced Control cells at terminal differentiation.

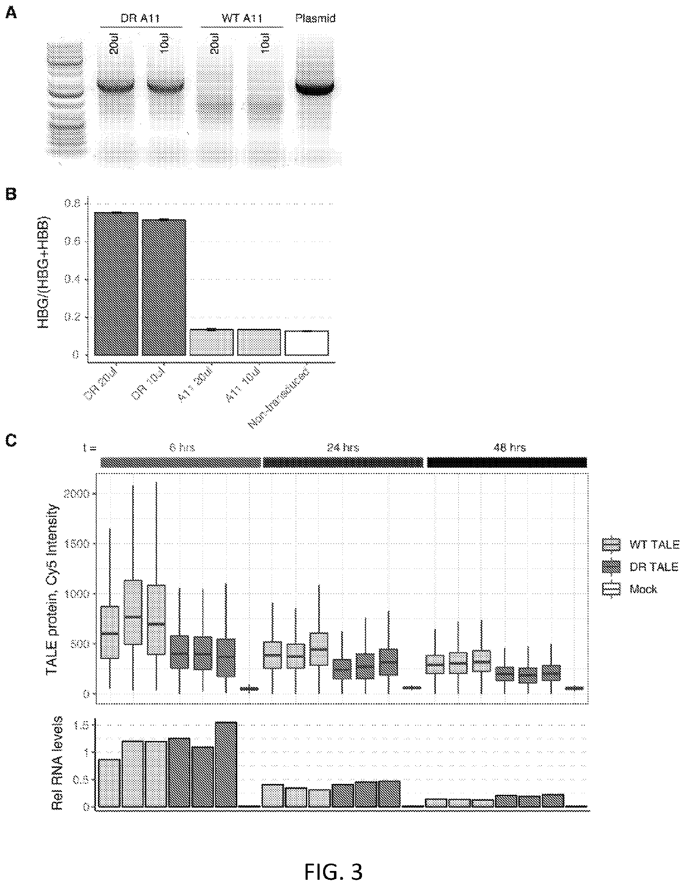

**FIG. 3.** Diversified Repeat (DR) TALEs expressed with lentivirus integrate without recombination and produce functional proteins at similar expression levels as WT TALEs. **A.** PCR amplifying the TALE sequence from genomic DNA of HUDEP-2 cells transduced with lentivirus carrying the DRA11 or the WT A11 TALE sequence. Amplification of plasmid DNA is shown for size reference. **B.** Relative expression of HBG out of total globin (HBG + HBB) in HUDEP-2 cells 10 days post-transduction. **C.** Top Panel: Immunofluorescent imaging of FLAG (FLAG IF) at 6, 24, or 48 hours post-transfection of triplicate in vitro transcription (IVT) preparations of the WT A11 TALE or DR A11 TALE, and a no mRNA (Mock) control. Bottom Panel: Relative RNA levels at 6, 24, and 48 hours post-transfection measured by qPCR of a region in the TALE N-terminus.

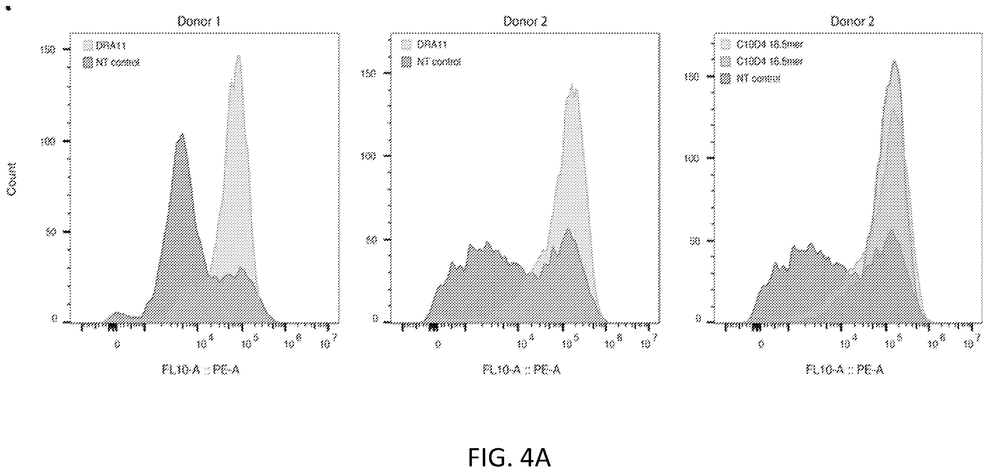

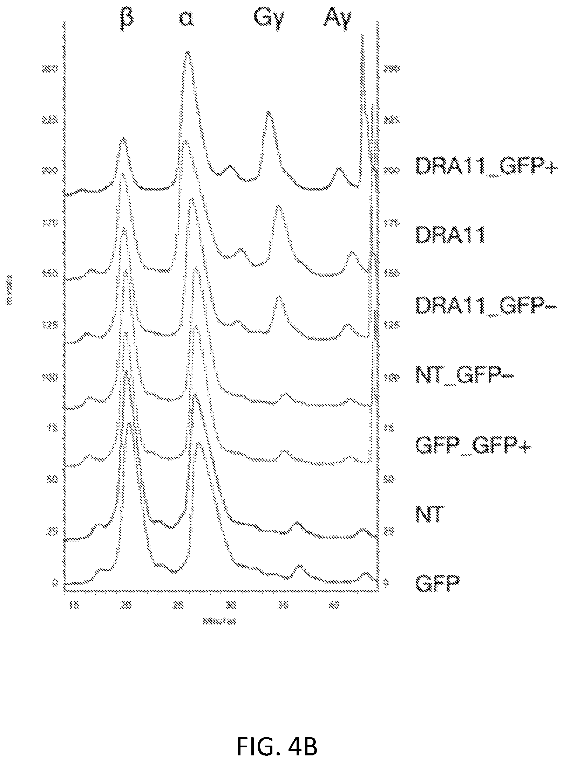

**FIGS. 4A-4B.** TALEs activate fetal hemoglobin (HbF) expression in primary human cells. **FIG. 4A.** HbF FACS of terminally differentiated, enucleated RBCs derived from CD34+ hematopoietic stem cells (HSCs) from two different donors. Cells were transduced with DRA11 (left and middle panels), or alternatively diversified versions of HBG-A11 or HBG-A11+2 (C10D4 16.5mer and C10D4 18.5mer respectively; right panel). Non-transduced cells (NT) were included as a control. **FIG. 4B.** HPLC of protein lysate from terminally differentiated RBCs that were differentiated from CD34+ HSCs transduced with DRA11, GFP, or not transduced. Populations were also sorted for GFP+ and/or GFP- cells at D5 of the differentiation.

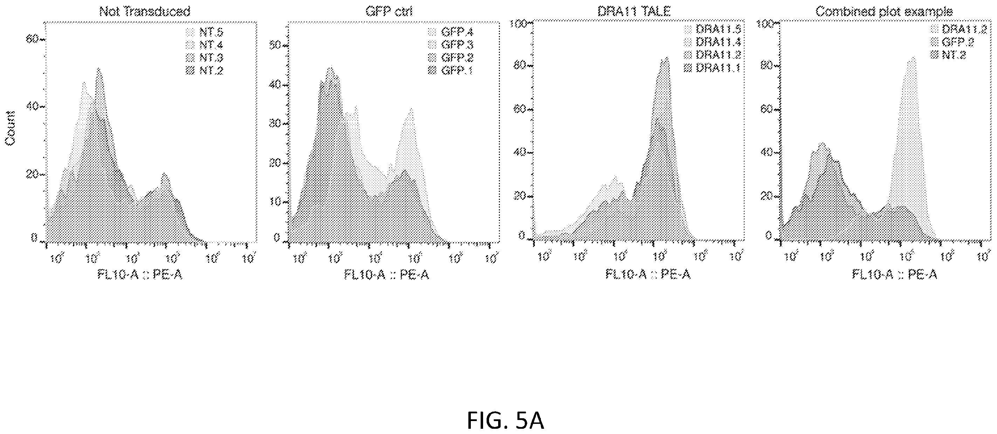

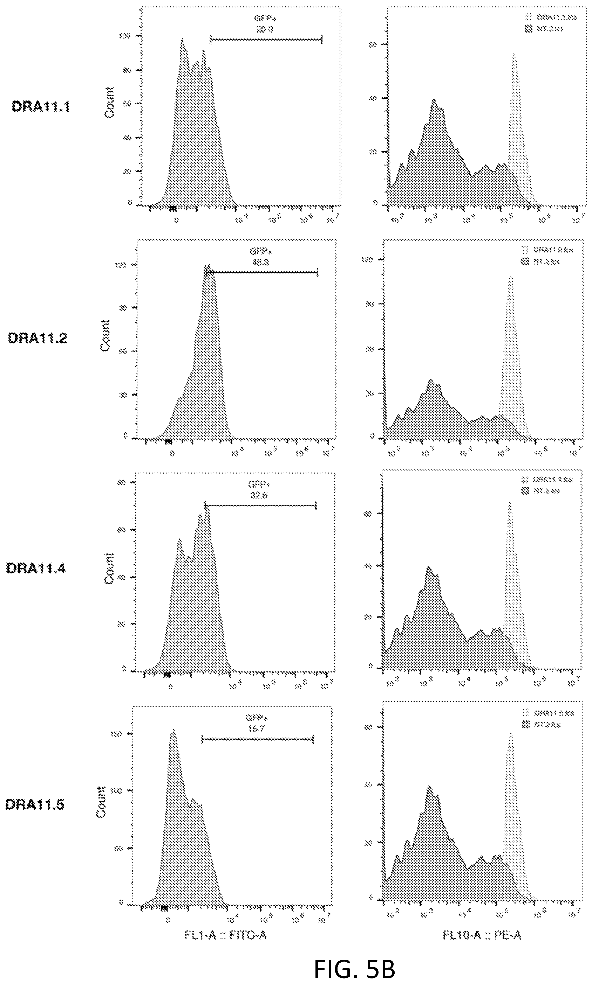

**FIGS. 5A-5B.** DRA11-TALE-transduced CD34+ HSCs engraft into mice, persist in vivo, and differentiate into high HbF-expressing cells. **FIG. 5A.** CD34+ HSCs were transduced with DRA11, GFP, or not transduced, engrafted into NSGW41 mice, and bone marrow was harvested ~5 months later. Cells from the mouse bone marrow were differentiated ex vivo. HbF FACS was performed and the enucleated population distributions for HbF expression (PE) are shown. **FIG. 5B.** GFP+ differentiated cells from mice all show strong HbF expression.

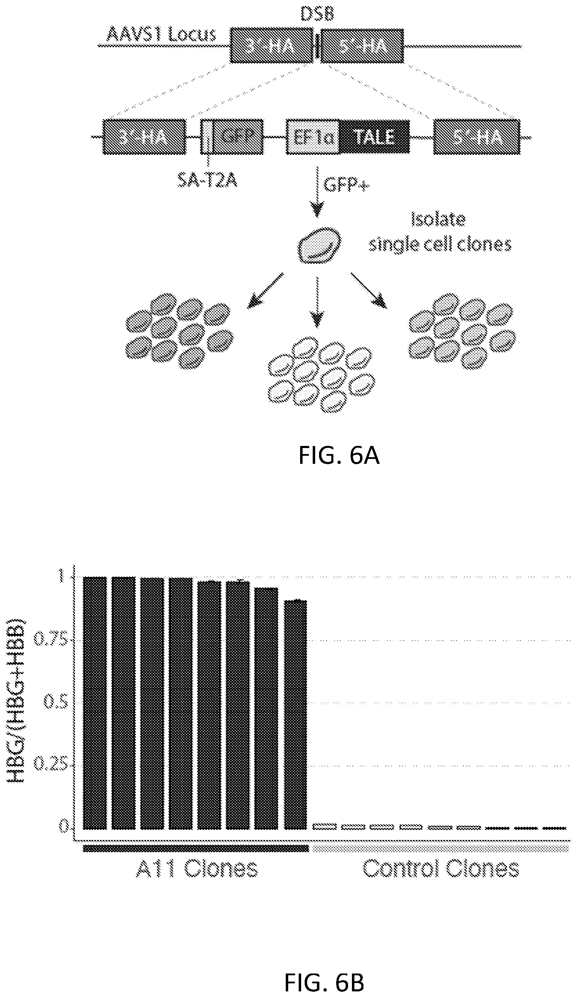

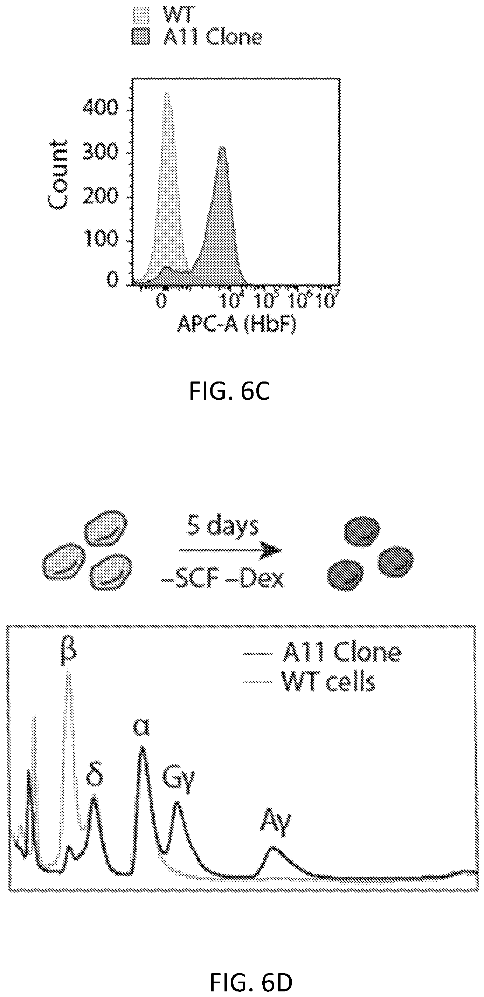

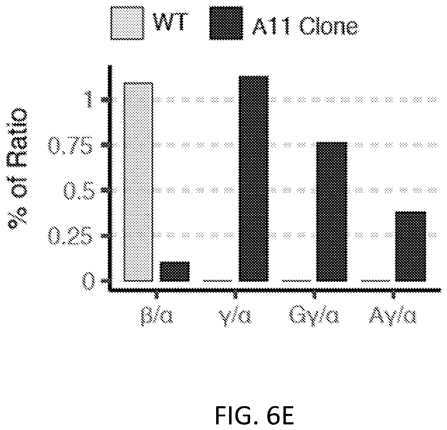

**FIGS. 6A-6E.** Stable expression of TALEs binding to the -200 region of the HBF promoter completely reverse the hemoglobin switch, activating fetal hemoglobin (HbF) expression and concomitantly reducing adult beta-globin expression. **FIG. 6A.** The A11 TALE was stably integrated into the AAVS1 locus of HUDEP-2 cells and single cell clones were generated by sorting GFP+ single cells with FACS. **FIG. 6B.** RT-qPCR of HBG and HBB mRNA expression reveals clones with nearly 100% HBG expression out of total HBG and HBB expression, while control clones show no activation of HBG expression. **FIG. 6C.** Flow cytometry of HbF stained cells reveals activation of HbF expression in nearly 100% of cells, demonstrating HbF activation the protein level. **FIG. 6D.** Reverse phase HPLC chromatogram traces of protein lysate from differentiated HUDEP-2 cells. Cells expressing the A11 TALE show strong G-gamma and A-gamma globin expression and almost no beta globin expression. These results are quantified in **FIG. 6E.**

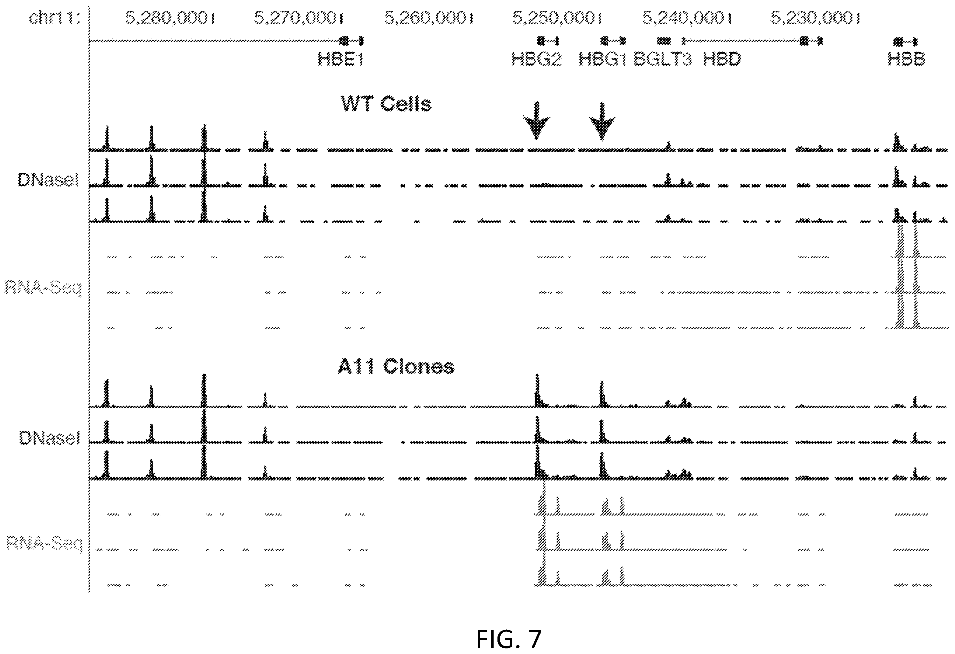

**FIG. 7.** TALEs can penetrate closed chromatin to exert effect. Normalized density traces of DNase1 Sequencing and RNA Sequencing in three A11-expressing clones and three WT clones at the globin locus. Black arrows show the location of the TALE recognition sequence, in which chromatin is closed in WT cells. The TALE catalyzes the formation of an active DNase hypersensitive site in A11-expressing clones.

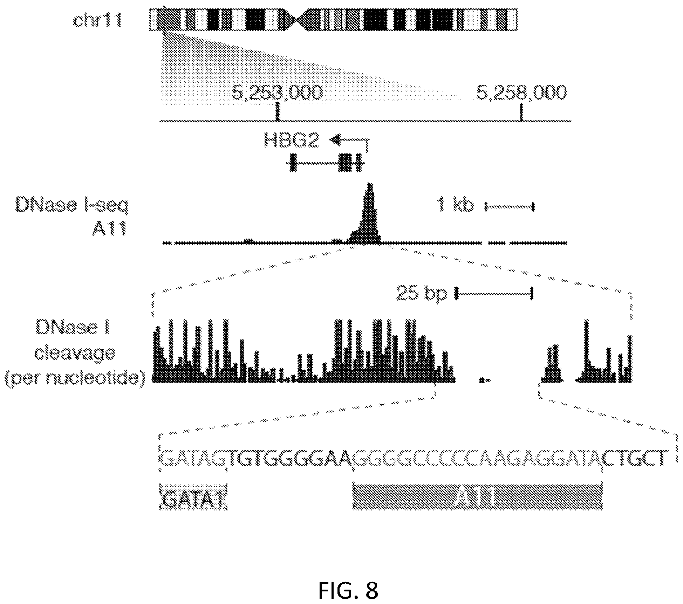

**FIG. 8.** A11 TALE blocks ZBTB7A binding site and potentiates TF binding to nearby GATA site. DNase1 Sequencing cut counts reveal TF footprints where DNase cleavage is blocked by the presence of a bound TF. DNase1-Seq from A11-expressing clones shows a deep footprint of the A11 TALE, reflective of the factor being bound at every allele with an active DNase1 hypersensitive site. The footprint extends past the binding site of the A11 TALE and into the neighboring GATA1 binding site, demonstrating TF binding to this neighboring site (SEQ ID NO:214).

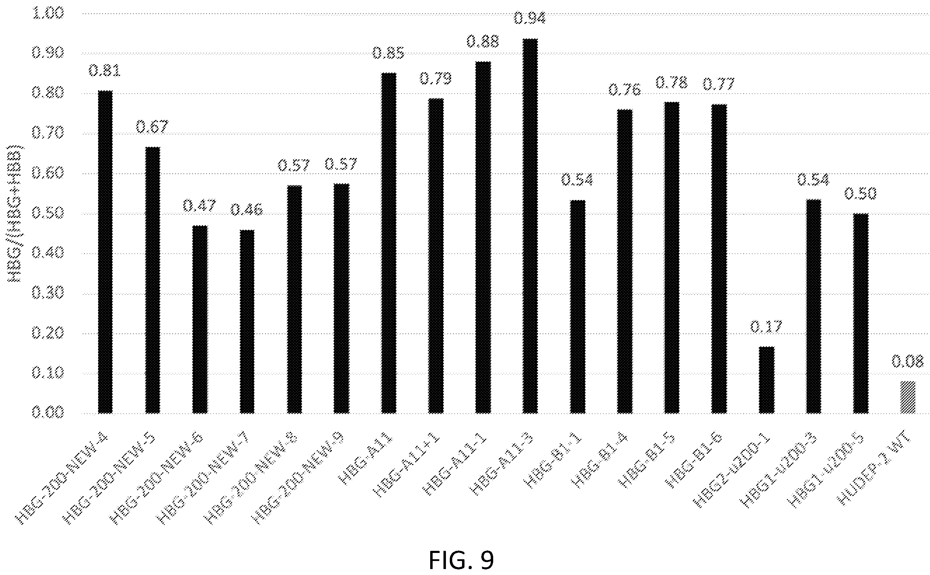

**FIG. 9.** Lentiviral delivered TALEs drive strong globin switching. Relative expression of HBG out of total globin (HBG + HBB), in HUDEP-2 cells transduced with lentivirus containing TALEs of varying lengths and binding locations (see table 2) with no effector domain and measured by TaqMan qPCR.

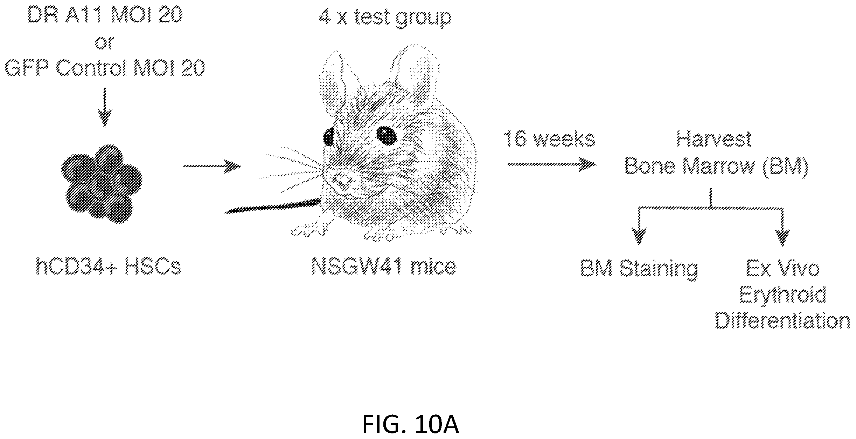

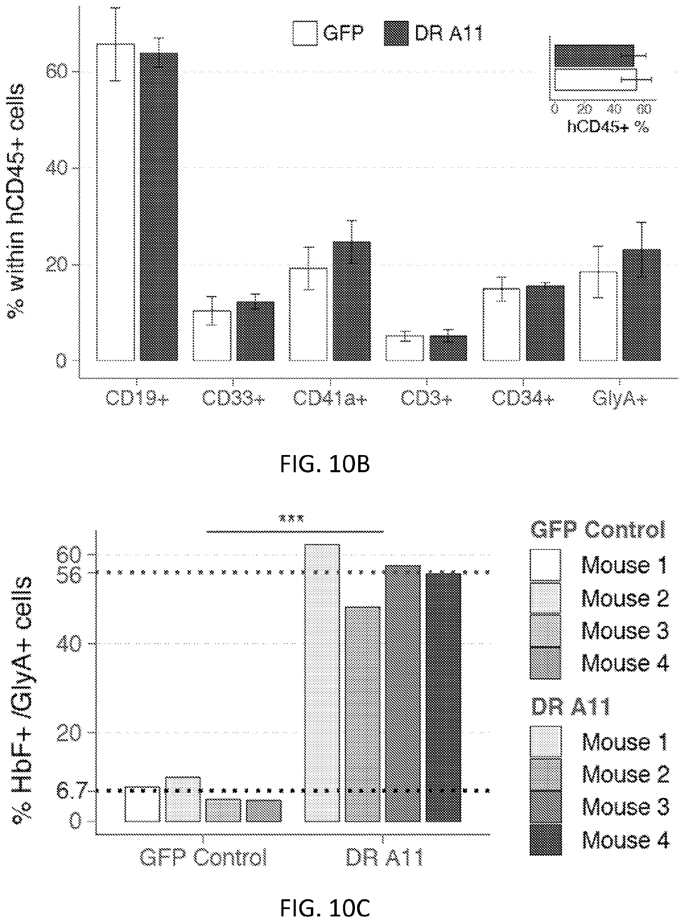

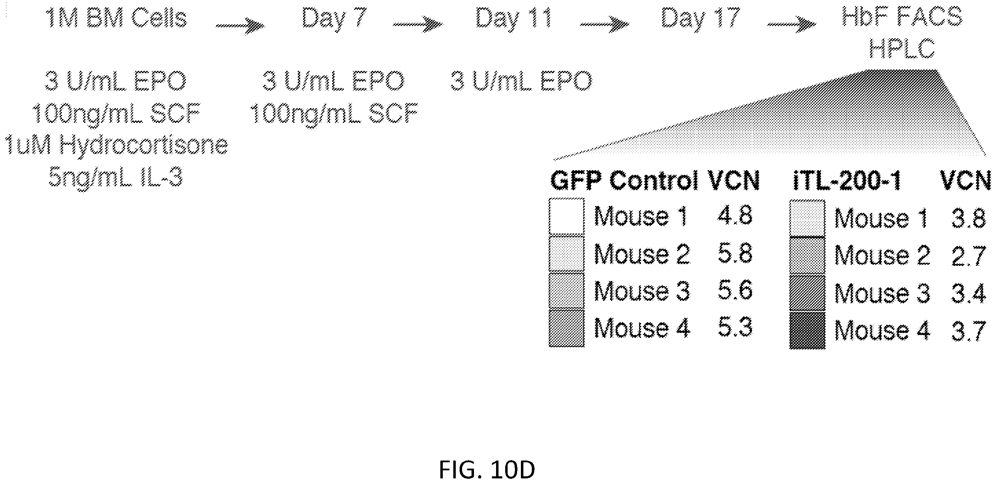

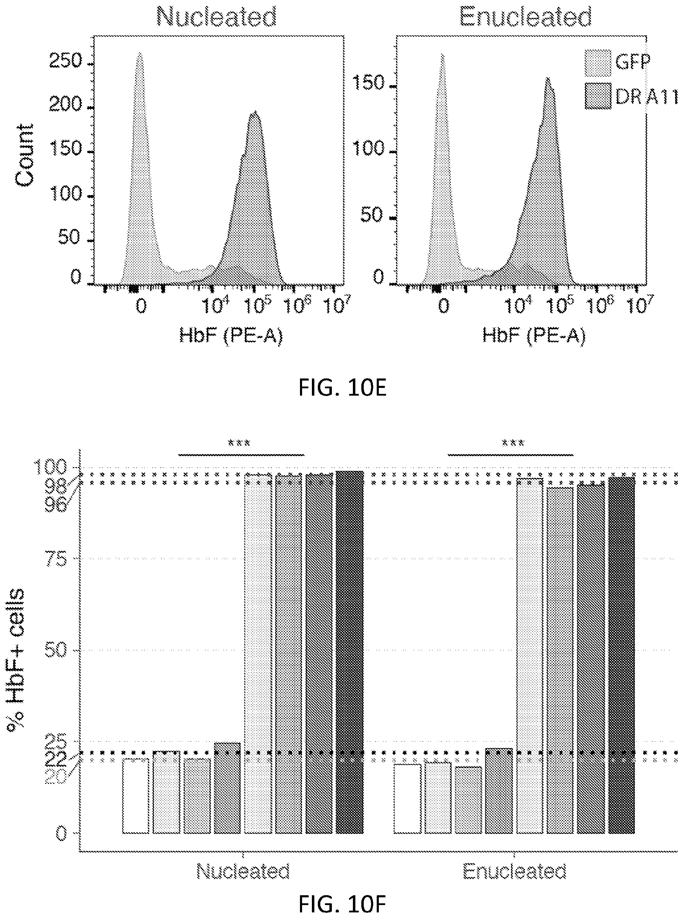

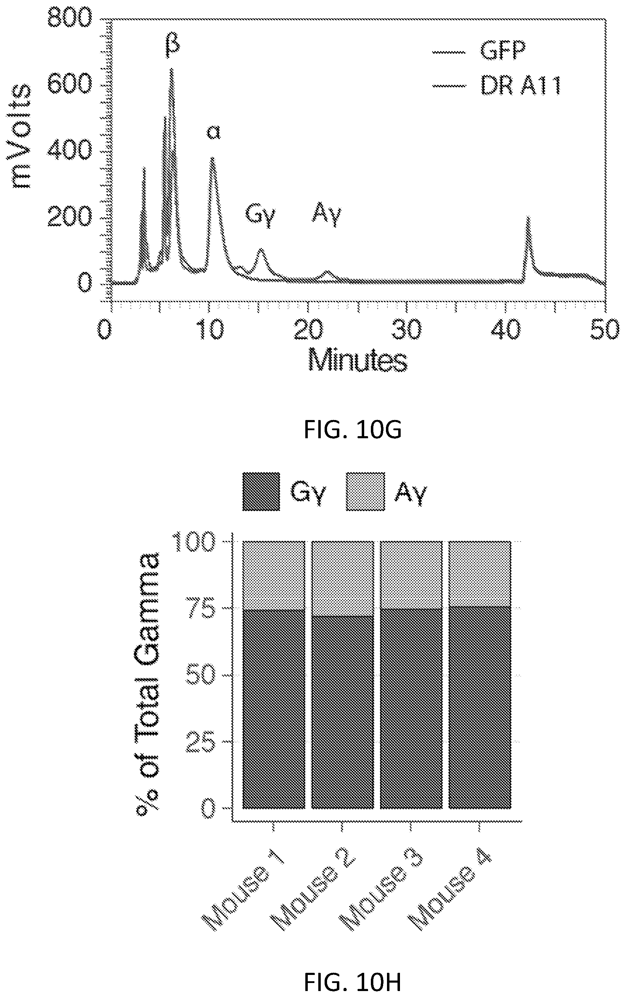

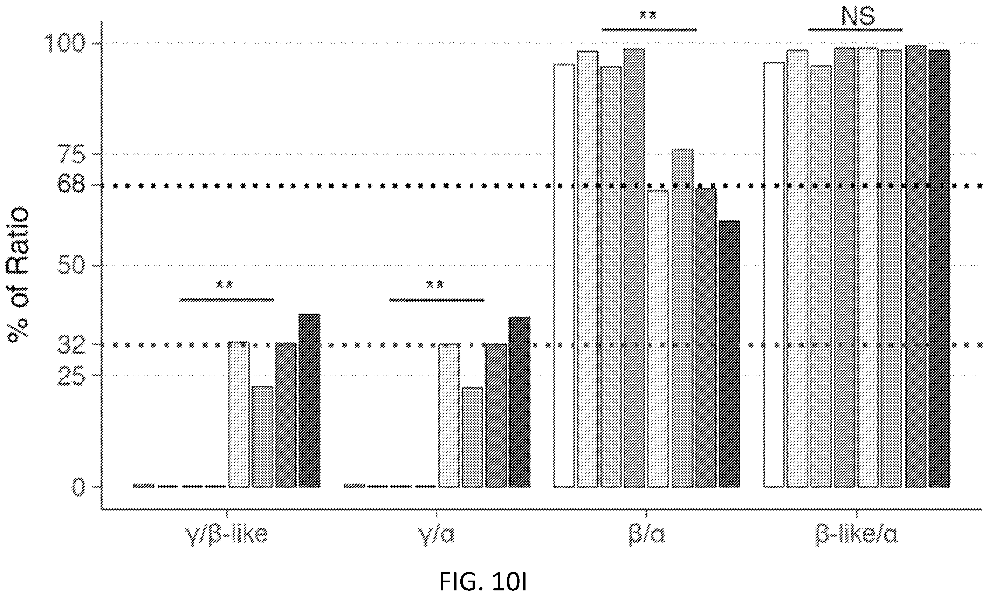

**FIGS. 10A-10I.** Long-term survival of modified cells and persistence of hemoglobin switching in mice. **FIG. 10A.** Human CD34+ hematopoietic stem cells transduced with DR A11 or control GFP lentivirus were engrafted into NSGW41 mice. Bone marrow (BM) was harvested 16 weeks post-engraftment for BM staining and ex vivo erythroid differentiation. **FIG. 10B.** BM was stained with various lineage markers and assessed by flow cytometry. No significant differences were observed between DR A11 and GFP control treated groups. **FIG. 10C.** HbF staining and flow cytometry on bone marrow revealed an average of 56% HbF+ cells out of all GlyA+ cells compared to 6.7% HbF+ cells in GFP controls. **FIG. 10D.** 1M BM cells from each mouse differentiated ex vivo into terminal red blood cells (RBCs). **FIG. 10E.** HbF staining and flow cytometry of ex vivo differentiated RBCs shows HbF activation in nearly 100% of cells within both nucleated and enucleated populations. These results are quantified in **FIG. 10F. FIG. 10G.** Reverse phase HPLC chromatogram traces of protein lysate from ex vivo cultured terminally differentiated RBCs. Results are quantified in **FIG. 10I. FIG. 10H.** G-gamma and A-gamma globin proportions, determined by RP-HPLC, reflect the proportions observed in fetal cells.

## DETAILED DESCRIPTION

The present disclosure provides methods and DBP for opening a transcriptionally inactive region of the genome in a cell. The method and the DBP may be used for increasing expression of a gene located in the transcriptionally inactive region. The method involves introducing in the cell a DBP that binds to a sequence in a regulatory region (e.g., a promoter) region of a gene of interest. The cell may not express the gene in absence of the DBP or may express the gene at a low level in absence of the DBP.

The present disclosure provides methods and polypeptides for increasing expression of fetal hemoglobin G (HBG) in a cell by reducing binding of an endogenous transcription repressor to a regulatory sequence in the fetal $\gamma$-globin gene promoter. The present disclosure also provides methods and DNA binding polypeptides (DBPs) for modulating expression of a gene in a cell which gene includes a regulatory region bound by a transcription factor (TF), wherein the DBP competes with the TF for binding to a regulatory sequence comprising CACC box or GATA site.

Before exemplary aspects of the present invention are described, it is to be understood that this invention is not limited to particular aspects described, as such may, of course, vary. It is also to be understood that the terminology used herein is for the purpose of describing particular aspects only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limits of that range is also specifically disclosed. Each smaller range between any stated value or intervening value in a stated range and any other stated or intervening value in that stated range is encompassed within the invention. The upper and lower limits of these smaller ranges may independently be included or excluded in the range, and each range where either, neither or both limits are included in the smaller ranges is also encompassed within the invention, subject to any specifically excluded limit in the stated range. Where the stated range includes one or both of the limits, ranges excluding either or both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, some potential and exemplary methods and materials may now be described. Any and all publications mentioned herein are incorporated herein by reference to disclose and describe the methods and/or materials in connection with which the publications are cited. It is understood that the present disclosure supersedes any disclosure of an incorporated publication to the extent there is a contradiction.

It must be noted that as used herein and in the appended claims, the singular forms "a", "an", and "the" include plural referents unless the context clearly dictates otherwise. Thus, for example, reference to "a protein" includes a plurality of such proteins and reference to "the polynucleotide" includes reference to one or more polynucleotides, and so forth.

It is further noted that the claims may be drafted to exclude any element which may be optional. As such, this statement is intended to serve as antecedent basis for use of such exclusive terminology as "solely", "only" and the like in connection with the recitation of claim elements, or the use of a "negative" limitation.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed. To the extent such publications may set out definitions of a term that conflicts with the explicit or implicit definition of the present disclosure, the definition of the present disclosure controls.

As will be apparent to those of skill in the art upon reading this disclosure, each of the individual aspects described and illustrated herein has discrete components and features which may be readily separated from or combined with the features of any of the other several aspects without departing from the scope or spirit of the present invention. Any recited method can be carried out in the order of events recited or in any other order which is logically possible.

## DEFINITIONS

As used herein, the term "derived" in the context of a polypeptide refers to a polypeptide that has a sequence that is based on that of a protein from a particular source (e.g., an animal pathogen such as *Legionella* or a plant pathogen such as Xanthomonas). A polypeptide derived from a protein from a particular source may be a variant of the protein from the particular source (e.g., an animal pathogen such as Legionella or a plant pathogen such as Xanthomonas). For example, a polypeptide derived from a protein from a particular source may have a sequence that is modified with respect to the protein's sequence from which it is derived. A polypeptide derived from a protein from a particular source shares at least 30% sequence identity with, at least 40% sequence identity with, at least 50% sequence identity with, at least 60% sequence identity with, at least 70% sequence identity with, at least 80% sequence identity with, or at least 90% sequence identity with the protein from which it is derived.

The DBP disclosed herein may be derived from a nucleic acid binding domain of a DNA binding protein of an animal or plant pathogen. The term "modular" as used herein in the context of a nucleic acid binding domain, e.g., a modular animal pathogen derived DNA binding polypeptide (MAP-DBP) indicates that the plurality of repeat units present in the DBP can be rearranged and/or replaced with other repeat units and can be arranged in an order such that the DBP binds to the target nucleic acid. For example, any repeat unit in a modular nucleic acid binding domain can be switched with a different repeat unit. In some aspects, modularity of the nucleic acid binding domains disclosed herein allows for switching the target nucleic acid base for a particular repeat unit by simply switching it out for another repeat unit. In some aspects, modularity of the nucleic acid binding domains disclosed herein allows for swapping out a particular repeat unit for another repeat unit to increase the affinity of the repeat unit for a particular target nucleic acid. Overall, the modular nature of the nucleic acid binding domains disclosed herein enables the development of DBP that can precisely target any nucleic acid sequence of interest.

The terms "polypeptide," "peptide," and "protein", used interchangeably herein, refer to a polymeric form of amino acids of any length, which can include genetically coded and non-genetically coded amino acids, chemically or biochemically modified or derivatized amino acids, and polypeptides having modified polypeptide backbones. The terms include fusion proteins, including, but not limited to, fusion proteins with a heterologous amino acid sequence, fusion proteins with heterologous and homologous leader sequences, with or without N-terminus methionine residues; immunologically tagged proteins; and the like. In specific aspects, the terms refer to a polymeric form of amino acids of any length which include genetically coded amino acids. In particular aspects, the terms refer to a polymeric form of amino acids of any length which include genetically coded amino acids fused to a heterologous amino acid sequence.

The term "heterologous" refers to two components that are defined by structures derived from different sources. For example, in the context of a polypeptide, a "heterologous" polypeptide may include operably linked amino acid sequences that are derived from different polypeptides (e.g., a NBD and a functional domain derived from different sources). Similarly, in the context of a polynucleotide encoding a chimeric polypeptide, a "heterologous" polynucleotide may include operably linked nucleic acid sequences that can be derived from different genes. Other exemplary "heterologous" nucleic acids include expression constructs in which a nucleic acid comprising a coding sequence is operably linked to a regulatory element (e.g., a promoter) that is from a genetic origin different from that of the coding sequence (e.g., to provide for expression in a host cell of interest, which may be of different genetic origin than the promoter, the coding sequence or both). In the context of recombinant cells, "heterologous" can refer to the presence of a nucleic acid (or gene product, such as a polypeptide) that is of a different genetic origin than the host cell in which it is present.

The term "operably linked" refers to linkage between molecules to provide a desired function. For example, "operably linked" in the context of nucleic acids refers to a functional linkage between nucleic acid sequences. By way of example, a nucleic acid expression control sequence (such as a promoter, signal sequence, or array of transcription factor binding sites) may be operably linked to a second polynucleotide, wherein the expression control sequence affects transcription and/or translation of the second polynucleotide. In the context of a polypeptide, "operably linked" refers to a functional linkage between amino acid sequences (e.g., different domains) to provide for a described activity of the polypeptide.

As used herein, the term "cleavage" refers to the breakage of the covalent backbone of a nucleic acid, e.g., a DNA molecule. Cleavage can be initiated by a variety of methods including, but not limited to, enzymatic or chemical hydrolysis of a phosphodiester bond. Both single-stranded cleavage and double-stranded cleavage are possible, and double-stranded cleavage can occur as a result of two distinct single-stranded cleavage events. DNA cleavage can result in the production of either blunt ends or staggered ends. In certain aspects, the polypeptides provided herein are used for targeted double-stranded DNA cleavage.

A "cleavage half-domain" is a polypeptide sequence which, in conjunction with a second polypeptide (either identical or different) forms a complex having cleavage activity (preferably double-strand cleavage activity).

A "target nucleic acid," "target sequence," or "target site" is a nucleic acid sequence that defines a portion of a nucleic acid to which a binding molecule, such as, the DBP disclosed herein will bind. The target nucleic acid may be present in inside a cell. A target nucleic acid may be present in a regulatory region, e.g., promoter sequence, of a target gene whose expression is to be modulated by the DBP.

An "exogenous" molecule is a molecule that is not normally present in a cell but can be introduced into a cell by one or more genetic, biochemical or other methods. An exogenous molecule can comprise, for example, a functioning version of a malfunctioning endogenous molecule, e.g., a gene or a gene segment lacking a mutation present in the endogenous gene. An exogenous nucleic acid can be present in an infecting viral genome, a plasmid or episome introduced into a cell. Methods for the introduction of exogenous molecules into cells are known to those of skill in the art and include, but are not limited to, lipid-mediated transfer (i.e., liposomes, including neutral and cationic lipids), electroporation, direct injection, cell fusion, particle bombardment, calcium phosphate co-precipitation, DEAE-dextran-mediated transfer and viral vector-mediated transfer.

By contrast, an "endogenous" molecule is one that is normally present in a particular cell at a particular developmental stage under particular environmental conditions. For example, an endogenous nucleic acid can comprise a chromosome, the genome of a mitochondrion, chloroplast or other organelle, or a naturally-occurring episomal nucleic acid. Additional endogenous molecules can include proteins, for example, transcription factors and enzymes.

A "gene," for the purposes of the present disclosure, includes a DNA region encoding a gene product, as well as all DNA regions which regulate the production of the gene product, whether or not such regulatory sequences are adjacent to coding and/or transcribed sequences. Accordingly, a gene includes, but is not necessarily limited to, promoter sequences, terminators, translational regulatory sequences such as ribosome binding sites and internal ribosome entry sites, enhancers, silencers, insulators, boundary elements, replication origins, matrix attachment sites and locus control region.

"Gene expression" refers to the conversion of the information, contained in a gene, into a gene product. A gene product can be the direct transcriptional product of a gene (e.g., mRNA, tRNA, rRNA, antisense RNA, ribozyme, structural RNA, shRNA, RNAi, miRNA or any other type of RNA) or a protein produced by translation of a mRNA. Gene products also include RNAs which are modified, by processes such as capping, polyadenylation, methylation, and editing, and proteins modified by, for example, methylation, acetylation, phosphorylation, ubiquitination, ADP-ribosylation, myristylation, and glycosylation.

"Modulation" of gene expression refers to a change in the activity of a gene. Modulation of expression can include, but is not limited to, gene activation and gene repression. Genome editing (e.g., cleavage, alteration, inactivation, donor integration, random mutation) can be used to modulate expression. Gene inactivation refers to any reduction in gene expression as compared to a cell that does not include a polypeptide or has not been modified by a polypeptide as described herein. Thus, gene inactivation may be partial or complete.

The terms "patient" or "subject" are used interchangeably to refer to a human or a non-human animal (e.g., a mammal).

The terms "treat", "treating", treatment" and the like refer to a course of action (such as administering a polypeptide or a nucleic acid encoding the polypeptide or a cell comprising the nucleic acid encoding the polypeptide or expressing the polypeptide) initiated after a disease, disorder or condition, or a symptom thereof, has been diagnosed, observed, and the like so as to eliminate, reduce, suppress, mitigate, or ameliorate, either temporarily or permanently, at least one of the underlying causes of a disease, disorder, or condition afflicting a subject, or at least one of the symptoms associated with a disease, disorder, condition afflicting a subject.

The terms "prevent", "preventing", "prevention" and the like refer to a course of action (such as administering a polypeptide or a nucleic acid encoding the polypeptide or a cell comprising the nucleic acid encoding the polypeptide or expressing the polypeptide) initiated in a manner (e.g., prior to the onset of a disease, disorder, condition or symptom thereof) so as to prevent, suppress, inhibit or reduce, either temporarily or permanently, a subject's risk of developing a disease, disorder, condition or the like (as determined by, for example, the absence of clinical symptoms) or delaying the onset thereof, generally in the context of a subject predisposed to having a particular disease, disorder or condition. In certain instances, the terms also refer to slowing the progression of the disease, disorder or condition or inhibiting progression thereof to a harmful or otherwise undesired state.

The phrase "therapeutically effective amount" refers to the administration of an agent to a subject, either alone or as a part of a pharmaceutical composition and either in a single dose or as part of a series of doses, in an amount that is capable of having any detectable, positive effect on any symptom, aspect, or characteristics of a disease, disorder or condition when administered to a patient. The therapeutically effective amount can be ascertained by measuring relevant physiological effects.

The terms "conjugating," "conjugated," and "conjugation" refer to an association of two entities, for example, of two molecules such as two proteins, two domains (e.g., a binding domain and a cleavage domain), or a protein and an agent, e.g., a protein binding domain and a small molecule. The association can be, for example, via a direct or indirect (e.g., via a linker) covalent linkage or via non-covalent interactions. In some aspects, the association is covalent. In some aspects, two molecules are conjugated via a linker connecting both molecules. For example, in some aspects where two proteins are conjugated to each other, e.g., a binding domain and a cleavage domain of an engineered nuclease, to form a protein fusion, the two proteins may be conjugated via a polypeptide linker, e.g., an amino acid sequence connecting the C-terminus of one protein to the N-terminus of the other protein. Such conjugated proteins may be expressed as a fusion protein.

The term "consensus sequence," as used herein in the context of nucleic acid or amino acid sequences, refers to a sequence representing the most frequent nucleotide/amino acid residues found at each position in a plurality of similar sequences. Typically, a consensus sequence is determined by sequence alignment in which similar sequences are compared to each other. A consensus sequence of a protein can provide guidance as to which residues can be substituted without significantly affecting the function of the protein.

As used herein, the term "genome modifying proteins" refer to nucleic acid binding domains and functional domains which cooperate to modify genome or epigenome is a cell. Examples of genome modifying proteins are provided herein and include but are not limited to nucleic acid binding proteins comprising modular repeat units, nucleic acid binding proteins comprising zinc fingers, functional domains such as labels, tags, polypeptides having nuclease activity, methyltransferase activity, demethylase activity, DNA repair activity, DNA damage activity, deamination activity, dismutase activity, alkylation activity, depurination activity, oxidation activity, pyrimidine dimer forming activity, integrase activity, transposase activity, recombinase activity, polymerase activity, ligase activity, helicase activity, photolyase activity or glycosylase activity, e.g., nucleases, transcriptional activators, transcriptional repressors, chromatin modifying protein, and the like. Genome modifying proteins also encompass a single polypeptide comprising a nucleic acid binding domain and functional domain or two or more polypeptides, where a first polypeptide comprises a nucleic acid binding domain and a second polypeptide comprises a functional domain and wherein the first and second polypeptide associate with each other via a non-covalent interaction, such as, via a interactions mediated by first and second members of a heterodimer, where one of the first and second polypeptide is conjugated to the first member and the other polypeptide is conjugated to the second member. The DBPs provided here are capable of modifying the genome by opening a closed chromatin without being fused to a functional domain.

As used herein, a "fusion protein" includes a first protein moiety, e.g., a nucleic acid binding domain, having a peptide linkage with a second protein moiety. In certain aspects, the fusion protein is encoded by a single fusion gene.

"Domain" is used to describe a segment of a protein or nucleic acid. Unless otherwise indicated, a domain is not required to have any specific functional property.

As used herein, the term "gene therapy" refers to the introduction of extra genetic material into the total genetic material in a cell that restores, corrects, or modifies expression of a gene or gene product, or for the purpose of expressing a therapeutic polypeptide. In particular aspects, introduction of genetic material into the cell's genome for the purpose of expressing a therapeutic polypeptide is considered gene therapy.

<u>PROTEINS</u>

The recombinant DNA binding proteins (DBPs) of the present disclosure include repeat units that mediate binding to a nucleotide sequence in the regulatory region of a target gene in a cell. The target gene may be fetal $\gamma$-globin gene and the DBP may bind to a regulatory region of the gene.

The target gene may be fetal $\gamma$-globin gene and the DBP may displace a transcriptional repressor (TR) bound to a region in the fetal $\gamma$-globin gene promoter. The TR may be ZBTB7A. The target gene may be adult $\beta$-globin gene and the DBP may displace a transcriptional activator (TA) bound to a region, e.g., a CACC box in the adult $\beta$-globin gene promoter. The target gene may be BCL11A and the DBP may displace a TA bound to a region, e.g., a GATA site in the BCL11A gene promoter region. The DBP may be designed to bind a sequence comprising the binding site for the TF or a complement thereof and additional nucleotides present on one or both sides of the sequence. Accordingly, the DBP specifically binds to binding site for the TF in the target gene but not in other genes that are also regulated by binding of the TF but do not include the nucleotides present on one or both sides of the sequence.

The repeat units may be derived from DNA binding domains of proteins known to specifically bind to a nucleotide sequence. Such repeat units include zing fingers, megaTAL, repeat units from TALE protein, repeat units from DNA binding proteins from animal pathogens, and the like.

A recombinant DBP of the present invention may comprise a plurality of repeat units (RUs) ordered from N-terminus to C-terminus of the DBP to bind to the nucleic acid sequence CCTCTTGGGGGC (SEQ ID NO:201) or a complement thereof present in the fetal $\gamma$-globin gene promoter, wherein eleven of the RUs each comprise the sequence $X_{1-11}X_{12}X_{13}X_{14-33,\ 34,\ or35}$ (SEQ ID NO: 4), the last RU at the C-terminus is a half-repeat comprising the amino acid sequence $X_{1-11}X_{12}X_{13}X_{14-19,\ 20,\ or\ 21}$ (SEQ ID NO: 5), wherein: $X_{1-11}$ is a chain of 11 contiguous amino acids, $X_{14-33\ or\ 34\ or\ 35}$ is a chain of 20, 21 or 22 contiguous amino acids, $X_{14-20\ or\ 21\ or\ 22}$ is a chain of 7, 8 or 9 contiguous amino acids, $X_{12}X_{13}$ is selected from: (a) NH, HH, KH, NK, NQ, RH, RN, SS, NN, SN, KN, GN, VN, LN, DN, QN, EN, AN, or FN for binding to guanine (G); (b) NI, KI, RI, HI, CI, or SI for binding to adenine (A); (c) NG, HG, KG, RG, VG, IG, EG, MG, YG, AA, EP, VA, or QG for binding to thymine (T); (d) HD, RD, SD, ND, KD, AD, or YG for binding to cytosine (C); (e) NV or HN for binding to A or G; and (f) H*, HA, KA, N*, NA, NC, NS, RA, or S* for binding to A or T or G or C, wherein (*) means that the amino acid at X13 is absent.

The recombinant DBP may bind to the nucleic acid sequence CCTCTTGGGGGC (SEQ ID NO:201) and $X_{12}X_{13}$ in the 12 RUs from N-terminus to C-terminus may be HD, HD, NG, HD, NG, NG, NH, NH, NH, NH, NH, and HD.

The recombinant DBP may further comprise at least one additional RU at the N-terminus that binds to T such that the DBP binds to the nucleotide sequence: TCCTCTTGGGGGC (SEQ ID NO:200).

The recombinant DBP may further comprise at least one additional RU at the N-terminus that binds to A such that the DBP binds to the nucleotide sequence: ATCCTCTTGGGGGC (SEQ ID NO: 199). The $X_{12}X_{13}$ in the 14 RUs from N-terminus to C-terminus may be NI, NG, HD, HD, NG, HD, NG, NG, NH, NH, NH, NH, NH, and HD.

The recombinant DBP may further comprise at least one additional RU at the C-terminus that binds to C such that the DBP binds to the nucleotide sequence: ATCCTCTTGGGGGCC (SEQ ID NO: 198), where the one additional RU at the C-terminus that binds to C is positioned immediately before the half-repeat unit. The $X_{12}X_{13}$ in the RUs from N-terminus to C-terminus may be NI, NG, HD, HD, NG, HD, NG, NG, NH, NH, NH, NH, NH, HD, HD, and HD.

The plurality of RUs may be arranged from N-terminus to C-terminus in the recombinant DBP to bind to the nucleotide sequence: ATCCTCTTGGGGGCCC (SEQ ID NO:191); ATCCTCTTGGGGGCCCCT (SEQ ID NO:192); ATCCTCTTGGGGGCCCCTT (SEQ ID NO:193); ATCCTCTTGGGGGCCCCTTC (SEQ ID NO:194); ATCCTCTTGGGGGCCCCTTCC (SEQ ID NO:195); ATCCTCTTGGGGGCCCCTTCCC (SEQ ID NO:196); or ATCCTCTTGGGGGCCCCTTCCCC (SEQ ID NO:197).

The DBP may further comprise at one additional RU at the C-terminus that binds to C such that the DBP binds to the nucleotide sequence: CCTCTTGGGGGCC (SEQ ID NO: 190), wherein the one additional RU at the C-terminus that binds to C is positioned immediately before the half-repeat unit. The $X_{12}X_{13}$ in the RUs from N-terminus to C-terminus may be HD, HD, NG, HD, NG, NG, NH, NH, NH, NH, NH, HD, and HD. The plurality of RUs in the DBP may be arranged from N-terminus to C-terminus to bind to the nucleotide sequence: CCTCTTGGGGGCCCCTTCCCCAC (SEQ ID NO: 182); CCTCTTGGGGGCCCCTTCCCCA (SEQ ID NO: 183); CCTCTTGGGGGCCCCTTCCCC (SEQ ID NO: 184); CCTCTTGGGGGCCCCTTCC (SEQ ID NO:185); CCTCTTGGGGGCCCCTTC (SEQ ID NO:186); CCTCTTGGGGGCCCCT (SEQ ID NO:187); CCTCTTGGGGGCCCC (SEQ ID NO:188); or CCTCTTGGGGGCCCC (SEQ ID NO:189).

Also disclosed are additional recombinant DBPs. Another recombinant DBP of the present invention may comprise a plurality of repeat units (RUs) ordered from N-terminus to C-terminus of the DBP to bind to the nucleic acid sequence CACCCTGTGGAGCCACAC (SEQ ID NO:181) or a complement thereof present in the adult β-globin (HBB) gene promoter, where seventeen of the RUs each comprise the sequence $X_{1-11}X_{12}X_{13}X_{14-33,\ 34,\ or\ 35}$ (SEQ ID NO: 4), the last RU at the C-terminus is a half-repeat comprising the amino acid sequence $X_{1-11}X_{12}X_{13}X_{14-19,\ 20,\ or\ 21}$ (SEQ ID NO: 5), where: $X_{1-11}$ is a chain of 11 contiguous amino acids, $X_{14-33\ or\ 34\ or\ 35}$ is a chain of 20, 21 or 22 contiguous amino acids, $X_{14-20\ or\ 21\ or\ 22}$ is a chain of 7, 8 or 9 contiguous amino acids, $X_{12}X_{13}$ is selected from: (a) NH, HH, KH, NK, NQ, RH, RN, SS, NN, SN, KN, GN, VN, LN, DN, QN, EN, AN, or FN for binding to guanine (G); (b) NI, KI, RI, HI, CI, or SI for binding to adenine (A); (c) NG, HG, KG, RG, VG, IG, EG, MG, YG, AA, EP, VA, or QG for binding to thymine (T); (d) HD, RD, SD, ND, KD, AD, or YG for binding to cytosine (C); (e) NV or HN for binding to A or G; and (f) H*, HA, KA, N*, NA, NC, NS, RA, or S* for binding to A or T or G or C, wherein (*) means that the amino acid at $X_{13}$ is absent. The DBP may bind to the nucleic acid sequence CACCCTGTGGAGCCACAC (SEQ ID NO:181) and the $X_{12}X_{13}$ in the RUs from N-terminus to C-terminus may be HD, NI, HD, HD, HD, NG, NH, NG, NH, NH, NI, NH, HD, HD, NI, HD, NI, and HD.

Another recombinant DBP of the present invention may comprise a plurality of repeat units (RUs) ordered from N-terminus to C-terminus of the DBP to bind to the nucleic acid sequence TGCTTTTATCACAGGCT (SEQ ID NO:180) or a complement thereof present in the BCL11A gene promoter, where sixteen of the RUs each comprise the sequence $X_{1-11}X_{12}X_{13}X_{14-33,\ 34,\ or\ 35}$ (SEQ ID NO: 4), the last RU at the C-terminus is a half-repeat comprising the amino acid sequence $X_{1-11}X_{12}X_{13}X_{14-19,\ 20,\ or\ 21}$ (SEQ ID NO: 5), wherein: $X_{1-11}$ is a chain of 11 contiguous amino acids, $X_{14-33\ or\ 34\ or\ 35}$ is a chain of 20, 21 or 22 contiguous amino acids, $X_{14-20\ or\ 21\ or\ 22}$ is a chain of 7, 8 or 9 contiguous amino acids, $X_{12}X_{13}$ is selected from: (a) NH, HH, KH, NK, NQ, RH, RN, SS, NN, SN, KN, GN, VN, LN, DN, QN, EN, AN, or FN for binding to guanine (G); (b) NI, KI, RI, HI, CI, or SI for binding to adenine (A); (c) NG, HG, KG, RG, VG, IG, EG, MG, YG, AA, EP, VA, or QG for binding to thymine (T); (d) HD, RD, SD, ND, KD, AD, or YG for binding to cytosine (C); (e) NV or HN for binding to A or G; and (f) H*, HA, KA, N*, NA, NC, NS, RA, or S* for binding to A or T or G or C, wherein (*) means that the amino acid at $X_{13}$ is absent. The DBP may bind to the nucleic acid sequence TGCTTTTATCACAGGCT (SEQ ID NO: 180) and the $X_{12}X_{13}$ in the RUs from N-terminus to C-terminus may be NG, NH, HD, NG, NG, NG, NG, NI, NG, HD, NI, HD, NI, NH, NH, HD, and NG.

The DBP may comprise a plurality of RUs where the number of the RUs ranges from 12 to 40, 13 to 35, 14 to 30, 12 to 28, 12 to 27, 12 to 26, 12 to 25, 12 to 24, 13 to 25, 13 to 24, 14 to 25, 14 to 24, 15 to 25, 15 to 24, 16 to 25, 16 to 24, 17 to 25, or 17 to 24.

The DBP may comprise a plurality of RUs where the plurality of RUs consist of a specified number of RUs. For example, a DBP that binds to the sequence ATCCTCTTGGGGGCCC (SEQ ID NO: 191) consists of 16 RUs, where the last RU at the C-terminus may be a half-repeat unit. Unless specified otherwise, the term repeat unit includes a half RU. Alternatively, a half-repeat unit may be referred to as 0.5RU. Thus, for example, the DBP that binds to the sequence ATCCTCTTGGGGGCCC (SEQ ID NO:191) can consist of 15.5 RUs. The DBP that binds to the sequence ATCCTCTTGGGGGCC (SEQ ID NO: 198) may consist of 14.5 RUs. The DBP that binds to the sequence ATCCTCTTGGGGGC (SEQ ID NO:199) may consist of 13.5 RUs.

The DBP that binds to the sequence CCTCTTGGGGGCCCCTTCC (SEQ ID NO:185) may consist of 18.5 RUs. The DBP that binds to the sequence CCTCTTGGGGGCCCCTTC (SEQ ID NO:186) may consist of 17.5 RUs. The DBP that binds to the sequence CCTCTTGGGGGCCCCTT (SEQ ID NO:211) may consist of 16.5 RUs. The DBP that binds to the sequence TCCTCTTGGGGGCCCCT (SEQ ID NO:175) may consist of 15.5 RUs. The DBP that binds to the sequence CCTCTTGGGGGCCCC (SEQ ID NO:188) may consist of 14.5 RUs. The DBP that binds to the sequence CCTCTTGGGGGCCC (SEQ ID NO:189) may consist of 13.5 RUs.

As noted herein, the RUs may be derived from any source, such as, transcription activator-like effector (TALE) proteins from *Xanthomonas, Burkholderia,* or *Paraburkholderia.* or DBPs comprising repeat units from *Legionella.*

**TALE Derived DBP**

In examples where the RUs are derived from RUs of TALE proteins, $X_{1-11}$ is at least 80% identical, at least 90% identical, or 100% identical to LTPEQVVAIAS (SEQ ID NO: 6). $X_{14-20 \text{ or } 21 \text{ or } 22}$ is at least 80% identical to GGRPALE (SEQ ID NO: 7).

The RUs may each comprise 33-36 amino acid long sequence having at least 80% sequence identity to the amino acid sequence:

LTPDQVVAIASX$^{12}$X$^{13}$GGKQALETVQRLLPVL QDHG (SEQ ID NO: 1), or having the sequence of SEQ ID NO:1 with one or more conservative amino acid substitutions thereto; wherein $X_{12}X_{13}$ is HH, KH, NH, NK, NQ, RH, RN, SS, NN, SN, KN, NI, KI, RI, HI, SI, NG, HG, KG, RG, RD, SD, HD, ND, KD, YG, YK, NV, HN, H\*, HA, KA, N\*, NA, NC, NS, RA, CI, or S\*, where (\*) means $X_{13}$ is absent.

In certain aspects, the RUs may comprise a 33-36 amino acid long sequence having a sequence at least 85%, at least 90%, at least 91%, at least 92%, at least 93%, at least 94%, at least 95%, or more, or identical to SEQ ID NO: 1.

In certain aspects, the RUs and the half-RU are derived from Xanthomonas TALE. In certain aspects, $X_{1-11}$ is at least 80%, at least 90%, or 100% identical to LTPEQVVAIAS (SEQ ID NO: 6), LTPAQVVAIAS (SEQ ID NO: 9), LTPDQVVAIAN (SEQ ID NO: 10), LTPDQVVAIAS (SEQ ID NO: 11), LTPYQVVAIAS (SEQ ID NO: 12), LTREQVVAIAS (SEQ ID NO: 13), or LSTAQVVAIAS (SEQ ID NO: 14).

In certain aspects, $X_{14-20 \text{ or } 21 \text{ or } 22}$ is at least 80%, at least 90%, at least 95%, or 100% identical to GGKQALETVQRLLPVLCQDHG (SEQ ID NO: 15), GGKQALATVQRLLPVLCQDHG (SEQ ID NO: 16), GGKQALETVQRVLPVLCQDHG (SEQ ID NO: 17), or GGKQALETVQRVLPVLCQDHG (SEQ ID NO: 17).

In certain aspects, the DBP may include a plurality of RUs ordered from N-terminus to C-terminus of the DBP. For example, the DBP may include 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 RUs. In certain aspects, the DBP may include a plurality of RUs of naturally occurring transcription activator like effector (TALE) proteins, such as RUs from *Xanthomonas* or *Ralstonia* TALE proteins.

In certain aspects, one or more RUs in a DBP may be at least 80%, 85%, 90%, 91%, 92%, 93%, 94%, 95%, 96%, 97%, 98%, or 99%, or a 100% identical to a RU provided herein. Percent identity between a pair of sequences may be calculated by multiplying the number of matches in the pair by 100 and dividing by the length of the aligned region, including gaps. Identity scoring only counts perfect matches and does not consider the degree of similarity of amino acids to one another. Only internal gaps are included in the length, not gaps at the sequence ends.

$$\text{Percent Identity} = (\text{Matches} \times 100)/\text{Length of aligned region (with gaps)}$$

The phrase "conservative amino acid substitution" refers to substitution of amino acid residues within the following groups: 1) L, I, M, V, F; 2) R, K; 3) F, Y, H, W, R; 4) G, A, T, S; 5) Q, N; and 6) D, E. Conservative amino acid substitutions may preserve the activity of the protein by replacing an amino acid(s) in the protein with an amino acid with a side chain of similar acidity, basicity, charge, polarity, or size of the side chain.

Guidance for substitutions, insertions, or deletions may be based on alignments of amino acid sequences of proteins from different species or from a consensus sequence based on a plurality of proteins having the same or similar function.

In certain aspects, the disclosed DBP may include a nuclear localization sequence (NLS) to facilitate entry into an organelle of a cell, e.g. the nucleus of a cell, e.g., an animal or a plant cell. In certain aspects, the disclosed DBP may include a half-RU or a partial RU that is 15-20 amino acid long sequence. Such a half-RU may be included after the last RU present in the DBP and may be derived from a RU identified in Xanthomonas or Ralstonia TALE protein.

The disclosed DBP may include an N-terminus region. The N-terminus region may be the N-cap domain or a fragment thereof from TALE proteins like those expressed in *Burkholderia, Paraburkholderia, or Xanthomonas.* In certain aspects, the disclosed DBP may include a C-terminus region. The C-terminus region may be a C-cap domain or a fragment thereof from TALE proteins like those expressed in *Burkholderia, Paraburkholderia, or Xanthomonas.*

In certain aspects, the N-terminus region may be at least 80%, 85%, 90%, 91%, 92%, 93%, 94%, 95%, 96%, 97%, 98%, or 99%, or a 100% identical to the N-terminus region sequence provided in Table 2. This amino acid sequence includes a M added to the N-terminus which is not present in the wild type N-cap region of a Xanthomonas TALE protein.

This amino acid sequence is generated by deleting amino acids N+288 through N+137 of the N-terminus region of a TALE protein, adding a M, such that amino acids N+136 through N+1 of the N-terminus region of the TALE protein are present.

In some aspects, the N-terminus region can be a truncated version of the wild type N-cap region such that the N-terminus region includes amino acids from position 1 (N) through position 120 (K) of the naturally occurring *Xanthomonas spp.*-N-cap region and has the follow sequence:

KPKVRSTVAQHHEALVGHGFTHAHIVALSQHPAALGTVAVKYQDMIAALPEATHEAIV

GVGKQWSGARALEALLTVAGELRGPPLQLDTGQLLKIAKRGGVTAVEAVHAWRNALT

GAPLN (SEQ ID NO: 18).

In some aspects, the N-cap region can be truncated such that the N-terminus region includes amino acids from position 1 (N) through position 115 (S) of the naturally occurring *Xanthomonas spp.*- N-cap region and has the follow sequence:

STVAQHHEALVGHGFTHAHIVALSQHPAALGTVAVKYQDMIAALPEATHEAIVGVGK

QWSGARALEALLTVAGELRGPPLQLDTGQLLKIAKRGGVTAVEAVHAWRNALTGAPL

N (SEQ ID NO: 19).

In some aspects, the N-cap region can be truncated such that the N-terminus region includes amino acids from position 1 (N) through position 110 (H) of the naturally occurring *Xanthomonas spp.*-N-cap region and has the follow sequence:

HHEALVGHGFTHAHIVALSQHPAALGTVAVKYQDMIAALPEATHEAIVGVGKQWSGA

RALEALLTVAGELRGPPLQLDTGQLLKIAKRGGVTAVEAVHAWRNALTGAPLN (SEQ

ID NO: 20).

In certain aspects, the DBP may include a C-terminus region at C-terminus of the recombinant polypeptide which C-terminus region is derived from the C-cap region of a Xanthomonas TALE protein and follows the last half-RU. In certain aspects, the C-terminus region at the C-terminus may be linked to the last half-RU via a linker.

In certain aspects, the C-terminus region may be at least 80%, 85%, 90%, 91%, 92%, 93%, 94%, 95%, 96%, 97%, 98%, or 99%, or a 100% identical to the C-terminus region sequence provided in Table 2.

In certain aspects, the RUs are derived from Xanthomonas TALEs. In plant genomes, such as Xanthomonas, the natural TALE-binding sites begin with a thymine (T), which may be specified by a cryptic signal within the non-repetitive N-terminus region of the TALE polypeptide; in some cases this region may be referred to as repeat 0. In animal genomes, TALE binding sites do not necessarily have to begin with a thymine (T) and recombinant DBP disclosed herein may target DNA sequences that begin with T, A, G or C. In certain aspects, the recombinant DBP disclosed herein may target DNA sequences that begin with T. The tandem repeat of TALE RUs ends with a half-length repeat or a stretch of sequence that may share identity with only the first 20 amino acids of a repetitive full length TALE RU and this half repeat may be referred to as a half-monomer, a half RU, or a half repeat. Therefore, it follows that the length of the DNA sequence being targeted by DBP derived from TALEs is equal to the number of full RUs plus two. Thus, for example, DBP may be engineered to include X number (e.g., 9, 10, 11, 12, 13, 14, 15, 16, 17, 18, 19, 20, 21, 22, 23, 24, 25, or 26) full length RUs that are specifically ordered or arranged to target nucleic acid sequences of X+2 length (e.g., 11, 12, 13, 14, 15, 16, 17, 18, 19, 20, 21, 22, 23, 24, 25, 26, 27, or 28 nucleotides, respectively), with the N-terminus region binding "T" and the last RU being a half-repeat.

In certain aspects, a Xanthomonas spp.-derived repeat units can have a sequence of LTPDQVVAIASNHGGKQALETVQRLLPVLCQDHG (SEQ ID NO: 21) comprising an RVD of NH, which recognizes guanine. A Xanthomonas spp.-derived repeat units can have a sequence of LTPDQVVAIASNGGGKQALETVQRLLPVLCQDHG (SEQ ID NO: 22) comprising an RVD of NG, which recognizes thymidine. A Xanthomonas spp.-derived repeat units can have a sequence of LTPDQVVAIASNIGGKQALETVQRLLPVLCQDHG (SEQ ID NO: 23) comprising an RVD of NI, which recognizes adenosine. A Xanthomonas spp.-derived repeat units can have a sequence of LTPDQVVAIASHDGGKQALETVQRLLPVLCQDHG (SEQ ID NO: 24) comprising an RVD of HD, which recognizes cytosine.

In some aspects, expression of the target gene (e.g., HBB or BCL11A) can be reduced by at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 92%, at least 95%, at least 97%, or at least 99% by using a DBP that displaces a transcriptional activator as compared to untreated cells.

In some aspects, expression of the target gene (e.g., HBG) can be increased by at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 92%, at least 95%, at least 97%, or at least 99% by using a DNA binding polypeptide that displaces a transcriptional repressor as compared to untreated cells.

In some aspects, modulation of gene expression (e.g., repression or activation of the target gene) by a DNA binding polypeptide that displaces a transcriptional factor can last for at least 1 day, at least 2 days, at least 3 days, at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, at least 18 days, at least 19 days, at least 20 days, at least 21 days, at least 22 days, at least 23 days, at least 24 days, at least 25 days, at least 26 days, at least 27 days, at least 28 days, 1 days to 3 days, 3 days to 5 days, 5 days to 7 days, 7 days to 9 days, 9 days to 11 days, 11 days to 13 days, 13 days to 15 days, 15 days to 17 days, 17 days to 19 days, 19 days to 21 days, 21 days to 23 days, 23 days to 25 days, or 25 days to 28 days.

As used herein, the amount of HBG expression can refer to total amount of HBG measured using measurement of the HBG protein or measurement of mRNA or the total amount of HBG normalized to the total amount of HBB or the total amount of HBG normalized to the total amount of HBB and HBG.

## DBP derived from Ralstonia

In certain aspects, the RUs and one or both N-terminus and C-terminus regions may be derived from a transcription activator like effector-like protein (TALE-like protein) of *Ralstonia solanacearum.* Repeat units derived from *Ralstonia solanacearum* can be 33-35 amino acid residues in length. In some aspects, the repeat can be derived from the naturally occurring *Ralstonia solanacearum* TALE-like protein.

As noted herein, the RUs may have the sequence $X_{1-11}X_{12}X_{13}X_{14-33,\ 34,\ or\ 35}$ (SEQ ID NO: 4), where $X_{1-11}$ is a chain of 11 contiguous amino acids, $X_{14-33\ or\ 34\ or\ 35}$ is a chain of 20, 21 or 22 contiguous amino acids, $X_{12}X_{13}$ is RVD and is selected from: (a) NH, HH, KH, NK, NQ, RH, RN, SS, NN, SN, or KN for recognition of guanine (G); (b) NI, KI, RI, HI, or SI for recognition of adenine (A); (c) NG, HG, KG, or RG for recognition of thymine (T); (d) HD, RD, SD, ND, KD, or YG for recognition of cytosine (C); and (e) NV or HN for recognition of A or G; and (f) H*, HA, KA, N*, NA, NC, NS, RA, or S*for recognition of A or T or G or C, wherein (*) means that the amino acid at $X_{13}$ is absent. In certain aspects, $X_{1-11}$ may include a stretch of amino acids at least 80%, at least 90%, or a 100% identical to the $X_{1-11}$ residues of the following RUs from Ralstonia. In certain aspects, $X_{14-33\ or\ 34\ or\ 35}$ may include a stretch of 20, 21, or 22 amino acids at least 80%, at least 90%, or a 100% identical to the $X_{14-33\ or\ 34\ or\ 35}$ residues of the following RUs from Ralstonia: LDTFQVVAIASHNGGKQALEAVKADLLDLLGAPYV (SEQ ID NO: 26), LNTEQVVAVASNKGGKQALEAVGAQLLALRAVPYE (SEQ ID NO: 27), LSTAQVAAIASHDGGKQALEAVGTQLVVLRAAPYA (SEQ ID NO: 28), LSTAQVVAVAGRNGGKQALEAVRAQLPALRAAPYG (SEQ ID NO: 29), or LSTAQVVAVASSNGGKQALEAVWALLPVLRATPYD (SEQ ID NO: 30).

In certain aspects, a *Ralstonia solanacearum*- repeat unit can have at least 80% sequence identity with any one of the Ralstonia RUs provided herein.

In certain aspects, the DBP may include a N-cap region at the N-terminus which may be present immediately adjacent the first RU or may be linked to the first RU via a linker. In some aspects, an DBP of the present disclosure can have the full length naturally occurring N-terminus of a naturally occurring *Ralstonia solanacearum-derived* protein. In some aspects, any truncation of the full length naturally occurring N-terminus of a naturally occurring *Ralstonia solanacearum-derived* protein can be used at the N-terminus of a DBP of the present disclosure. For example, in some aspects, amino acid residues at positions 1 (H) to position 137 (F) of the naturally occurring *Ralstonia solanacearum-derived* protein N-terminus can be used as the N-cap region. In particular aspects, the truncated N-terminus from position 1 (H) to position 137 (F) can have a sequence as follows: FGKLVALGYSREQIRKLKQESLSEIAKYHTTLTGQGFTHADI CRISRRRQSLRVVARNYP ELAAALPELTRAHIVDIARQRSGDLALQALLPVATALTAAPLRLSASQIATVAQYGERP AI-QALYRLRRKLTRAPLH (SEQ ID NO: 31). In some aspects, the naturally occurring N-terminus of *Ralstonia solanacearum* can be truncated to any length and used as the N-cap of the engineered DNA binding polypeptide. For example, the naturally occurring N-terminus of *Ralstonia solanacearum* can be truncated to include amino acid residues at position 1 (H) to position 120 (K) as follows: KQESLSEIAKYHTTLTGQGFTHADICRISRRRQSLRVVARNYPELAAALPELTRAHIVDI ARQRSGDLALQALLPVATALTAAPLRLSASQIATVAQYGERPAIQALYRLRRKLTRAPL H (SEQ ID NO: 32) and used as the N-cap of the DBP. The naturally occurring N-terminus of *Ralstonia solanacearum* can be truncated amino acid residues to include positions 1 to 115 and used at the N-cap of the engineered DNA binding domain. The naturally occurring N-terminus of *Ralstonia solanacearum* can be truncated to amino acid residues at positions 1 to 50, 1 to 70, 1 to 100, 1 to 120, 1 to 130, 10 to 40, 60 to 100, or 100 to 120 and used as the N-cap of the engineered DNA binding domain. As noted for N-cap region derived from Xanthomonas TALE, the amino acid residues are numbered backward from the first repeat unit such that the amino acid (H in this case) of the N-cap adjacent the first RU is numbered 1 while the N-terminal amino acid of the N-cap is numbered 137 (and is F in this case) or 120 (and is K in this case).

In some aspects, the N-cap, referred to as the amino terminus or the "NH2" domain, can recognize a guanine. In some aspects, the N-cap can be engineered to bind a cytosine, adenosine, thymidine, guanine, or uracil.

In some aspects, an DBP of the present disclosure can include a plurality of RUs followed by a final single half-repeat also derived from *Ralstonia solanacearum.* The half repeat can have 15 to 23 amino acid residues, for example, the half repeat can have 19 amino acid residues. In particular aspects, the half-repeat can have a sequence as follows: LSTAQVVAIACISGQQALE (SEQ ID NO: 33).

In some aspects, an DBP of the present disclosure can have the full length naturally occurring C-terminus of a naturally occurring *Ralstonia solanacearum*-derived protein as a C-cap region that is conjugated to the last RU. In some aspects, any truncation of the full length naturally occurring C-terminus of a naturally occurring *Ralstonia solanacearum-derived* protein can be used as the C-cap. For example, in some aspects, the DBP can comprise amino acid residues at position 1 (A) to position 63 (S) as follows: AIEAHMPTLRQASHSLSPERVAAIACIGGRSAVEAVRQGLPVKAIRRIRRE KAPVAGPPP AS (SEQ ID NO: 34) of the naturally occurring *Ralstonia solanacearum*-derived protein C-terminus. In some aspects, the naturally occurring C-terminus of *Ralstonia solanacearum* can be truncated to any length and used as the C-cap of the DBP. For example, the naturally occurring C-terminus of *Ralstonia solanacearum* can be truncated to amino acid residues at positions 1 to 63 and used as the C-terminus of the DBP. The naturally occurring C-terminus of *Ralstonia solanacearum* can be truncated amino acid residues at positions 1 to 50 and used as the C-cap of the DBP. The naturally occurring C-terminus of *Ralstonia solanacearum* can be truncated to amino acid residues at positions 1 to 63, 1 to 50, 1 to 70, 1 to 100, 1 to 120, 1 to 130, 10 to 40, 60 to 100, or 100 to 120 and used as the C-cap of the DBP. Exemplary sequences of domains of a DBP as disclosed herein are as follows:

| Description | Sequence |
|---|---|
| Truncated N-terminus; positions 1 (H) to 115 (S) of the naturally occurring *Ralstonia solanacearum*-derived protein N-terminus | SEIAKYHTTLTGQGFTHADICRISRRRQSLRVVARNYPELAAALP ELTRAHIVDIARQRSGDLALQALLPVATALTAAPLRLSASQIATV AQYGERPAIQALYRLRRKLTRAPLH (SEQ ID NO: 35) |
| Truncated N-terminus; positions 1 (H) to 137 (F) of the naturally occurring *Ralstonia solanacearum*-derived protein N-terminus | FGKLVALGYSREQIRKLKQESLSEIAKYHTTLTGQGFTHADICRI SRRRQSLRVVARNYPELAAALPELTRAHIVDIARQRSGDLALQA LLPVATALTAAPLRLSASQIATVAQYGERPAIQALYRLRRKLTR APLH (SEQ ID NO: 31) |
| Truncated N-terminus: positions 1 (H) to 120 (K) of the naturally occurring *Ralstonia solanacearum*-derived protein N-terminus | KQESLSEIAKYHTTLTGQGFTHADICRISRRRQSLRVVARNYPEL AAALPELTRAHIVDIARQRSGDLALQALLPVATALTAAPLRLSAS QIATVAQYGERPAIQALYRLRRKLTRAPLH (SEQ ID NO: 32) |
| Half-repeat | LSTAQVVAIACISGQQALE (SEQ ID NO: 33) |
| Truncated C-terminus: positions 1 (A) to 63 (S) of the naturally occurring *Ralstonia solanacearum-derived* protein C-terminus | AIEAHMPTLRQASHSLSPERVAAIACIGGRSAVEAVRQGLPVKAI RRIRREKAPVAGPPPAS (SEQ ID NO: 34) |

## DBP derived from Animal Pathogens

In some aspects, the present disclosure provides DNA binding polypeptide in which the repeat units can be derived from a *Legionellales* bacterium, a species of the genus of *Legionella,* such as *L. quateirensis* or *L. maceachernii,* the genus of *Burkholderia,* the genus of *Paraburkholderia,* or the genus of *Francisella.*

As noted herein, the RUs may have the sequence $X_{1-11}X_{12}X_{13}X_{14-33,\ 34,\ or\ 35}$ (SEQ ID NO: 4), where $X_{1-11}$ is a

chain of 11 contiguous amino acids, $X_{14-33 \text{ or } 34 \text{ or } 35}$ is a chain of 20, 21 or 22 contiguous amino acids, $X_{12}X_{13}$ is selected from: (a) NH, HH, KH, NK, NQ, RH, RN, SS, NN, SN, HN, or KN for recognition of guanine (G); (b) NI, KI, RI, HI, HA, or SI for recognition of adenine (A); (c) NG, HG, KG, or RG for recognition of thymine (T); (d) HD, RD, SD, ND, KD, or YG for recognition of cytosine (C); and (e) NV or HN for recognition of A or G; and (f) H*, HA, KA, N*, NA, NC, NS, RA, or S* for recognition of A or T or G or C, wherein (*) means that the amino acid at $X_{13}$ is absent. In certain aspects, $X_{1-11}$ may include a stretch of amino acids at least 80%, at least 90%, or a 100% identical to the $X_{1-11}$ residues of the following RUs from animal pathogens, *Legionella, Burkholderia, Paraburkholderia, or Francisella.* In certain aspects, $X_{14-33, 34, \text{ or } 35}$ may include a stretch of 20, 21, or 22 amino acids at least 80%, at least 90%, or a 100% identical to the $X_{14-33, 34, \text{ or } 35}$ residues of the RUs from animal pathogens, *Legionella* (e.g., *L. quateirensis or L. maceachernii), Burkholderia, Paraburkholderia, or Francisella* listed below.

| Organism | Repeat Unit Sequence ($X_{1-11}X_{12}X_{13}X_{14-33, 34, \text{ or } 35}$) | SEQ ID | BCR ($X_{12}X_{13}$) |
|---|---|---|---|
| *L. quateirensis* | FSSQQIIRMVSHAGGANNLKAVTANHDDLQNMG | 37 | HA |
| *L. quateirensis* | FNVEQIVRMVSHNGGSKNLKAVTDNHDDLKNMG | 38 | HN |
| *L. quateirensis* | FNAEQIVRMVSHGGGSKNLKAVTDNHDDLKNMG | 39 | HG |
| *L. quateirensis* | FNAEQIVSMVSNNGGSKNLKAVTDNHDDLKNMG | 40 | NN |
| *L. quateirensis* | FNAEQIVSMVSNGGGSLNLKAVKKYHDALKDRG | 41 | NG |
| *L. quateirensis* | FNTEQIVRMVSHDGGSLNLKAVKKYHDALRERK | 42 | HD |
| *L. quateirensis* | FNVEQIVSIVSHGGGSLNLKAVKKYHDVLKDRE | 43 | HG |
| *L. quateirensis* | FNAEQIVRMVSHDGGSLNLKAVTDNHDDLKNMG | 44 | HD |
| *L. maceachernii* | FSAEQIVRIAAHDGGSRNIEAVQQAQHVLKELG | 45 | HD |
| *L. maceachernii* | FSAEQIVSIVAHDGGSRNIEAVQQAQHILKELG | 46 | HD |
| *Legionellales bacterium* | LDRQQILRIASHDGGSKNIAAVQKFLPKLMNFG | 47 | HD |
| *L. maceachernii* | FSAEQIVRIAAHDGGSLNIDAVQQAQQALKELG | 48 | HD |
| *L. maceachernii* | FSTEQIVCIAGHGGGSLNIKAVLLAQQALKDLG | 49 | HG |
| *L. maceachernii* | YSSEQIVRVAAHGGGSLNIKAVLQAHQALKELD | 50 | HG |
| *L. maceachernii* | FSAEQIVHIAAHGGGSLNIKAILQAHQTLKELN | 51 | HG |
| *L. maceachernii* | FSAEQIVRIAAHIGGSRNIEAIQQAHHALKELG | 52 | HI |
| *L. maceachernii* | FSAEQIVRIAAHIGGSHNLKAVLQAQQALKELD | 53 | HI |
| *L. maceachernii* | FSAKHIVRIAAHIGGSLNIKAVQQAQQALKELG | 54 | HI |
| *L. quateirensis* | FNAEQIVRMVSHKGGSKNLALVKEYFPVFSSFH | 55 | HK |
| *L. maceachernii* | FSADQIVRIAAHKGGSHNIVAVQQAQQALKELD | 56 | HK |
| *L. maceachernii* | FSAEQIVSIAAHVGGSHNIEAVQKAHQALKELD | 57 | HV |
| *Burkholderia* | FSSGETVGATVGAGGTETVAQGGTASNTTVSSG | 58 | GA |
| *Burkholderia* | FSGGMATSTTVGSGGTQDVLAGGAAVGGTVGTG | 59 | GS |
| *Burkholderia* | FSAADIVKIAGKIGGAQALQAFITHRAALIQAG | 60 | KI |
| *Burkholderia* | FNPTDIVKIAGNDGGAQALQAVLELEPALRERG | 61 | ND |
| *Burkholderia* | FNPTDIVRMAGNDGGAQALQAVFELEPAFRERS | 62 | ND |
| *Burkholderia* | FNPTDIVRMAGNDGGAQALQAVLELEPAFRERG | 63 | ND |
| *Burkholderia* | FSQVDIVKIASNDGGAQALYSVLDVEPTFRERG | 64 | ND |
| *Burkholderia* | FSRADIVKIAGNDGGAQALYSVLDVEPPLRERG | 65 | ND |

(continued)

| Organism | Repeat Unit Sequence ($X_{1-11}X_{12}X_{13}X_{14-33, 34, or 35}$) | SEQ ID | BCR ($X_{12}X_{13}$) |
|---|---|---|---|
| *Burkholderia* | FSRGDIVKIAGNDGGAQALYSVLDVEPPLRERG | 66 | ND |
| *Burkholderia* | FNRADIVRIAGNGGGAQALYSVRDAGPTLGKRG | 67 | NG |
| *Burkholderia* | FRQADIVKIASNGGSAQALNAVIKLGPTLRQRG | 68 | NG |
| *Burkholderia* | FRQADIVKMASNGGSAQALNAVIKLGPTLRQRG | 69 | NG |
| *Burkholderia* | FSRADIVKIAGNGGGAQALQAVLELEPTFRERG | 70 | NG |
| *Burkholderia* | FSRADIVRIAGNGGGAQALYSVLDVGPTLGKRG | 71 | NG |
| *Burkholderia* | FSRGDIVRIAGNGGGAQALQAVLELEPTLGERG | 72 | NG |
| *Burkholderia* | FSRADIVKIAGNGGGAQALQAVITHRAALTQAG | 73 | NG |
| *Burkholderia* | FSRGDTVKIAGNIGGAQALQAVLELEPTLRERG | 74 | NI |
| *Burkholderia* | FNPTDIVKIAGNIGGAQALQAVLELEPAFRERG | 75 | NI |
| *Burkholderia* | FSAADIVKIAGNIGGAQALQAIFTHRAALIQAG | 76 | NI |
| *Burkholderia* | FSAADIVKIAGNIGGAQALQAVITHRATLTQAG | 77 | NI |
| *Burkholderia* | FSATDIVKIASNIGGAQALQAVISRRAALIQAG | 78 | NI |
| *Burkholderia* | FSQPDIVKIAGNIGGAQALQAVLELEPAFRERG | 79 | NI |
| *Burkholderia* | FSRADIVKIAGNIGGAQALQAVLELESTFRERS | 80 | NI |
| *Burkholderia* | FSRADIVKIAGNIGGAQALQAVLELESTLRERS | 81 | NI |
| *Burkholderia* | FSRGDIVKMAGNIGGAQALQAGLELEPAFRERG | 82 | NI |
| *Burkholderia* | FSRGDIVKMAGNIGGAQALQAVLELEPAFHERS | 83 | NI |
| *Burkholderia* | FTLTDIVKMAGNIGGAQALKAVLEHGPTLRQRD | 84 | NI |
| *Burkholderia* | FTLTDIVKMAGNIGGAQALKVVLEHGPTLRQRD | 85 | NI |
| *Burkholderia* | FNPTDIVKIAGNNGGAQALQAVLELEPALRERG | 86 | NN |
| *Burkholderia* | FNPTDIVKIAGNNGGAQALQAVLELEPALRERS | 87 | NN |
| *Burkholderia* | FNPTDMVKIAGNNGGAQALQAVLELEPALRERG | 88 | NN |
| *Burkholderia* | FSAADIVKIASNNGGAQALQALIDHWSTLSGKT | 89 | NN |
| *Burkholderia* | FSAADIVKIASNNGGAQALQAVISRRAALIQAG | 90 | NN |
| *Burkholderia* | FSAADIVKIASNNGGAQALQAVITHRAALAQAG | 91 | NN |
| *Burkholderia* | FSAADIVKIASNNGGARALQALIDHWSTLSGKT | 92 | NN |
| *Burkholderia* | FTLTDIVEMAGNNGGAQALKAVLEHGSTLDERG | 93 | NN |
| *Burkholderia* | FTLTDIVKMAGNNGGAQALKAVLEHGPTLDERG | 94 | NN |
| *Burkholderia* | FTLTDIVKMAGNNGGAQALKVVLEHGPTLRQRG | 95 | NN |
| *Burkholderia* | FTLTDIVKMASNNGGAQALKAVLEHGPTLDERG | 96 | NN |
| *Burkholderia* | FSAADIVKIAGNSGGAQALQAVISHRAALTQAG | 97 | NS |
| *Burkholderia* | FSGGDAVSTVVRSGGAQSVASGGTASGTTVSAG | 98 | RS |
| *Burkholderia* | FRQTDIVKMAGSGGSAQALNAVIKHGPTLRQRG | 99 | SG |
| *Burkholderia* | FSLIDIVEIASNGGAQALKAVLKYGPVLTQAGR | 100 | SN |
| *Burkholderia* | FSGGDAAGTVVSSGGAQNVTGGLASGTTVASGG | 101 | SS |
| *Paraburkholderia* | FNLTDIVEMAANSGGAQALKAVLEHGPTLRQRG | 102 | NS |
| *Paraburkholderia* | FNRASIVKIAGNSGGAQALQAVLKHGPTLDERG | 103 | NS |
| *Paraburkholderia* | FSQANIVKMAGNSGGAQALQAVLDLELVFRERG | 104 | NS |

(continued)

| Organism | Repeat Unit Sequence ($X_{1-11}X_{12}X_{13}X_{14-33,\ 34,\ or\ 35}$) | SEQ ID | BCR ($X_{12}X_{13}$) |
|---|---|---|---|
| *Paraburkholderia* | FSQPDIVKMAGNSGGAQALQAVLDLELAFRERG | 105 | NS |
| *Paraburkholderia* | FSLIDIVEIASNGGAQALKAVLKYGPVLMQAGR | 106 | SN |
| *Francisella* | YKSEDIIRLASHDGGSVNLEAVLRLHSQLTRLG | 107 | HD |
| *Francisella* | YKPEDIIRLASHGGGSVNLEAVLRLNPQLIGLG | 108 | HG |
| *Francisella* | YKSEDIIRLASHGGGSVNLEAVLRLHSQLTRLG | 109 | HG |
| *Francisella* | YKSEDIIRLASHGGGSVNLEAVLRLNPQLIGLG | 110 | HG |
| *L. quateirensis* | LGHKELIKIAARNGGGNNLIAVLSCYAKLKEMG | 111 | RN |
| *Paraburkholderia* | FNLTDIVEMAGKGGGAQALKAVLEHGPTLRQRG | 112 | KG |
| *Paraburkholderia* | FRQADIIKIAGNDGGAQALQAVIEHGPTLRQHG | 113 | ND |
| *Paraburkholderia* | FSQADIVKIAGNDGGTQALHAVLDLERMLGERG | 114 | ND |
| *Paraburkholderia* | FSRADIVKIAGNGGGAQALKAVLEHEATLDERG | 115 | NG |
| *Paraburkholderia* | FSRADIVRIAGNGGGAQALYSVLDVEPTLGKRG | 116 | NG |
| *Paraburkholderia* | FSQPDIVKMASNIGGAQALQAVLELEPALRERG | 117 | NI |
| *Paraburkholderia* | FSQPDIVKMAGNIGGAQALQAVLSLGPALRERG | 118 | NI |
| *Paraburkholderia* | FSQPEIVKIAGNIGGAQALHTVLELEPTLHKRG | 119 | NI |
| *Paraburkholderia* | FSQSDIVKIAGNIGGAQALQAVLDLESMLGKRG | 120 | NI |
| *Paraburkholderia* | FSQSDIVKIAGNIGGAQALQAVLELEPTLRESD | 121 | NI |
| *Paraburkholderia* | FNPTDIVKIAGNKGGAQALQAVLELEPALRERG | 122 | NK |
| *Paraburkholderia* | FSPTDIIKIAGNNGGAQALQAVLDLELMLRERG | 123 | NN |
| *Paraburkholderia* | FSQADIVKIAGNNGGAQALYSVLDVEPTLGKRG | 124 | NN |
| *Paraburkholderia* | FSRGDIVTIAGNNGGAQALQAVLELEPTLRERG | 125 | NN |
| *Paraburkholderia* | FSRIDIVKIAANNGGAQALHAVLDLGPTLRECG | 126 | NN |
| *Paraburkholderia* | FSQADIVKIVGNNGGAQALQAVFELEPTLRERG | 127 | NN |
| *Paraburkholderia* | FSQPDIVRITGNRGGAQALQAVLALELTLRERG | 128 | NR |
| *Legionellales* | FKADDAVRIACRTGGSHNLKAVHKNYERLRARG | 129 | RT |
| *Legionellales* | FNADQVIKIVGHDGGSNNIDVVQQFFPELKAFG | 130 | HD |
| *L. maceachernii* | FSAEQIVRIAAHIGGSRNIEATIKHYAMLTQPP | 131 | HI |
| *Francisella* | YKSEDIIRLASHDGGSVNLEAVLRLNPQLIGLG | 132 | HD |
| *Francisella* | YKSEDIIRLASHDGGSINLEAVLRLNPQLIGLG | 133 | HD |
| *Francisella* | YKSEDIIRLASSNGGSVNLEAVLRLNPQLIGLG | 134 | SN |
| *Francisella* | YKSEDIIRLASSNGGSVNLEAVIAVHKALHSNG | 135 | SN |
| *Legionellales* | FSADQVVKIAGHSGGSNNIAVMLAVFPRLRDFG | 136 | HS |
| *Francisella* | YKINHCVNLLKLNHDGFMLKNLIPYDSKLTGLG | 137 | LN |

Residues $X_{12}X_{13}$ of the RU may a base contacting residues (BCR) as listed in the table 8 and may be chosen based upon the target nucleic acid sequence.

In certain aspects, the last RU in the DBP may be a half RU. In certain aspects, the half RU may include a sequence that is at least 80%, at least 90%, at least 95% or a 100% identical to the half RU from *L. quateirensis* (FNAEQIVRMVS $X_{12}X_{13}$ GGSKNL; SEQ ID NO: 138). In certain aspects, the half RU may include a sequence that is at least 80%, at least 90%, at least 95% or a 100% identical to the half RU from *Francisella* (YNKKQIVLIAS $X_{12}X_{13}$ SGG; SEQ ID NO: 139)

In certain aspects, the polypeptide comprises an N-cap region, where the C-terminus (i.e., the last amino acid) of the N-cap region is covalently linked to the N-terminus (i.e., the first amino acid) of the first RU of the DBP either directly or via a linker. In certain aspects, the N-cap region is the N-terminus of *L. quateirensis* protein and may have an amino acid sequence that is at least 80% (e.g., at least 85%, at least 90%, 95%, or 99%, or a 100%) identical to the amino acid sequence:

MPDLELNFAIPLHLFDDETVFTHDATNDNSQASSSYSSKSSPASANARKR TSRKEMSGPPSKEPANTKSRRANSQNN KLSLADRLTKYNIDEEFYQTRSDSLLSLNYTK KQIERLILYKGRTSAVQQLLCKHEELLNLISPDG (SEQ ID NO: 140) In certain aspects, the N-cap region is a N-terminal domain or a fragment thereof from TALE proteins like those expressed in *Burkholderia, Paraburkholderia, or Xanthomonas.*

In certain aspects, the polypeptide comprises a C-cap region, where the N-terminus (i.e., the first amino acid) of the C-terminal domain is covalently linked to the C-terminus (i.e., the last amino acid) of the last RU or the half-repeat unit, if present, in the DBP either directly or via a linker. In certain aspects, the C-cap region is the C-terminal domain of L. quateirensis protein and may have an amino acid sequence that is at least 80% (e.g., at least 85%, at least 90%, 95%, or 99%, or a 100%) identical to the amino acid sequence:

ALVKEYFPVFSSFHFTADQIVALICQSKQCFRNLKKNHQQWKNKGLSAE

QIVDLILQETPPKPNFNNTSSSTPSPSAPSFFQGPSTPIPTPVLDNSPAPIFSNPVCFFSSRSE

NNTEQYLQDSTLDLDSQLGDPTKNFNVNNFWSLFPFDDVGYHPHSNDVGYHLHSDEES

PFFDF (SEQ ID NO: 141)

In certain aspects, the C-cap region has the amino acid sequence ALVKEYFPVFSSFHFTADQIVALICQSKQ CFRNLKKNHQQWKNKGLSAEQIVDLILQETP PKP (SEQ ID NO: 142). In certain aspects, the C-cap region domain is a C-terminal domain or a fragment thereof from TALE proteins like those expressed in *Burkholderia, Paraburkholderia, or Xanthomonas.*

**Mixed DNA Binding Domains**

In some aspects, the present disclosure provides DNA binding domains in which the repeat units, the N-cap, and the C-ap can be derived from any one of *Ralstonia solanacearum, Xanthomonas spp., Legionella quateirensis, Burkholderia, Paraburkholderia,* or *Francisella.* For example, the present disclosure provides a DNA binding domain wherein the plurality of repeat units are selected from any one of the RUs as provided herein and can further comprise an N-cap and/or C-cap as provided herein.

**Functional Domains**

A DBP as disclosed herein can be associated with a functional domain as described in the preceding sections. The functional domain can provide different types of activity, such as genome editing, gene regulation (e.g., activation or repression), or visualization of a genomic locus via imaging. In certain aspects, the functional domain is heterologous to the DBP. Heterologous in the context of a functional domain and a DBP as used herein indicates that these domains are derived from different sources and do not exist together in nature. In some aspects, the nuclease can be a cleavage half domain, which dimerizes to form an active full domain capable of cleaving DNA. In other aspects, the nuclease can be a cleavage domain, which is capable of cleaving DNA without needing to dimerize. For example, a nuclease comprising a cleavage half domain can be an endonuclease, such as FokI or BfiI. In some aspects, two cleavage half domains (e.g., FokI or BfiI) can be fused together to form a fully functional single cleavage domain.

A nuclease domain fused to a DBP can be an endonuclease or an exonuclease. An endonuclease can include restriction endonucleases and homing endonucleases. An endonuclease can also include S1 Nuclease, mung bean nuclease, pancreatic DNase I, micrococcal nuclease, or yeast HO endonuclease. An exonuclease can include a 3'-5' exonuclease or a 5'-3' exonuclease. An exonuclease can also include a DNA exonuclease or an RNA exonuclease. Examples of exonuclease includes exonucleases I, II, III, IV, V, and VIII; DNA polymerase I, RNA exonuclease 2, and the like.

A nuclease domain fused to a DBP as disclosed herein can be a restriction endonuclease (or restriction enzyme). In some instances, a restriction enzyme cleaves DNA at a site removed from the recognition site and has a separate binding and cleavage domains. In some instances, such a restriction enzyme is a Type IIS restriction enzyme.

As another example, DBP as disclosed herein can be linked to a gene regulating domain. A gene regulation domain can be an activator or a repressor. For example, a DBP as disclosed herein can be linked to an activation domain, such as VP16, VP64, p65, p300 catalytic domain, TET1 catalytic domain, TDG, Ldb1 self-associated domain, SAM activator (VP64, p65, HSF1), or VPR (VP64, p65, Rta). Alternatively, a DBP can be linked to a repressor, such as KRAB, Sin3a, LSD1, SUV39H1, G9A (EHMT2), DNMT1, DNMT3A-DNMT3L, DNMT3B, KOX, TGF-beta-inducible early gene (TIEG), v-erbA, SID, MBD2, MBD3, Rb, or MeCP2. The terms "repressor," "repressor domain," and "transcriptional repressor" are used herein interchangeably to refer to a polypeptide that decreases expression of a gene.

In some aspects, a DBP as disclosed herein can be linked to a DNA modifying protein, such as DNMT3a. A DBP can be linked to a chromatin-modifying protein, such as lysine-specific histone demethylase 1 (LSD1). A DBP can be linked to a protein that is capable of recruiting other proteins, such as KRAB. The DNA modifying protein (e.g., DNMT3a) and proteins capable of recruiting other proteins (e.g., KRAB) can serve as repressors of transcription. Thus, DBP linked to a DNA modifying protein (e.g., DNMT3a) or a domain capable of recruiting other proteins (e.g., KRAB, a domain found in transcriptional repressors, such as Kox1) can provide gene repression functionality, can serve as transcription factors, wherein the DBP provides specificity and targeting and the DNA modifying protein and the protein capable of recruiting other proteins provides gene repression functionality, which can be referred to as an engineered genomic regulatory complex or a DBP-gene regulator (DBP-GR) and, more specifically, as a DBP -transcription factor (DBP-TF).

In certain aspects, the functional domain may be an imaging domain, e.g., a fluorescent protein, biotinylation reagent, tag (e.g., 6X-His or HA). A DBP can be linked to a fluorophore, such as Hydroxycoumarin, methoxycoumarin, Alexa fluor, aminocoumarin, Cy2, FAM, Alexa fluor 488, Fluorescein FITC, Alexa fluor 430, Alexa fluor 532, HEX, Cy3, TRITC, Alexa fluor 546, Alexa fluor 555, R-phycoerythrin (PE), Rhodamine Red-X, Tamara, Cy3.5, Rox, Alexa fluor 568, Red 613, Texas Red, Alexa fluor 594, Alexa fluor 633, Allophycocyanin, Alexa fluor 633, Cy5, Alexa fluor 660, Cy5.5, TruRed, Alexa fluor 680, Cy7, GFP, or mCHERRY.

In certain aspects, the DBP is not fused with a functional domain having a genome modifying activity, such as, cleavage activity, DNA methylation activity, chromatin-modifying protein, transcriptional activation, or transcriptional repression.

## METHODS

The present disclosure provides methods for remodeling chromatin architecture in a regulatory region of a gene of interest in a cell by providing in the cell a DNA-binding polypeptide (DBP) that binds to a sequence in the regulatory region of the gene. In certain embodiments, the method may be used to modulate expression of the gene. In certain embodiments, the DBP may have no functional domain, e.g., an enzyme, a transcription factor, etc. In certain embodiments, the remodeling comprises decreasing chromatin condensation and/or removing nucleosomes bound to the chromatin. Thus, the methods and DBPs disclosed herein may be used for localized opening of a closed region of the chromatin resulting in expression of gene(s) present in the closed region. In certain embodiments, the opening of a closed chromatin may be assayed by measuring sensitivity of the chromatin to cleavage by a DNase, e.g., detecting DNase I hypersensitive sites (DHSs) in the region upon exposure to the DBP and comparing to the number and/or pattern of DHSs in the same region in a cell not exposed to the DBP.

In certain embodiments, the DBP may be used to open a transcriptionally inactive region of the chromatin and enhance binding of TFs to sequences adjacent to the region at which the DBP binds. In certain embodiments, the DBP may be designed to bind to a site bound by a transcription factor (TF) and compete with the TF for binding to the site. In certain embodiments, the DBP may not only compete with the TF but may also enhance binding of TFs to sequences adjacent to the region at which the DBP binds. In certain embodiments, the DBP may not compete with a TF but may enhance binding of TFs to sequences adjacent to the region at which the DBP binds. In certain embodiments, the DBP may bind to the same site as a transcriptional repressor and in addition to decreasing the activity of the repressor may also enhance binding of transcriptional activator(s) to site(s) adjacent the site to which the transcriptional repressor binds. In certain embodiments, the DBP may bind to the same site as a transcriptional activator and in addition to decreasing the activity of the activator may also enhance binding of transcriptional activator(s) to site(s) adjacent the site to which the transcriptional activator binds.

In certain embodiments, the DBP may bind to a sequence in a regulatory region of the gene of interest which sequence is present at a position at least 100 nucleotides upstream of the transcription start site (TSS) (i.e., at least position -100). In certain embodiments, the DBP may bind to a sequence in a regulatory region of the gene of interest which sequence is present at a position up to 400 nucleotides upstream of the transcription start site (i.e., up to position - 400). In certain embodiments, the DBP may bind to a sequence in a regulatory region of the gene of interest which sequence is present at a position between -350 to -150; -300 to -175; - 250 to -150; -230 to -180; or -230 to -190 with reference to the TSS.

In certain embodiments, the gene of interest may be $\gamma$-globin gene (*HBG1* and/or *HBG2)* encoding fetal hemoglobin G (HBG) and the method may include increasing expression of fetal hemoglobin G (HBG) in a cell by binding of a DBP to a regulatory sequence in the fetal $\gamma$-globin gene promoter (*HBG1* and/or *HBG2)*. In certain embodiments, the DBP may bind to a nucleotide sequence present between -350 to -150; -300 to -175; -250 to -150; -230 to -180; or - 230 to -190 with reference to the TSS in the fetal $\gamma$-globin gene promoter of *HBG1* and/or *HBG2* gene. In certain embodiments, the DBP may bind to a nucleotide sequence present between positions -222 to -193 with reference to the TSS in the *HBG1* and/or *HBG2* gene. In certain embodiments, the DBP may bind to a nucleotide sequence present between positions -222 to -193 with reference to the TSS in the *HBG1* gene. In certain embodiments, the DBP may bind to a nucleotide sequence present between positions -227 to -193 with reference to the TSS in the *HBG2* gene.

In certain embodiments, a method for increasing expression of fetal hemoglobin G (HBG) in a cell, e.g., an adult erythrocyte, may include expressing in the cell a DBP that binds to a sub-sequence present in the sequence AGCAG TATCCTCTTGGGGGCCCCTTCCCCA (SEQ ID NO:3) in the regulatory region of the HBG1 and/or the HBG2 gene or a complement thereof. In certain embodiments, the sub-sequence may include the nucleotide sequence TCTT or a complement thereof. In certain embodiments, the DBP may bind to at least the nucleotide sequence TCTT and up to 16 nt present upstream or downstream of the sequence TCTT. In certain embodiments, the DBP may bind to at least the nucleotide sequence TCTT and up to 16 nt present downstream of the sequence TCTT. In certain embodiments, the DBP may bind to at least the nucleotide sequence TCTT and up to 11 nt present upstream of the sequence TCTT.

In certain embodiments, a method for increasing expression of fetal hemoglobin G (HBG) in a cell, e.g., an adult erythrocyte, may include expressing in the cell a DBP that binds to a sub-sequence present in the sequence TAAGC AGCAGTATCCTCTTGGGGGCCCCTTCCCCA (SEQ ID NO:8) in the regulatory region of the HBG2 gene or a complement thereof. In certain embodiments, the sub-sequence may include the nucleotide sequence TCTT or a complement thereof. In certain embodiments, the DBP may bind to at least the nucleotide sequence TCTT and up to 16 nt present upstream or downstream of the sequence TCTT. In certain embodiments, the DBP may bind to at least the nucleotide sequence TCTT and up to 16 nt present downstream of the sequence TCTT. In certain embodiments, the DBP may bind to at least the nucleotide sequence TCTT and up to 15 nt present upstream of the sequence TCTT.

The DBP disclosed herein and used in the methods provided herein may include a plurality of repeat units (RUs) that are arranged from N-terminus to C-terminus to bind to a sequence provided herein. The RUs may be derived from a TALE protein and the DBP may include at least 11.5 RUs, which include 11 RUs and a terminal half-repeat unit (0.5 RU).

The present disclosure provides a method for modulating expression of a target gene in a cell, the method comprising introducing into the cell a DNA binding polypeptide (DBP) that binds a sequence in regulatory region of a gene bound by a transcription factor (TF), thereby displacing the TF and modulating expression of the gene.

In certain aspects, the sequence include a binding site for a TF that has also previously been identified as being associated with activity of the TF based on reduced activity of the TF when the binding site includes or is adjacent to a single nucleotide polymorphism (SNP) or a mutation. In certain aspects, the binding site may include or is adjacent to SNPs or mutations that lead to an increased expression of the protein encoded by the gene, which may be indicative of reduced binding of a TF, such as a transcriptional repressor, to the binding site. In certain aspects, the binding site may include or is adjacent to SNPs or mutations that lead to a decreased expression of the protein encoded by the gene, which may be indicative of reduced binding of a TF, such as a transcriptional activator, to the binding site. In certain aspects, it may be desirable to displace the TF from the binding site in the regulatory region of a target gene. In certain aspects, the method for modulating expression of the target gene in a cell includes introducing into the cell a DBP that binds to the binding site and additional nucleotides present adjacent the binding site, e.g., nucleotides flanking the binding site.

The mutation in or adjacent the binding site may be a deletion, insertion, or substitution. As used herein, the term binding site refers to a core sequence that is required for binding of a TF to the regulatory region of a gene and modulate gene expression. A binding site is usually less than 10 nucleotides in length and more commonly 4-8 nucleotides in length. A TF may bind to more than one binding site in the regulatory region of a gene. As noted herein, the DBP may bind to a binding site as well as nucleotides adjacent the binding site to increase the binding specificity of the DBP such that it binds to the binding site for the TF for a target gene but not to other genes that also including a binding site for the TF but include different nucleotide sequences adjacent the binding site.

In certain aspects, the DBP may bind to at least a 12 nucleotides long sequence comprising the sequence bound by the TF. In certain aspects, the DBP may bind to at least a 14, 16, 18, 20, 24, 26, 28, 30, or up to 45 nucleotides long sequence comprising the sequence bound by the TF. In certain aspects, the DBP may bind to the binding site for a transcriptional activator and the introducing results in reduced expression of the gene. In certain aspects, the DBP may bind to the binding site for a transcriptional repressor and the introducing results in increased expression of the gene.

In certain aspects, the DBP is introduced into the cell as a nucleic acid encoding the DBP. The nucleic acid may be a deoxyribonucleic acid (DNA) or a ribonucleic acid (RNA). In certain aspects, the sequence of the nucleic acid is codon optimized for expression in a human cell. Methods and compositions for introducing a nucleic acid into a cell are described in detail in subsequent sections of this disclosure.

In certain aspects, the cell is a human cell, such as, a cancer cell, an ex vivo cell obtained from a subject, a stem cell, a hematopoietic stem cell, or the like. Cells and cell lines containing a target gene are described in detail in subsequent sections of this disclosure. In certain aspects, the target gene may fetal hemoglobin gamma.

In certain aspects, introducing the DBP into a cell may include administering the polypeptide or a nucleic acid encoding the polypeptide to a subject in need thereof.

The present disclosure provides a method for increasing expression of fetal hemoglobin-y (HBG) in a subject in need thereof, the method comprising administering to the subject the DBPs disclosed herein. The DBP may be a DBP that binds to the nucleic acid sequence CCTCTTGGGGGC (SEQ ID NO:201) or a complement thereof present in the fetal $\gamma$-globin gene promoter, as described in the preceding section. The DBP may be a DBP that binds to the nucleic acid sequence CACCCTGTGGAGCCACAC (SEQ ID NO:181) or a complement thereof present in the adult $\beta$-globin (HBB) gene promoter, as described in the preceding section. The DBP may be a DBP that binds to the nucleic acid sequence TGCTTTTATCACAGGCT (SEQ ID NO: 180) or a complement thereof present in the BCL11A gene promoter, as described in the preceding section. One or two or more of these different DBPs may be administered to the subject.

A method for modulating expression of a gene in a cell is also provided, the method comprising introducing into the cell a DNA binding polypeptide (DBP), or a nucleic acid encoding the DBP, that binds a sequence in regulatory region of a gene bound by a transcription factor (TF), thereby displacing the TF and modulating expression of the gene, wherein the DBP competes with the TF for binding to a regulatory sequence comprising CACC box or GATA site, wherein the DBP binds to the sequence CACC or the complement thereof in the CACC box and at least eight, at least nine, at least ten, at least eleven, at least twelve, at least thirteen, or at least fourteen nucleotides 5' and/or 3' of the CACC sequence or the complement thereof or wherein the DBP binds to the sequence GATA or the complement thereof at the GATA site and at least eight, at least nine, at least ten, at least eleven, at least twelve, at least thirteen, or at least fourteen nucleotides 5' and/or 3' of the GATA sequence or the complement thereof.

The DBP may bind to the CACC box present at position -90 upstream of the transcription start site of the human hemoglobin B (HBB) gene. The TF may be a transcription activator and the binding of the DBP to the CACC box may result in decreased expression of HBB protein. The DBP may comprise a plurality of RUs arranged from N-terminus to C-terminus to bind to the sequence CACCCTGTGGAGCCACAC (SEQ ID NO:181) at the CACC box. The DBP that binds to a sequence at the CACC box may be as described in the preceding section.

The DBP binds to the GATA site in + 58 DHS of BCL11A enhancer. The TF may be a transcriptional activator and binding of the DBP to the GATA site results in reduced expression of BCL11A protein. The DBP may comprise a plurality of repeat units (RUs) arranged from N-terminus to C-terminus to bind to the complement of GATA at the GATA site. The DBP may comprise a plurality of RUs arranged from N-terminus to C-terminus to bind to the sequence TGCTTTTATCACAGGCT (SEQ ID NO:180) at the GATA site. The DBP that binds to a sequence at the GATA site may be as described in the preceding section.

The subject may be in need of treatment of a hemoglobin-related disorder, such as, sickle cell anemia or thalassemia.

**Targets**

In some aspects, a cell that expresses the DBP disclosed herein may be a mammalian cell such as a stem cell (e.g., human embryonic stem cell or induced pluripotent stem cell), human hematopoietic stem cell "HSC" (e.g., $CD34^+$ HSC), hematopoietic progenitor cell (HPC), a cell in the erythroid lineage, a lymphocyte, a T-cell, CAR-T cells, a cancer cell, ex vivo cell, etc. A cell may be selected for stable expression of the DBP. A cell may be selected for expressing a threshold level of the DBP. A cell selected for expression of the DBP may be subjected to expansion, freeze/thaw or otherwise prepared for introduction into a subject in need thereof.

A cell expressing a DBP either constitutively or in an inducible manner may be administered to the subject, for instance in the circulatory system by means of intravenous delivery or delivery into a solid tissue such as bone marrow.

Exemplary mammalian cells can include, but are not limited to, 293A cell line, 293FT cell line, 293F cells , 293 H cells, HEK 293 cells, CHO DG44 cells, CHO-S cells, CHO-K1 cells, Expi293F™ cells, Flp-In™ T-REx™ 293 cell line, Flp-In™-293 cell line, Flp-In™-3T3 cell line, Flp-In™-BHK cell line, Flp-In™-CHO cell line, Flp-In™-CV-1 cell line, Flp-In™-Jurkat cell line, FreeStyle™ 293-F cells, FreeStyle™ CHO-S cells, GripTite™ 293 MSR cell line, GS-CHO cell line, HepaRG™ cells, T-REx™ Jurkat cell line, Per.C6 cells, T-REx™-293 cell line, T-REx™-CHO cell line, T-REx™-HeLa cell line, NC-HIMT cell line, PC12 cell line, primary cells (e.g., from a human) including primary T cells, primary hematopoietic stem cells, primary human embryonic stem cells (hESCs), and primary induced pluripotent stem cells (iPSCs).

In some cases, the cell may be a human erythroid progenitor or precursor cells. The erythroid progenitor or precursor cells may be autologous.

In some cases, a target cell is a cancerous cell. Cancer can be a solid tumor or a hematologic malignancy. The solid tumor can include a sarcoma or a carcinoma. Exemplary sarcoma target cell can include, but are not limited to, cell obtained from alveolar rhabdomyosarcoma, alveolar soft part sarcoma, ameloblastoma, angiosarcoma, chondrosarcoma, chordoma, clear cell sarcoma of soft tissue, dedifferentiated liposarcoma, desmoid, desmoplastic small round cell tumor, embryonal rhabdomyosarcoma, epithelioid fibrosarcoma, epithelioid hemangioendothelioma, epithelioid sarcoma, esthesioneuroblastoma, Ewing sarcoma, extrarenal rhabdoid tumor, extraskeletal myxoid chondrosarcoma, extraskeletal osteosarcoma, fibrosarcoma, giant cell tumor, hemangiopericytoma, infantile fibrosarcoma, inflammatory myofibroblastic tumor, Kaposi sarcoma, leiomyosarcoma of bone, liposarcoma, liposarcoma of bone, malignant fibrous histiocytoma (MFH), malignant fibrous histiocytoma (MFH) of bone, malignant mesenchymoma, malignant peripheral nerve sheath tumor, mesenchymal chondrosarcoma, myxofibrosarcoma, myxoid liposarcoma, myxoinflammatory fibroblastic sarcoma, neoplasms with perivascular epitheioid cell differentiation, osteosarcoma, parosteal osteosarcoma, neoplasm with perivascular epitheioid cell differentiation, periosteal osteosarcoma, pleomorphic liposarcoma, pleomorphic rhabdomyosarcoma, PNET/extraskeletal Ewing tumor, rhabdomyosarcoma, round cell liposarcoma, small cell osteosarcoma, solitary fibrous tumor, synovial sarcoma, or telangiectatic osteosarcoma.

Exemplary carcinoma target cell can include, but are not limited to, cell obtained from anal cancer, appendix cancer, bile duct cancer (i.e., cholangiocarcinoma), bladder cancer, brain tumor, breast cancer, cervical cancer, colon cancer, cancer of Unknown Primary (CUP), esophageal cancer, eye cancer, fallopian tube cancer, gastroenterological cancer, kidney cancer, liver cancer, lung cancer, medulloblastoma, melanoma, oral cancer, ovarian cancer, pancreatic cancer, parathyroid disease, penile cancer, pituitary tumor, prostate cancer, rectal cancer, skin cancer, stomach cancer, testicular cancer, throat cancer, thyroid cancer, uterine cancer, vaginal cancer, or vulvar cancer.

Alternatively, the cancerous cell can comprise cells obtained from a hematologic malignancy. Hematologic malignancy can comprise a leukemia, a lymphoma, a myeloma, a non-Hodgkin's lymphoma, or a Hodgkin's lymphoma. In some cases, the hematologic malignancy can be a T-cell based hematologic malignancy. Other times, the hematologic malignancy can be a B-cell based hematologic malignancy. Exemplary B-cell based hematologic malignancy can include, but are not limited to, chronic lymphocytic leukemia (CLL), small lymphocytic lymphoma (SLL), high-risk CLL, a non-CLL/SLL lymphoma, prolymphocytic leukemia (PLL), follicular lymphoma (FL), diffuse large B-cell lymphoma (DLBCL), mantle cell lymphoma (MCL), Waldenström's macroglobulinemia, multiple myeloma, extranodal marginal zone B cell lymphoma, nodal marginal zone B cell lymphoma, Burkitt's lymphoma, non-Burkitt high grade B cell lymphoma, primary mediastinal B-cell lymphoma (PMBL), immunoblastic large cell lymphoma, precursor B-lymphoblastic lymphoma, B cell prolymphocytic leukemia, lymphoplasmacytic lymphoma, splenic marginal zone lymphoma, plasma cell myeloma, plasmacytoma, mediastinal (thymic) large B cell lymphoma, intravascular large B cell lymphoma, primary effusion lymphoma, or lymphomatoid granulomatosis. Exemplary T-cell based hematologic malignancy can include, but are not limited to, peripheral T-cell lymphoma not otherwise specified (PTCL-NOS), anaplastic large cell lymphoma, angioimmunoblastic lymphoma, cutaneous T-cell lymphoma, adult T-cell leukemia/lymphoma (ATLL), blastic NK-cell lymphoma, enteropathy-type T-cell lymphoma, hematosplenic gamma-delta T-cell lymphoma, lymphoblastic lymphoma, nasal NK/T-cell lymphomas, or treatment-related T-cell lymphomas.

In some cases, a cell can be a tumor cell line. Exemplary tumor cell line can include, but are not limited to, 600MPE, AU565, BT-20, BT-474, BT-483, BT-549, Evsa-T, Hs578T, MCF-7, MDA-MB-231, SkBr3, T-47D, HeLa, DU145, PC3, LNCaP, A549, H1299, NCI-H460, A2780, SKOV-3/Luc, Neuro2a, RKO, RKO-AS45-1, HT-29, SW1417, SW948, DLD-1, SW480, Capan-1, MC/9, B72.3, B25.2, B6.2, B38.1, DMS 153, SU.86.86, SNU-182, SNU-423, SNU-449, SNU-475, SNU-387, Hs 817.T, LMH, LMH/2A, SNU-398, PLHC-1, HepG2/SF, OCI-Ly1, OCI-Ly2, OCI-Ly3, OCI-Ly4, OCI-Ly6, OCI-Ly7, OCI-Ly10, OCI-Ly18, OCI-Ly19, U2932, DB, HBL-1, RIVA, SUDHL2, TMD8, MEC1, MEC2, 8E5, CCRF-CEM, MOLT-3, TALL-104, AML-193, THP-1, BDCM, HL-60, Jurkat, RPMI 8226, MOLT-4, RS4, K-562, KASUMI-1, Daudi, GA-10, Raji, JeKo-1, NK-92, and Mino.

Genetic modification can involve introducing a functional gene for therapeutic purposes, knocking out a gene for therapeutic gene, or engineering a cell ex vivo (e.g., HSCs or CAR T cells) to be administered back into a subject in need thereof. Cells, such as hematopoietic stem cells (HSCs) and T cells, can be engineered ex vivo to express the DBP. Alternatively, nucleic acid encoding the DBP can be directly administered to a subject in need thereof.

The target gene may be an endogenous gene such as human fetal gamma globin gene, PDCD 1 gene, a CTLA4 gene, a LAG3 gene, a TET2 gene, a ETLA gene, a HA VCR2 gene, a CCR5 gene, a CXCR4 gene, a TRA gene, a TRE gene, a E2M gene, an albumin gene, a HEE gene, a HEAI gene, a TTR gene, a NR3Cl gene, a CD52 gene, an erythroid specific enhancer of the ECLIIA gene, a CELE gene, a TGFERI gene, a SERPINAl gene, a HEV genomic DNA in infected cells, a CEP290 gene, a DMD gene, a CFTR gene, or an IL2RG gene.

## Compositions

The compositions disclosed herein may comprise one or more DBP, polynucleotides encoding the DBPs, vectors comprising same, and cells comprising the DBP or polynucleotides encoding the DBPs, as disclosed herein. These compositions are useful for increasing level of human $\gamma$-globin protein in a cell or a population of cells. The cell may be a hematopoietic cell, e.g., a hematopoietic stem or progenitor cell, or a CD34$^+$ cell.

In certain aspects, the polypeptides described herein may be present in a pharmaceutical composition comprising a pharmaceutically acceptable excipient. In certain aspects, the polypeptides are present in a therapeutically effective amount in the pharmaceutical composition. A therapeutically effective amount can be determined based on an observed effectiveness of the composition. A therapeutically effective amount can be determined using assays that measure the desired effect in a cell.

The pharmaceutical compositions can be administered ex vivo or in vivo to a subject in order to practice the therapeutic methods and uses described herein.

The pharmaceutical compositions of the present disclosure can be formulated to be compatible with the intended method or route of administration; exemplary routes of administration are set forth herein. Suitable pharmaceutically acceptable or physiologically acceptable diluents, carriers or excipients include, but are not limited to, nuclease inhibitors, protease inhibitors, a suitable vehicle such as physiological saline solution or citrate buffered saline.

A pharmaceutical composition comprising the DBP as disclosed herein and a pharmaceutically acceptable excipient is provided. A pharmaceutical composition comprising the nucleic acid as disclosed herein or the vector as disclosed herein and a pharmaceutically acceptable excipient is provided. A pharmaceutical composition comprising the host cell of comprising the DBP disclosed herein or a nucleic acid as disclosed herein or the vector as disclosed herein is provided.

The compositions of the present disclosure find use in a variety of therapeutic and research applications.

In certain aspects, the compositions disclosed herein such as a compositions comprising a nucleic acid encoding a DBP that displaces the TF ZBTB7A from the fetal $\gamma$-globin gene promoter and results in expression of fetal hemoglobin- y (HBG) from the fetal $\gamma$-globin gene, the DBP encoded by the nucleic acid, or a cell comprising the nucleic acid or the DBP may be in a method for the increasing expression of fetal hemoglobin-y (HBG) in a subject in need thereof. In certain aspects, the compositions disclosed herein such as a compositions comprising a nucleic acid encoding a DBP that displaces the TA from the adult human $\beta$-globin gene promoter and results in reduced expression of HBB, the DBP encoded by the nucleic acid, or a cell comprising the nucleic acid or the DBP may be in a method for the increasing total fetal hemoglobin-y (HBG) when normalized to level of HBB or the level of HBG and HBB in a subject in need thereof. In certain aspects, the compositions disclosed herein such as a compositions comprising a nucleic acid encoding a DBP that displaces the TF from the BCL11A gene promoter and results in decreased expression of BCL11A which leads to increased expression of fetal hemoglobin- y (HBG) from the fetal $\gamma$-globin gene, the DBP encoded by the nucleic acid, or a cell comprising the nucleic acid or the DBP may be in a method for the increasing expression of fetal hemoglobin-y (HBG) in a subject in need thereof. The subject may have a blood cell disorder such as a hemoglobinopathy. In certain aspects the subject has sickle cell anemia or thalassemia.

**Delivery**

The DBP disclosed herein and compositions comprising the disclosed polypeptides can be delivered into a target cell by any suitable means, including, for example, by contacting the cell with the polypeptide or a nucleic acid encoding the polypeptide.

In certain aspects, the DBP or a can be delivered into cells in a particular tissue (e.g., a solid tumor) by injecting a composition comprising the positively charged polypeptide directly into the solid tumor.

In other aspects, administration involves systemic administration (e.g., intravenous, intraperitoneal, intramuscular, subdermal, or intracranial infusion), direct injection (e.g., intrathecal), or topical application, etc.

Nucleic acids encoding the DBPs may be delivered into a target cell by e.g., vectors (e.g., viral or non-viral vectors), non-vector based methods (e.g., using naked DNA or DNA complexes, e.g., cationic liposomes, polymeric nanoparticles, polymers, etc.), or a combination thereof. Nucleic acids encoding the DBPs can be delivered directly to cells as naked DNA or RNA, for instance by means of transfection or electroporation, or can be conjugated to molecules (e.g., N-acetylgalactosamine) promoting uptake by the target cells (e.g., erythrocytes, HSCs). The nucleic acid vector can also include any suitable number of regulatory/control elements, e.g., promoters, enhancers, introns, polyadenylation signals, Kozak consensus sequences, or internal ribosome entry sites (IRES).

Viral vectors used for delivering the nucleic acid encoding a DBP include retroviral vectors, lentiviral vectors, adenoviral vectors, adeno-associated viral vectors, and the like.

**Routes of Administration**

Nucleic acid encoding DBP or cells comprising such a nucleic acid or cells expressing the DBP, can be administered to subjects by any suitable mode or route, whether local or systemic. Systemic modes of administration include oral and parenteral routes. Parenteral routes include, by way of example, intravenous, intramarrow, intrarterial, intramuscular, intradermal, subcutaneous, intranasal, and intraperitoneal routes. Nucleic acids administered systemically can be modified or formulated to target, e.g., HSCs, hematopoietic stem/progenitor cells, or erythroid progenitors or precursor cells.

Local modes of administration include, by way of example, intramarrow injection into the trabecular bone or intrafemoral injection into the marrow space, and infusion into the portal vein.

Administration can be provided as a periodic bolus (for example, intravenously) or as continuous infusion from an internal reservoir or from an external reservoir (for example, from an intravenous bag or implantable pump). Nucleic acids and/or cells can be administered locally, for example, by continuous release from a sustained release drug delivery device.

**Subjects**

A subject in need of modulation of gene expression by administering a composition as disclosed herein such as in need of increased expression of HBG may have a deficiency of globin synthesis such as thalassemia syndromes and/or structural abnormalities of globin such as sickle cell syndromes and syndromes associated with unstable hemoglobins. These diseases are referred to as hemoglobinopathy. Thalassemia syndromes result from deficiencies in either alpha-globin ($\alpha$-thalassemia) or beta-like globin ($\beta$-thalassemia) chains. The diseases become apparent when the deficient globin is required during development. $\alpha$-Thalassemia is symptomatic during gestation, as $\alpha$-globin is required for fetal hemoglobin (HbF, $\alpha 2\gamma 2$). As $\beta$-globin is not required in large amounts before birth, $\beta$-thalassemia is asymptomatic until around 6 months after birth. Mutations that cause prolonged production of fetal $\gamma$-globin chains may present later, at 2 to 4 years of age.

The major pathologic process in thalassemia is the imbalance of alpha and non-alpha globin chain accumulation. The unaffected chains, produced in normal amounts, precipitate during erythropoiesis. In $\beta$-thalassemia, the precipitated $\alpha$-globin chains are particularly toxic, damaging cell membranes and causing apoptosis. $\beta 0$-Thalassemias are characterized by a complete absence of any beta globin chains. $\beta +$-Thalassemias are characterized by detectable presence of a reduced amount of beta chains. There are three principal categories of beta thalassemias: thalassemia major, thalassemia intermedia, and thalassemia minor.

In certain instances, a person carries the beta thalassemia trait and the hemoglobin S trait (the abnormal hemoglobin found in people with sickle cell disease), which leads to HbS beta thalassemia. The severity of this condition varies according to the amount of normal beta globin produced by the beta globin gene. When no beta globin is produced by the beta globin gene, the condition is almost identical to sickle cell disease.

In sickle cell disease (SCD), one amino acid substitution in the beta globin chain results in the generation of hemoglobin S (HbS). Upon deoxygenation, HbS molecules undergo aggregation and polymerization ultimately leading to a morphological distortion of the red cells, which acquire a sickle or holly-leaf shape. Sickle cell anemia $\beta S/\beta S$, a common form of sickle cell disease (SCD), is caused by Hemoglobin S (HbS). Additional mutations in the $\beta$-globin gene can also cause other abnormalities in $\beta$-globin, leading to other types of sickle cell disease. These abnormal forms of $\beta$- globin are often designated by letters of the alphabet or sometimes by a name. In these other types of sickle cell disease, one $\beta$-globin subunit is replaced with HbS and the other $\beta$-globin subunit is replaced with a different abnormal variant, such as hemoglobin C (HbC; $\beta$-globin allele noted as $\beta^C$) or hemoglobin E (HbE; $\beta$-globin allele noted as $\beta^E$).

In hemoglobin SC (HbSC) disease, the $\beta$-globin subunits are replaced by HbS and HbC. HbC results from a mutation in the $\beta$-globin gene and is the predominant hemoglobin found in people with HbC disease ($\alpha_2\beta^C_2$). HbC disease is relatively benign, producing a mild hemolytic anemia and splenomegaly. The severity of HbSC disease is variable, but it can be as severe as sickle cell anemia.

HbE is caused when the amino acid glutamic acid is replaced with the amino acid lysine at position 26 in $\beta$-globin, noted as Glu26Lys or E26K. People with HbE disease have a mild hemolytic anemia and mild splenomegaly. In some cases, the HbE mutation is present with HbS. In these cases, a person may have more severe signs and symptoms associated with sickle cell anemia, such as episodes of pain, anemia, and abnormal spleen function.

In certain aspects, the subject is a subject who has been diagnosed as having a hemoglobinopathy selected from the group consisting of hemoglobin C disease, hemoglobin E disease, sickle cell anemia, sickle cell disease (SCD), thalassemia, $\beta$-thalassemia, thalassemia major, thalassemia intermedia, hemoglobin Bart syndrome and hemoglobin H disease.

The cells may be administered as part of a bone marrow or cord blood transplant in an individual that has or has not undergone bone marrow ablative therapy. In one aspect, cells contemplated herein are administered in a bone marrow transplant to an individual that has undergone chemoablative or radioablative bone marrow therapy.

In one aspect, a dose of cells expressing the DBPs is delivered to a subject intravenously. In one aspect, the cells are hematopoietic stem cells which are intravenously administered to a subject.

In one aspect, the effective amount of genome edited cells provided to a subject is at least $2 \times 10^6$ cells/kg, at least $3 \times 10^6$ cells/kg, at least $4 \times 10^6$ cells/kg, at least $5 \times 10^6$ cells/kg, at least $6 \times 10^6$ cells/kg, at least $7 \times 10^6$ cells/kg, at least $8 \times 10^6$ cells/kg, at least $9 \times 10^6$ cells/kg, or at least $10 \times 10^6$ cells/kg, or more cells/kg, including all intervening doses of cells.

In another aspect, the effective amount of genome edited cells provided to a subject is about $2 \times 10^6$ cells/kg, about $3 \times 10^6$ cells/kg, about $4 \times 10^6$ cells/kg, about $5 \times 10^6$ cells/kg, about $6 \times 10^6$ cells/kg, about $7 \times 10^6$ cells/kg, about $8 \times 10^6$ cells/kg, about $9 \times 10^6$ cells/kg, or about $10 \times 10^6$ cells/kg, or more cells/kg, including all intervening doses of cells.

For reasons of completeness, certain aspects of the polypeptides, composition, and methods of the present disclosure are set out in the following numbered clauses:

1. A method for remodeling chromatin architecture in a regulatory region of a gene of interest in a cell, the method comprising:
providing in the cell a DNA-binding polypeptide (DBP) that binds to a sequence in the regulatory region of the gene.

2. The method of clause 1, wherein the remodeling chromatin architecture comprises localized opening of a closed region of the chromatin and increase in expression of the gene.

3. The method of clause 1 or 2, wherein the DBP comprises a plurality of repeat units (RUs) ordered from N-terminus to C-terminus of the DBP to bind to a sequence in the regulatory region of the gene.

4. The method of any one of clauses 1-3, wherein the sequence bound by the DBP is present at a position at least - 100 nucleotides upstream of the transcription start site (TSS) to up to - 400 nucleotides upstream of the TSS.

5. The method of any one of clauses 1-4, wherein the gene of interest is γ-globin gene (*HBG1* and/or *HBG2)* encoding fetal hemoglobin G (HBG).

6. The method of any one of clauses 1-5, wherein the sequence bound by the DBP is present at a position between -350 to -150; -300 to -175; -250 to -150; -230 to -180; or -230 to - 190 with reference to the TSS.

7. The method of any one of clauses 1-6, wherein the sequence bound by the DBP is present between positions -222 to -193 with reference to the TSS in the *HBG1* and/or *HBG2* gene.

8. The method of any one of clauses 1-6, wherein the sequence bound by the DBP is present between positions -227 to -193 with reference to the TSS in the *HBG2* gene.

9. The method of any one of clauses 1-8, wherein the DBP binds to a sub-sequence in the sequence AGCAGTA TCCTCTTGGGGGCCCCTTCCCCA (SEQ ID NO:3) or a complement thereof in the regulatory region of the HBG1 and/or the HBG2 gene, wherein the sub-sequence is at least 9 nucleotides in length.

10. The method of any one of clauses 1-8, wherein the DBP binds to a sub-sequence in the sequence TAAGCA GCAGTATCCTCTTGGGGGCCCCTTCCCCA (SEQ ID NO:8) or a complement thereof in the regulatory region of the HBG2 gene.

11. The method of any one of clauses 1-10, wherein the DBP binds to the sequence TCTT or a complement thereof and to the sequence up to 16 nt present upstream or downstream of the sequence TCTT.

12. The method of any one of clauses 1-10, wherein the DBP binds to the sequence TCTT or a complement thereof and to the sequence up to 16 nt present downstream of the sequence TCTT.

13. The method of any one of clauses 1-10, wherein the DBP binds to the sequence TCTT or a complement thereof and to the sequence up to 15 nt present upstream of the sequence TCTT.

14. The method of any one of clauses 1-13, wherein providing the DBP comprises expressing the DBP in the cell from a nucleic acid integrated into the genome of the cell.

15. The method of any one of clauses 1-13, wherein providing the DBP comprises introducing in the cell a nucleic acid encoding the DBP.

16. The method of any one of clauses 1-13, wherein the nucleic acid encoding the DBP is codon-optimized for expression in the cell.

17. The method of any one of clauses 1-13, wherein providing the DBP comprises introducing the DBP into the cell.

18. The method of any one of clauses 1-17, wherein the cell is a human hematopoietic stem cell.

19. The method of any one of clauses 1-18, wherein the DBP comprises at least 9 RUs, at least 10 RUs, or at least 11 RUs, wherein RUs each comprise the sequence $X_{1-11}X_{12}X_{13}X_{14-33,\ 34,\ or\ 35}$ (SEQ ID NO: 4), the last RU at the C-terminus is a half-repeat comprising the amino acid sequence $X_{1-11}X_{12}X_{13}X_{14-19,\ 20,\ or\ 21}$ (SEQ ID NO: 5), wherein:

$X_{1-11}$ is a chain of 11 contiguous amino acids,

$X_{14-33\ or\ 34\ or\ 35}$ is a chain of 20, 21 or 22 contiguous amino acids,

$X_{14-20\ or\ 21\ or\ 22}$ is a chain of 7, 8 or 9 contiguous amino acids,

$X_{12}X_{13}$ is selected from:

(a) NH, HH, KH, NK, NQ, RH, RN, SS, NN, SN, KN, GN, VN, LN, DN, QN, EN, AN, or FN for binding to guanine (G);

(b) NI, KI, RI, HI, CI, or SI for binding to adenine (A);

(c) NG, HG, KG, RG, VG, IG, EG, MG, YG, AA, EP, VA, or QG for binding to thymine (T);

(d) HD, RD, SD, ND, KD, AD, or YG for binding to cytosine (C);

(e) NV or HN for binding to A or G; and

(f) H* , HA, KA, N* , NA, NC, NS, RA, or S* for binding to A or T or G or C, wherein

(* ) means that the amino acid at $X_{13}$ is absent.

20. The method of any one of clauses 1-19, wherein the RUs of the DBP are arranged from the N-terminus to the C-terminus of DBP to bind to the sequence:

CCTCTTGGGGGCCCCTTCCCCA (SEQ ID NO: 183)

CCTCTTGGGGGCCCCTTCCCC (SEQ ID NO: 184)

CCTCTTGGGGGCCCCTTCC (SEQ ID NO: 185)
CCTCTTGGGGGCCCCTTC (SEQ ID NO: 186)
CCTCTTGGGGGCCCCT (SEQ ID NO: 187)
CCTCTTGGGGGCCCC (SEQ ID NO: 188)
CCTCTTGGGGGCCC (SEQ ID NO:189)
CTTGGGGGCCCCTTCCCA (SEQ ID NO:25)
CTTGGGGGCCCCTTCCCC (SEQ ID NO:36)
CTTGGGGGCCCCTTCCC (SEQ ID NO:215)
CTTGGGGGCCCCTTCC (SEQ ID NO:216)
CTTGGGGGCCCCTTC (SEQ ID NO:217)
CTTGGGGGCCCCTT (SEQ ID NO:218)
ATCCTCTTGGGGGCCCCT (SEQ ID NO:192)
ATCCTCTTGGGGGCCCCTT (SEQ ID NO:193)
ATCCTCTTGGGGGCCCCTTC (SEQ ID NO:194)
ATCCTCTTGGGGGCCCCTTCC (SEQ ID NO: 195)
ATCCTCTTGGGGGCCCCTTCCC (SEQ ID NO: 196)
ATCCTCTTGGGGGCCCCTTCCCC (SEQ ID NO: 197)
ATCCTCTTGGGGGCCC (SEQ ID NO:191)
ATCCTCTTGGGGGC (SEQ ID NO:199)
CCTCTTGGGGGCCCCTTCC (SEQ ID NO: 185)
CCTCTTGGGGGCCCCT (SEQ ID NO: 187)
CCTCTTGGGGGCCCC (SEQ ID NO: 188)
CCTCTTGGGGGCCC (SEQ ID NO: 189)
TAAGCAGCAGTATCCTCTT (SEQ ID NO:219);
AGCAGTATCCTCTTGG (SEQ ID NO:220); or
AGCAGTATCCTCTTGGGG (SEQ ID NO:221).

21. The method of any one of clauses 1-20, wherein the DBP comprises an N-terminus region that binds to T.

22. A recombinant DNA binding polypeptide (DBP) comprising a plurality of repeat units (RUs) ordered from N-terminus to C-terminus of the DBP to bind to a regulatory region of a gene of interest in a cell, wherein binding of the DBP results in remodeling the chromatin architecture from a closed chromatin into an open chromatin architecture, wherein the RUs each comprise the sequence $X_{1-11}X_{12}X_{13}X_{14-33,\ 34,\ or\ 35}$ (SEQ ID NO: 4), the last RU at the C-terminus is a half-repeat comprising the amino acid sequence $X_{1-11}X_{12}X_{13}X_{14-19,\ 20,\ or\ 21}$ (SEQ ID NO: 5), wherein:

$X_{1-11}$ is a chain of 11 contiguous amino acids,
$X_{14-33\ or\ 34\ or\ 35}$ is a chain of 20, 21 or 22 contiguous amino acids,
$X_{14-20\ or\ 21\ or\ 22}$ is a chain of 7, 8 or 9 contiguous amino acids,
$X_{12}X_{13}$ is selected from:

(a) NH, HH, KH, NK, NQ, RH, RN, SS, NN, SN, KN, GN, VN, LN, DN, QN, EN, AN, or FN for binding to guanine (G);
(b) NI, KI, RI, HI, CI, or SI for binding to adenine (A);
(c) NG, HG, KG, RG, VG, IG, EG, MG, YG, AA, EP, VA, or QG for binding to thymine (T);
(d) HD, RD, SD, ND, KD, AD, or YG for binding to cytosine (C);
(e) NV or HN for binding to A or G; and
(f) H* , HA, KA, N* , NA, NC, NS, RA, or S* for binding to A or T or G or C, wherein

(* ) means that the amino acid at $X_{13}$ is absent.

23. The recombinant DBP of clause 22, wherein the DBP binds to a sequence present at a position at least - 100 nucleotides upstream of the transcription start site (TSS) to up to - 400 nucleotides upstream of the TSS of the gene.

24. The recombinant DBP of clause 23, wherein the gene of interest is γ-globin gene (*HBG1* and/or *HBG2)* encoding fetal hemoglobin G (HBG) and the sequence bound by the DBP is present at a position between -350 to -150; -300 to -175; -250 to -150; -230 to -180; or -230 to -190 with reference to the TSS.

25. The recombinant DBP of clause 24, wherein the sequence bound by the DBP is present between positions -222 to -193 with reference to the TSS in the *HBG1* and/or *HBG2* gene.

26. The recombinant DBP of clause 24, wherein the sequence bound by the DBP is present between positions -227 to -193 with reference to the TSS in the *HBG2* gene.

27. The recombinant DBP of any one of clauses 22-26, wherein the DBP binds to a sub-sequence in the sequence AGCAGTATCCTCTTGGGGGCCCCTTCCCCA (SEQ ID NO:2) or a complement thereof in the regulatory region of the HBG1 and/or the HBG2 gene, wherein the sub-sequence is at least 9 nucleotides in length.

28. The recombinant DBP of any one of clauses 22-26, wherein the DBP binds to a sub-sequence in the sequence TAAGCAGCAGTATCCTCTTGGGGGCCCCTTCCCCA (SEQ ID NO:8) or a complement thereof in the regulatory region of the HBG2 gene.

29. The recombinant DBP of any one of clauses 22-28, wherein the DBP binds to the sequence TCTT or a complement thereof and to the sequence up to 16 nt present upstream or downstream of the sequence TCTT.

30. The recombinant DBP of any one of clauses 22-28, wherein the DBP binds to the sequence TCTT or a complement thereof and to the sequence up to 16 nt present downstream of the sequence TCTT.

31. The recombinant DBP of any one of clauses 22-28, wherein the DBP binds to the sequence TCTT or a complement thereof and to the sequence up to 15 nt present upstream of the sequence TCTT.

32. The recombinant DBP of any one of clauses 22-31, wherein the RUs of the DBP are arranged from the N-terminus to the C-terminus of DBP to bind to the sequence:

CCTCTTGGGGGCCCCTTCCCCA (SEQ ID NO:183)
CCTCTTGGGGGCCCCTTCCCC (SEQ ID NO:184)
CCTCTTGGGGGCCCCTTCC (SEQ ID NO:185)
CCTCTTGGGGGCCCCTTC (SEQ ID NO:186)
CCTCTTGGGGGCCCCT (SEQ ID NO:187)
CCTCTTGGGGGCCCC (SEQ ID NO: 188)
CCTCTTGGGGGCCC (SEQ ID NO: 189)
CTTGGGGGCCCCTTCCCCA (SEQ ID NO:25)
CTTGGGGGCCCCTTCCCC (SEQ ID NO:36)
CTTGGGGGCCCCTTCCC (SEQ ID NO:215)
CTTGGGGGCCCCTTCC (SEQ ID NO:216)
CTTGGGGGCCCCTTC (SEQ ID NO:217)
CTTGGGGGCCCCTT (SEQ ID NO:218)
ATCCTCTTGGGGGCCCCT (SEQ ID NO:192)
ATCCTCTTGGGGGCCCCTT (SEQ ID NO:193)
ATCCTCTTGGGGGCCCCTTC (SEQ ID NO:194)
ATCCTCTTGGGGGCCCCTTCC (SEQ ID NO:195)
ATCCTCTTGGGGGCCCCTTCCC (SEQ ID NO: 196)
ATCCTCTTGGGGGCCCCTTCCCC (SEQ ID NO: 197)
ATCCTCTTGGGGGCCC (SEQ ID NO:191)
ATCCTCTTGGGGGC (SEQ ID NO:199)
CCTCTTGGGGGCCCCTTCC (SEQ ID NO: 185)
CCTCTTGGGGGCCCCT (SEQ ID NO: 187)
CCTCTTGGGGGCCCC (SEQ ID NO: 188)
CCTCTTGGGGGCCC (SEQ ID NO:189)
TAAGCAGCAGTATCCTCTT (SEQ ID NO:219);
AGCAGTATCCTCTTGG (SEQ ID NO:220); or
AGCAGTATCCTCTTGGGG (SEQ ID NO:221).

33. A recombinant DNA binding polypeptide (DBP) comprising a plurality of repeat units (RUs) ordered from N-terminus to C-terminus of the DBP to bind to the nucleic acid sequence CCTCTTGGGGGC (SEQ ID NO:201) or a complement thereof present in the fetal $\gamma$-globin gene promoter,
wherein eleven of the RUs each comprise the sequence $X_{1-11}X_{12}X_{13}X_{14-33,\ 34,\ or\ 35}$ (SEQ ID NO: 4), the last RU at the C-terminus is a half-repeat comprising the amino acid sequence $X_{1-11}X_{12}X_{13}X_{14-19,\ 20,\ or\ 21}$ (SEQ ID NO: 5), wherein:

$X_{1-11}$ is a chain of 11 contiguous amino acids,
$X_{14-33\ or\ 34\ or\ 35}$ is a chain of 20, 21 or 22 contiguous amino acids,
$X_{14-20\ or\ 21\ or\ 22}$ is a chain of 7, 8 or 9 contiguous amino acids,
$X_{12}X_{13}$ is selected from:

(a) NH, HH, KH, NK, NQ, RH, RN, SS, NN, SN, KN, GN, VN, LN, DN, QN, EN, AN, or FN for binding to guanine (G);
(b) NI, KI, RI, HI, CI, or SI for binding to adenine (A);

(c) NG, HG, KG, RG, VG, IG, EG, MG, YG, AA, EP, VA, or QG for binding to thymine (T);
(d) HD, RD, SD, ND, KD, AD, or YG for binding to cytosine (C);
(e) NV or HN for binding to A or G; and
(f) H*, HA, KA, N*, NA, NC, NS, RA, or S* for binding to A or T or G or C, wherein (*) means that the amino acid at $X_{13}$ is absent.

34. The recombinant DBP of clause33, wherein the DBP binds to the nucleic acid sequence CCTCTTGGGGGC (SEQ ID NO:201) and wherein $X_{12}X_{13}$ in the 12 RUs from N-terminus to C-terminus are HD, HD, NG, HD, NG, NG, NH, NH, NH, NH, NH, and HD.

35. The recombinant DBP of clause34, wherein the DBP comprises at least one additional RU at the N-terminus that binds to T such that the DBP binds to the nucleotide sequence: TCCTCTTGGGGGC. (SEQ ID NO:200).

36. The recombinant DBP of clause 35, wherein the DBP comprises at least one additional RU at the N-terminus that binds to A such that the DBP binds to the nucleotide sequence: ATCCTCTTGGGGGC. (SEQ ID NO: 199).

37. The recombinant DBP of clause 36, wherein the $X_{12}X_{13}$ in the 14 RUs from N-terminus to C-terminus are NI, NG, HD, HD, NG, HD, NG, NG, NH, NH, NH, NH, NH, and HD.

38. The recombinant DBP of clause 36 or 37, wherein the DBP comprises at one additional RU at the C-terminus that binds to C such that the DBP binds to the nucleotide sequence: ATCCTCTTGGGGGCC, (SEQ ID NO: 198), wherein the one additional RU at the C-terminus that binds to C is positioned immediately before the half-repeat unit.

39. The recombinant DBP of clause 38, wherein the $X_{12}X_{13}$ in the RUs from N-terminus to C-terminus are NI, NG, HD, HD, NG, HD, NG, NG, NH, NH, NH, NH, NH, HD, HD, and HD.

40. The recombinant DBP of any one of clauses 33-39, wherein the plurality of RUs are arranged from N-terminus to C-terminus to bind to the nucleotide sequence:
ATCCTCTTGGGGGCCC; (SEQ ID NO:191); ATCCTCTTGGGGGCCCCT; (SEQ ID NO:192); ATCCTCTTGGGGGCCCCTT; (SEQ ID NO:193); ATCCTCTTGGGGGCCCCTTC; (SEQ ID NO:194); ATCCTCTTGGGGGCCCCTTCC; (SEQ ID NO:195); ATCCTCTTGGGGGCCCCTTCCC; (SEQ ID NO:196); or ATCCTCTTGGGGGCCCCTTCCCC. (SEQ ID NO:197).

41. The recombinant DBP of any one of clauses 33-40, wherein the plurality of RUs consist of the RUs of any one of clauses 33-40.

42. The recombinant DBP of clause 33 or 34, wherein the DBP comprises at one additional RU at the C-terminus that binds to C such that the DBP binds to the nucleotide sequence: CCTCTTGGGGGCC, (SEQ ID NO:190), wherein the one additional RU at the C-terminus that binds to C is positioned immediately before the half-repeat unit.

43. The recombinant DBP of clause 42, wherein the $X_{12}X_{13}$ in the RUs from N-terminus to C-terminus are HD, HD, NG, HD, NG, NG, NH, NH, NH, NH, NH, HD, and HD.

44. The recombinant DBP of clauses 42 or 43, wherein the plurality of RUs are arranged from N-terminus to C-terminus to bind to the nucleotide sequence:

CCTCTTGGGGGCCCCTTCCCCAC; (SEQ ID NO:182); CCTCTTGGGGGCCCCTTCCCCA; (SEQ ID NO:183); CCTCTTGGGGGCCCCTTCCCC; (SEQ ID NO:184);
CCTCTTGGGGGCCCCTTCC; (SEQ ID NO:185); CCTCTTGGGGGCCCCTTC; (SEQ ID NO:186); CCTCTTGGGGGCCCCT; (SEQ ID NO:187); CCTCTTGGGGGCCCC; (SEQ ID NO:188); or CCTCTTGGGGGCCC. (SEQ ID NO:189).

45. The recombinant DBP of any one of clauses 42-44, wherein the plurality of RUs consist of the RUs of any one of clauses 42-44.

46. The recombinant DBP of any one of clauses 22-45, wherein $X_{1-11}$ is at least 80% identical to LTPEQVVAIAS (SEQ ID NO: 6).

47. The recombinant DBP of any one of clauses 22-46, wherein $X_{1-11}$ is at least 80% identical to LTPDQVVAIAS (SEQ ID NO: 11).

48. The recombinant DBP of any one of clauses 22-47, wherein the chain of 20, 21, or 22 contiguous amino acids is at least 80% identical to GGKQALETVQRLLPVLCQDHG (SEQ ID NO: 15).

49. The recombinant DBP of any one of clauses 22-48, wherein the chain of 7, 8 or 9 contiguous amino acids is at least 80% identical to GGRPALE (SEQ ID NO: 7).

50. The recombinant DBP of any one of clauses 22-49, further comprising an N-terminus region present before the first RU at the N-terminus, wherein the N-terminus region binds to T.

51. The recombinant DBP of clause 50, wherein the N-terminus region comprises an amino acid sequence at least 80%, at least 85%, at least 90%, at least 95%, or 100% identical to the amino acid sequence of SEQ ID NO: 150.

52. A recombinant DNA binding protein (DBP) comprising a plurality of repeat units (RUs) ordered from N-terminus to C-terminus of the DBP to bind to the nucleic acid sequence CACCCTGTGGAGCCACAC (SEQ ID NO:181) or a complement thereof present in the adult β-globin (HBB) gene promoter,
wherein seventeen of the RUs each comprise the sequence $X_{1-11}X_{12}X_{13}X_{14-33, \, 34, \, or \, 35}$ (SEQ ID NO: 4), the last RU at the C-terminus is a half-repeat comprising the amino acid sequence $X_{1-11}X_{12}X_{13}X_{14-19, \, 20, \, or \, 21}$ (SEQ ID NO: 5), wherein:

$X_{1-11}$ is a chain of 11 contiguous amino acids,
$X_{14-33 \, or \, 34 \, or \, 35}$ is a chain of 20, 21 or 22 contiguous amino acids,
$X_{14-20 \, or \, 21 \, or \, 22}$ is a chain of 7, 8 or 9 contiguous amino acids,
$X_{12}X_{13}$ is selected from:

(a) NH, HH, KH, NK, NQ, RH, RN, SS, NN, SN, KN, GN, VN, LN, DN, QN, EN, AN, or FN for binding to guanine (G);
(b) NI, KI, RI, HI, CI, or SI for binding to adenine (A);
(c) NG, HG, KG, RG, VG, IG, EG, MG, YG, AA, EP, VA, or QG for binding to thymine (T);
(d) HD, RD, SD, ND, KD, AD, or YG for binding to cytosine (C);
(e) NV or HN for binding to A or G; and
(f) H*, HA, KA, N*, NA, NC, NS, RA, or S* for binding to A or T or G or C, wherein (*) means that the amino acid at $X_{13}$ is absent.

53. The recombinant DBP of clause 52 wherein the DBP binds to the nucleic acid sequence CACCCTGTGGAG CCACAC (SEQ ID NO:181) and wherein $X_{12}X_{13}$ in the RUs from N-terminus to C-terminus are HD, NI, HD, HD, HD, NG, NH, NG, NH, NH, NI, NH, HD, HD, NI, HD, NI, and HD.

54. The recombinant DBP of clause 52 or 53, wherein the plurality of RUs consist of the RUs of clause 20 or 21.

55. The recombinant DBP of any one of clauses 52-54, wherein $X_{1-11}$ is at least 80% identical to LTPEQVVAIAS (SEQ ID NO: 6).

56. The recombinant DBP of any one of clauses 52-54, wherein $X_{1-11}$ is at least 80% identical to LTPDQVVAIAS (SEQ ID NO: 11).

57. The recombinant DBP of any one of clauses 52-56, wherein the chain of 20, 21, or 22 contiguous amino acids is at least 80% identical to GGKQALETVQRLLPVLCQDHG (SEQ ID NO: 15).

58. The recombinant DBP of any one of clauses 52-56, wherein the chain of 7, 8 or 9 contiguous amino acids is at least 80% identical to GGRPALE (SEQ ID NO: 7).

59. The recombinant DBP of any one of clauses 52-58, further comprising an N-terminus region present before the first RU at the N-terminus, wherein the N-terminus region binds to T.

60. The recombinant DBP of clause 59, wherein the N-terminus region comprises an amino acid sequence at least 80%, at least 85%, at least 90%, at least 95%, or 100% identical to the amino acid sequence of SEQ ID NO: 150.

61. A recombinant DNA binding protein (DBP) comprising a plurality of repeat units (RUs) ordered from N-terminus to C-terminus of the DBP to bind to the nucleic acid sequence TGCTTTTATCACAGGCT (SEQ ID NO:180) or a complement thereof present in the BCL11A gene promoter,
wherein sixteen of the RUs each comprise the sequence $X_{1-11}X_{12}X_{13}X_{14-33, \, 34, \, or \, 35}$ (SEQ ID NO: 4), the last RU at the C-terminus is a half-repeat comprising the amino acid sequence $X_{1-11}X_{12}X_{13}X_{14-19, \, 20, \, or \, 21}$ (SEQ ID NO: 5), wherein:

$X_{1-11}$ is a chain of 11 contiguous amino acids,
$X_{14-33 \, or \, 34 \, or \, 35}$ is a chain of 20, 21 or 22 contiguous amino acids,
$X_{14-20 \, or \, 21 \, or \, 22}$ is a chain of 7, 8 or 9 contiguous amino acids,
$X_{12}X_{13}$ is selected from:

(a) NH, HH, KH, NK, NQ, RH, RN, SS, NN, SN, KN, GN, VN, LN, DN, QN, EN, AN, or FN for binding to guanine (G);
(b) NI, KI, RI, HI, CI, or SI for binding to adenine (A);
(c) NG, HG, KG, RG, VG, IG, EG, MG, YG, AA, EP, VA, or QG for binding to thymine (T);
(d) HD, RD, SD, ND, KD, AD, or YG for binding to cytosine (C);
(e) NV or HN for binding to A or G; and
(f) H*, HA, KA, N*, NA, NC, NS, RA, or S* for binding to A or T or G or C, wherein (*) means that the amino acid at $X_{13}$ is absent.

62. The recombinant DBP of clause 61, wherein the DBP binds to the nucleic acid sequence TGCTTTTATCACA GGCT (SEQ ID NO:180) and wherein $X_{12}X_{13}$ in the RUs from N-terminus to C-terminus are NG, NH, HD, NG, NG, NG, NG, NI, NG, HD, NI, HD, NI, NH, NH, HD, and NG.

63. The recombinant DBP of clause 61 or 62, wherein the plurality of RUs consist of the RUs of clause 61 or 62.

64. The recombinant DBP of any one of clauses 61-63, wherein $X_{1-11}$ is at least 80% identical to LTPEQVVAIAS (SEQ ID NO: 6).

65. The recombinant DBP of any one of clauses 61-63, wherein $X_{1-11}$ is at least 80% identical to LTPDQVVAIAS (SEQ ID NO: 11).

66. The recombinant DBP of any one of clauses 61-65, wherein the chain of 20, 21, or 22 contiguous amino acids is at least 80% identical to GGKQALETVQRLLPVLCQDHG (SEQ ID NO: 15).

67. The recombinant DBP of any one of clauses 61-66, wherein the chain of 7, 8 or 9 contiguous amino acids is at least 80% identical to GGRPALE (SEQ ID NO: 7).

68. The recombinant DBP of any one of clauses 61-67, further comprising an N-terminus region present before the first RU at the N-terminus, wherein the N-terminus region binds to T.

69. The recombinant DBP of clause 68, wherein the N-terminus region comprises an amino acid sequence at least 80%, at least 85%, at least 90%, at least 95%, or 100% identical to the amino acid sequence of SEQ ID NO: 150.

70. A nucleic acid encoding the recombinant DBP of any of clauses 22-69.

71. The nucleic acid of clause 70, wherein the nucleic acid is operably linked to a promoter sequence that confers expression of the DBP.

72. The nucleic acid of clause 70 or 71, wherein the sequence of the nucleic acid is codon optimized for expression of the DBP in a human cell.

73. The nucleic acid of any one of clauses 70-72, wherein the nucleic acid is a deoxyribonucleic acid (DNA).

74. The nucleic acid of any one of clauses 70-72, wherein the nucleic acid is a ribonucleic acid (RNA).

75. A vector comprising the nucleic acid of any of clauses 70-73.

76. The vector of clause 75, wherein the vector is a viral vector.

77. The vector of clause 76, wherein the viral vector is a lentiviral vector.

78. A mammalian cell comprising the nucleic acid of any of clauses 70-74 or the vector of any one of clauses 75-77.

79. A mammalian cell that expresses the DBP of any of clauses 22-69.

80. The mammalian cell of clause 78 or 79, wherein the mammalian cell is a human cell.

81. The mammalian cell of any one of clauses 78-80, wherein the mammalian cell is present in a subject.

82. The mammalian cell of any one of clauses 78-80, wherein the cell is an ex vivo cell.

83. The mammalian cell of any one of clauses 78-82, wherein the cell is a cancer cell.

84. The mammalian cell of any one of clauses 78-82 wherein the mammalian cell is a hematopoietic progenitor cell.

85. The mammalian cell of any one of clauses 78-82, wherein the mammalian cell is an erythroid progenitor.

86. The mammalian cell of any one of clauses 78-82, wherein the cell is a pluripotent stem cell.

87. The mammalian cell of clause 86, wherein the cell is an induced pluripotent stem cell.

88. A pharmaceutical composition comprising the DBP of any of clauses 22-69 and a pharmaceutically acceptable excipient.

89. A pharmaceutical composition comprising the nucleic acid of any of clauses 70-73 or the vector of any of one of clauses 75-77 and a pharmaceutically acceptable excipient.

90. A pharmaceutical composition comprising the mammalian cell of any one of clauses 78-87.

91. A method for increasing expression of fetal hemoglobin-$\gamma$ (HBG) in a subject in need thereof, the method comprising administering to the subject the pharmaceutical composition of any one of clauses 88-90.

92. The method of clause 91, wherein the subject has sickle cell anemia.

93. The method of clause 91, wherein the subject has thalassemia.

94. A nucleic acid comprising a sequence of nucleotides having at least 50%, at least 60%, at least 70%, at least 75%, at least 80%, at least 85% identity to the sequence of SEQ ID NO: 145, 146, or 147.

95. A DNA binding protein comprising an amino acid sequence having at least at least 80%, at least 85%, at least 90%, at least 95%, or 100% identical to the amino acid sequence of SEQ ID NO: 148, 149, 156, or 157.

96. A method for modulating expression of a gene in a cell, the method comprising introducing into the cell a DNA binding polypeptide (DBP), or a nucleic acid encoding the DBP, that binds a sequence in regulatory region of a gene bound by a transcription factor (TF), thereby displacing the TF and modulating expression of the gene, wherein the DBP competes with the TF for binding to a regulatory sequence comprising CACC box or GATA site,

    wherein the DBP binds to the sequence CACC or the complement thereof in the CACC box and at least eight, at least nine, at least ten, at least eleven, at least twelve, at least thirteen, or at least fourteen nucleotides 5' and/or 3' of the CACC sequence or the complement thereof, or

    wherein the DBP binds to the sequence GATA or the complement thereof at the GATA site and at least eight, at least nine, at least ten, at least eleven, at least twelve, at least thirteen, or at least fourteen nucleotides 5' and/or 3' of the GATA sequence or the complement thereof.

97. The method of clause 96, wherein the DBP binds to the CACC box present at position -90 upstream of the transcription start site of the human hemoglobin B (HBB) gene.

98. The method of clause 97, wherein the TF is a transcription activator and wherein the binding of the DBP to the CACC box results in decreased expression of HBB protein.

99. The method of any one of clauses 96-98, wherein the DBP is the DBP according to any one of clauses 52-60.

100. The method of clause 99, wherein the DBP binds to the GATA site in + 58 DHS of BCL11A enhancer.

101. The method of clause 96, wherein the TF is a transcriptional activator and binding of the DBP to the GATA site results in reduced expression of BCL11A protein.

102. The method of clause 100 or 101, wherein the DBP comprises a plurality of repeat units (RUs) arranged from N-terminus to C-terminus to bind to the complement of GATA at the GATA site.

103. The method of any one of clauses 100-102, wherein the DBP comprises a plurality of RUs arranged from N-terminus to C-terminus to bind to the sequence TGCTTTTATCACAGGCT (SEQ ID NO: 180) at the GATA site.

104. The method of any one of clauses 100-103, wherein the DBP is the DBP according to any one of clauses 29-37.

105. The method of any one of clauses 96-104, further comprising introducing into the cell the DBP or the nucleic acid encoding the DBP.

106. The method of clause 105, wherein the nucleic acid is a deoxyribonucleic acid (DNA).

107. The method of clause 106, wherein the nucleic acid is a ribonucleic acid (RNA).

108. The method of clause 105 or 106, wherein the sequence of the nucleic acid is codon optimized for expression in a human cell.

109. The method of any one of clauses 96-108, wherein the cell is a human cell.

110. The method of any one of clauses 96-109, wherein the cell is a cancer cell.

111. The method of any one of clauses 96-110, wherein the introducing comprises administering the DBP or the nucleic acid encoding the DBP to a subject.

112. The method of clause 111, wherein the administering comprises parenteral administration.

113. The method of clause 111, wherein the administering comprises intravenous, intramuscular, intrathecal, or subcutaneous administration.

## EXAMPLES

As can be appreciated from the disclosure provided above, the present disclosure has a wide variety of applications. Accordingly, the following examples are put forth so as to provide those of ordinary skill in the art with a complete disclosure and description of how to make and use the present invention, and are not intended to limit the scope of what the inventors regard as their invention nor are they intended to represent that the experiments below are all or the only experiments performed. Those of skill in the art will readily recognize a variety of noncritical parameters that could be changed or modified to yield essentially similar results. Thus, the following examples are put forth so as to provide those of ordinary skill in the art with a complete disclosure and description of how to make and use the present invention, and are not intended to limit the scope of what the inventors regard as their invention nor are they intended to represent that the experiments below are all or the only experiments performed. Efforts have been made to ensure accuracy with respect to numbers used (e.g. amounts, dimensions, etc.) but some experimental errors and deviations should be accounted for.

## MATERIALS AND METHODS

**Cell culture.** HUDEP-2 cells (PMID 23533656) were grown in StemSpan SFEM (Stemcell Technologies) supplemented with 2% PennStrep, 1% L-Glutamine, 1 ug/mL doxycycline, 100 ng/mL recombinant human SCF (Peprotech), 3 IU/mL recombinant human EPO (Peprotech), and 10-6 M dexamethasone.

**TALE transfection.** mRNA was in vitro transcribed using T7 mScript™ Standard mRNA Production System (Cellscript) following manufacturer's instructions and RNA size and concentrations were determined using the Advanced Analytical Fragment Analyzer (Agilent). $5 \times 10^5$ HUDEP-2 cells were electroporated with 2 ug, 4ug, 5ug, or 10ug TALE mRNA and 100 ul BTXpress solution (BTX) in 96-well cuvette (BTX) using the ECM 830 Square Wave Electroporation System (Harvard Apparatus) and HT200 Plate Handler (BTX) with 250mS interval, 250V for 5msec pulse. Electroporated cells were transferred to 12 or 6-well plates, and RNA was harvested 12, 24, or 48 hours later. After the initial time course experiment, all samples were harvested at 48 hours.

**Generation of Clonal Lines.** $5 \times 10^6$ HUDEP-2 cells were transfected with TALEN pairs recognizing the AAVS1 safe harbor locus (5'-TTTCTGTCACCAATCCT-3' (SEQ ID NO: 143) and 5'-TCCCCTCCACCCCACAGT-3' (SEQ ID NO: 144); 2.5 ug mRNA per TALEN monomer), together with 2.5 ug AAVS1 donor plasmid using the Amaxa Human CD34+ Cell Nucleofector Kit (Lonza) on an Amaxa Nucleofector II Device (Lonza). Donor plasmid contains a splice acceptor site followed by T2A and GFP to utilize an endogenous promoter to drive expression of GFP after integration and an EF1alpha-driven TALE followed by WPRE (sequences provided below). Cells were maintained in a T-25 flask and media was changed every 24 hours post-transfection for two days. Approximately 7-12 days post-transfection, single cells were sorted for GFP expression into 96-well plates using the MoFlo Astrios (Beckman Coulter). Cells were maintained in 96-well plates for ~12 days with doxycycline supplemented every two days and media changes on days 8 and 10. Wells containing cells were identified, consolidated, and further maintained.

**Globin TaqMan qPCR.** Total RNA was extracted from 2-5 x $10^5$ HUDEP-2 cells from clonal lines expressing stably-integrated TALEs or WT cells transfected with TALE mRNA using the RNeasy Micro Kit (Qiagen), and 100ng RNA was reverse transcribed with iScript Reverse Transcription Supermix (Bio-Rad). qPCRs were run with multiplexed Taqman primer/probe sets specific to HBG (Hs00361131_g1, VIC) and HBB (Hs00747223_g1, FAM) from ThermoFisher Scientific, cDNA diluted at a 1:10 ratio, and SsoAdvanced Universal Probes Supermix (Bio-Rad) on a Bio-Rad CFX96 Real Time System. Primer/probe sets were confirmed to be accurate and robust in multiplexing assays as no difference was observed when tested individually or multiplexed. In TALE transfection experiments, HBG measurements were normalized to measurements of HBB and compared to transfected control cells in biological triplicate. For analysis of stably-integrated TALE HUDEP-2 cell lines, amount of HBG expression was normalized to total HBB and HBG expression, with 2 - 5 measurements taken at different timepoints in culture per sample.

**FLAG Immunofluorescence.** In a 24-well poly-l-lysine coated cover glass bottom plate (BioMedTech Laboratories, Inc.), 7.5 x $10^4$ cells were deposited in a 30 ul droplet to the center of the well and allowed to settle for 40 minutes. Cells were fixed with 4% PFA (Polysciences Inc, #18814-10) for 10 minutes at room temperature, washed 3 times for 3 minutes each with PBS, permeabilized with 0.25% Triton (Triton X-100) in PBS for 10 minutes, and washed 3 times for 5 minutes each with PBS. Cover glass bottoms were incubated with blocking solution (2% BSA-PBS (Jackson Immunoresearch, #001-000-161)) for 45 minutes, then incubated with a 1:500 dilution of primary mouse monoclonal ANTI-FLAG® M2 antibody (F1804; Sigma) in 2% BSA-PBS for 2 hours, washed 3 times for 3 minutes each with 0.05% Tween (Bio-rad, #161-0781) in PBS, incubated with a 1:500 dilution of secondary antibody conjugated to either Cy3 or AlexaFluor-647 (Jackson Labs) for 1 hour, and washed 3 times for 5 minutes each with 0.05% Tween-PBS with the second wash containing 0.1 mg/mL DAPI solution (100 ng/mL in 1x PBS) and washing for 10 minutes. Lastly, cell samples in individual wells were mounted with 7-10 uL Prolong Gold (Molecular Probes P36930) antifade, sealed with 12 mm coverglasses (1.5, Electron Microscopy Sciences), and cured either overnight or for at least 2 hours prior to imaging. Samples were imaged using an inverted Nikon Eclipse Ti widefield microscope equipped with an Andor Zyla 4.2CL10 CMOS camera with a 4.2-megapixel sensor and 6.5 $\mu$m pixel size (18.8 mm diagonal FOV). Focused 3D cell images were acquired using a 40x 0.9 NA air objective. Acquired images were subject to 100 rounds of iterative blind deconvolution using Microvolution software (Microvolution, CA) to minimize the effect of out-of-focus blurring that is inherent to widefield microscopy optics. Deconvolved images were processed using in-house Matlab (version 2017B, Mathworks, Natick, MA) scripts to numerically estimate the average FLAG protein content in every cell nucleus, and for downstream statistical analysis.

**Globin FACS.** Approximately 1 x $10^6$ cells were harvested, fixed with 4% PFA for 15 minutes, permeabilized with acetone, incubated with 1:200 anti-HbF-APC (MHFH05; ThermoFisher) and 1:400 anti-Hemoglobin β (sc-21757; Santa Cruz) for at least 20 minutes at 4°C, rinsed with 0.5% BSA-PBS, and stained with DAPI for 15 minutes. FACS was run on a CytoFLEX S (Beckman Coulter).

**Table 1. Target sequences from $\gamma$-globin proximal promoter.**

| TALE Protein | Target Sequence | SEQ ID NO |
|---|---|---|
| HBG-A11+6 | TATCCTCTTGGGGGCCCCTTCCCC | 158 |
| HBG-A11+5 | TATCCTCTTGGGGGCCCCTTCCC | 159 |
| HBG-A11+4 | TATCCTCTTGGGGGCCCCTTCC | 160 |
| HBG-A11+3 | TATCCTCTTGGGGGCCCCTTC | 161 |
| HBG-A11+2 | TATCCTCTTGGGGGCCCCTT | 162 |
| HBG-A11+1 | TATCCTCTTGGGGGCCCCT | 163 |
| HBG-A11 | TATCCTCTTGGGGGCCCC | 164 |
| HBG-A11-1 | TATCCTCTTGGGGGCCC | 165 |
| HBG-A11-2 | TATCCTCTTGGGGGCC | 166 |
| HBG-A11-3 | TATCCTCTTGGGGGC | 167 |
| HBG-B1+3 | TCCTCTTGGGGGCCCCTTCCCCAC | 168 |
| HBG-B1+2 | TCCTCTTGGGGGCCCCTTCCCCA | 169 |
| HBG-B1+1 | TCCTCTTGGGGGCCCCTTCCCC | 170 |

(continued)

| TALE Protein | Target Sequence | SEQ ID NO |
|---|---|---|
| B1 | TCCTCTTGGGGGCCCCTTCCC | 171 |
| HBG-B1-1 | TCCTCTTGGGGGCCCCTTCC | 172 |
| HBG-B1-2 | TCCTCTTGGGGGCCCCTTC | 173 |
| HBG-B1-3 | TCCTCTTGGGGGCCCCTT | 174 |
| HBG-B1-4 | TCCTCTTGGGGGCCCCT | 175 |
| HBG-B1-5 | TCCTCTTGGGGGCCCC | 176 |
| HBG-B1-6 | TCCTCTTGGGGGCCC | 177 |
| HBB-90-TLJC4 | TCACCCTGTGGAGCCACAC | 178 |
| BCL11A_e58_TL1+2 | TTGCTTTTATCACAGGCT | 179 |

### Table 2. TALE Protein Domain Sequences

| TALE Protein Domain | Sequence | SEQ ID NO |
|---|---|---|
| N-terminus region | MVDLRTLGYSQQQQEKIKPKVRSTVAQHH EALVGHGFTHAHIVALSQHPAALGTVAVK YQDMIAALPEATHEAIVGVGKQWSGARAL EALLTVAGELRGPPLQLDTGQLLKIAKRGG<br><br>VTAVEAVHAWRNALTGA | 150 |
| C-terminus region | SIVAQLSRPDPALAALTNDHLVALACLGGR PALDAVKKGLPHAPALIKRTNRRIPERTSHR VA | 151 |
| NI RVD (A) | LTPDQVVAIASNIGGKQALETVQRLLPVLC QDHG | 23 |
| NG RVD (T) | LTPDQVVAIASNGGGKQALETVQRLLPVLC QDHG | 22 |
| HD RVD (C) | LTPDQVVAIASHDGGKQALETVQRLLPVLC QDHG | 24 |
| NH RVD (G) | LTPDQVVAIASNHGGKQALETVQRLLPVLC QDHG | 21 |
| NI last half repeat (A) | LTPDQVVAIASNIGGRPALE | 152 |
| NG last half repeat (T) | LTPDQVVAIASNGGGRPALE | 153 |
| HD last half repeat (C) | LTPDQVVAIASHDGGRPALE | 154 |
| NH last half repeat (G) | LTPDQVVAIASNHGGRPALE | 155 |

HBG-A11 protein sequence:

[00192]     MDYKDHDGDYKDHDIDYKDDDDKMAPKKKRKVGIHRGVPMVDLRTL GYSQQQQEKIKPKVRSTVAQHHEALVGHGFTHAHIVALSQHPAALGTVAVKYQDMIA ALPEATHEAIVGVGKQWSGARALEALLTVAGELRGPPLQLDTGQLLKIAKRGGVTAVE AVHAWRNALTGAPLNLTPDQVVAIASNIGGKQALETVQRLLPVLCQDHGLTPDQVVAI ASNGGGKQALETVQRLLPVLCQDHGLTPDQVVAIASHDGGKQALETVQRLLPVLCQD HGLTPDQVVAIASHDGGKQALETVQRLLPVLCQDHGLTPDQVVAIASNGGGKQALETV QRLLPVLCQDHGLTPDQVVAIASHDGGKQALETVQRLLPVLCQDHGLTPDQVVAIASN GGGKQALETVQRLLPVLCQDHGLTPDQVVAIASNGGGKQALETVQRLLPVLCQDHGL TPDQVVAIASNHGGKQALETVQRLLPVLCQDHGLTPDQVVAIASNHGGKQALETVQRL LPVLCQDHGLTPDQVVAIASNHGGKQALETVQRLLPVLCQDHGLTPDQVVAIASNHGG KQALETVQRLLPVLCQDHGLTPDQVVAIASNHGGKQALETVQRLLPVLCQDHGLTPDQ

VVAIASHDGGKQALETVQRLLPVLCQDHGLTPDQVVAIASHDGGKQALETVQRLLPVL CQDHGLTPDQVVAIASHDGGKQALETVQRLLPVLCQDHGLTPDQVVAIASHDGGRPAL ESIVAQLSRPDPALAALTNDHLVALACLGGRPALDAVKKGLPHAPALIKRTNRRIPERTS HRVAGS (SEQ ID NO: 156)

HBG-A11-3 protein sequence:

        MDYKDHDGDYKDHDIDYKDDDDKMAPKKKRKVGIHRGVPMVDLRTL GYSQQQQEKIKPKVRSTVAQHHEALVGHGFTHAHIVALSQHPAALGTVAVKYQDMIA ALPEATHEAIVGVGKQWSGARALEALLTVAGELRGPPLQLDTGQLLKIAKRGGVTAVE AVHAWRNALTGAPLNLTPDQVVAIASNIGGKQALETVQRLLPVLCQDHGLTPDQVVAI ASNGGGKQALETVQRLLPVLCQDHGLTPDQVVAIASHDGGKQALETVQRLLPVLCQD HGLTPDQVVAIASHDGGKQALETVQRLLPVLCQDHGLTPDQVVAIASNGGGKQALETV QRLLPVLCQDHGLTPDQVVAIASHDGGKQALETVQRLLPVLCQDHGLTPDQVVAIASN GGGKQALETVQRLLPVLCQDHGLTPDQVVAIASNGGGKQALETVQRLLPVLCQDHGL TPDQVVAIASNHGGKQALETVQRLLPVLCQDHGLTPDQVVAIASNHGGKQALETVQRL LPVLCQDHGLTPDQVVAIASNHGGKQALETVQRLLPVLCQDHGLTPDQVVAIASNHGG KQALETVQRLLPVLCQDHGLTPDQVVAIASNHGGKQALETVQRLLPVLCQDHGLTPDQ VVAIASHDGGRPALESIVAQLSRPDPALAALTNDHLVALACLGGRPALDAVKKGLPHA PALIKRTNRRIPERTSHRVAGS (SEQ ID NO: 148)

HBG-B 1 protein sequence:

MDYKDHDGDYKDHDIDYKDDDDKMAPKKKRKVGIHRGVPMVDLRTL
GYSQQQQEKIKPKVRSTVAQHHEALVGHGFTHAHIVALSQHPAALGTVAVKYQDMIA
ALPEATHEAIVGVGKQWSGARALEALLTVAGELRGPPLQLDTGQLLKIAKRGGVTAVE
AVHAWRNALTGAPLNLTPDQVVAIASHDGGKQALETVQRLLPVLCQDHGLTPDQVVA
IASHDGGKQALETVQRLLPVLCQDHGLTPDQVVAIASNGGGKQALETVQRLLPVLCQD
HGLTPDQVVAIASHDGGKQALETVQRLLPVLCQDHGLTPDQVVAIASNGGGKQALETV
QRLLPVLCQDHGLTPDQVVAIASNGGGKQALETVQRLLPVLCQDHGLTPDQVVAIASN
HGGKQALETVQRLLPVLCQDHGLTPDQVVAIASNHGGKQALETVQRLLPVLCQDHGL
TPDQVVAIASNHGGKQALETVQRLLPVLCQDHGLTPDQVVAIASNHGGKQALETVQRL
LPVLCQDHGLTPDQVVAIASNHGGKQALETVQRLLPVLCQDHGLTPDQVVAIASHDGG
KQALETVQRLLPVLCQDHGLTPDQVVAIASHDGGKQALETVQRLLPVLCQDHGLTPDQ
VVAIASHDGGKQALETVQRLLPVLCQDHGLTPDQVVAIASHDGGKQALETVQRLLPVL
CQDHGLTPDQVVAIASNGGGKQALETVQRLLPVLCQDHGLTPDQVVAIASNGGGKQA
LETVQRLLPVLCQDHGLTPDQVVAIASHDGGKQALETVQRLLPVLCQDHGLTPDQVVA
IASHDGGKQALETVQRLLPVLCQDHGLTPDQVVAIASHDGGRPALESIVAQLSRPDPAL

AALTNDHLVALACLGGRPALDAVKKGLPHAPALIKRTNRRIPERTSHRVAGS (SEQ ID
NO: 157)

HBG-B1-6 protein sequence:

MDYKDHDGDYKDHDIDYKDDDDKMAPKKKRKVGIHRGVPMVDLRTL
GYSQQQQEKIKPKVRSTVAQHHEALVGHGFTHAHIVALSQHPAALGTVAVKYQDMIA
ALPEATHEAIVGVGKQWSGARALEALLTVAGELRGPPLQLDTGQLLKIAKRGGVTAVE
AVHAWRNALTGAPLNLTPDQVVAIASHDGGKQALETVQRLLPVLCQDHGLTPDQVVA
IASHDGGKQALETVQRLLPVLCQDHGLTPDQVVAIASNGGGKQALETVQRLLPVLCQD
HGLTPDQVVAIASHDGGKQALETVQRLLPVLCQDHGLTPDQVVAIASNGGGKQALETV
QRLLPVLCQDHGLTPDQVVAIASNGGGKQALETVQRLLPVLCQDHGLTPDQVVAIASN
HGGKQALETVQRLLPVLCQDHGLTPDQVVAIASNHGGKQALETVQRLLPVLCQDHGL
TPDQVVAIASNHGGKQALETVQRLLPVLCQDHGLTPDQVVAIASNHGGKQALETVQRL
LPVLCQDHGLTPDQVVAIASNHGGKQALETVQRLLPVLCQDHGLTPDQVVAIASHDGG
KQALETVQRLLPVLCQDHGLTPDQVVAIASHDGGKQALETVQRLLPVLCQDHGLTPDQ
VVAIASNGGGKQALETVQRLLPVLCQDHGLTPDQVVAIASNGGGKQALETVQRLLPVL
CQDHGLTPDQVVAIASHDGGRPALESIVAQLSRPDPALAALTNDHLVALACLGGRPAL
DAVKKGLPHAPALIKRTNRRIPERTSHRVAGS (SEQ ID NO: 149)

EXAMPLE 1: TALES FOR SPECIFIC REGULATION OF A TARGET GENE

Transcription factors regulate gene expression. Gene expression can be artificially perturbed by expressing dominant negative forms of transcription factors that lack activation/repression domains. However, naturally occurring TFs bind to thousands of gene in the genome, making dominant negative forms of TFs unsuitable for targeting a specific gene. Provided herein are DNA binding proteins that not only displace an endogenous TF but are specific for a target gene. The DNA binding protein replaces endogenous TF from only a target gene since it binds to a sequence present only in the target gene. TALE proteins that specifically bind to a sequence in a target gene were designed and tested.

$\beta$-hemoglobinopathies, such as, sickle cell disease, $\beta$-thalassemia, are caused by mutations in the adult $\beta$-globin gene. During development, globin genes undergo switching: adult $\beta$-globin is expressed and the fetal $\gamma$-globin promoter is directly bound and silenced by the transcription factors ZBTB7A and BCL11A (Bauer, D. et al., Blood 2012). Mutations in the fetal $\gamma$-globin promoter result in incomplete silencing of the fetal $\gamma$-globin promoter (Hereditary Persistence of Fetal Hemoglobin; HPFH). HPFH patients who inherit sickle cell gene (Hb S) show symptomatic amelioration of sickle cell disease. Accordingly, reactivation of fetal globin expression to increase level of functional Hb is avidly being sought as a therapeutic avenue.

Mutations in HPFH patients cluster in TF binding sites. TALE proteins that bind to TF binding sites were designed to test the hypothesis that displacing TFs bound to $\gamma$-globin proximal promoter may reactivate fetal $\gamma$-globin expression.

Two TALEs (A11 and B1) that bind to different nucleotide sequences spanning the binding site for the TF ZBTB7A were designed. A11 and B1 TALEs induced increase in relative expression of HBG as compared to total hemoglobin (adult hemoglobin (HBB)+HBG). FIG. 1, panel A. An extension/truncation series of TALEs based on the A11 and B1 TALEs are also able to de-repress human fetal globin (Table 1 and Figure 1, panel A), providing strong orthogonal proof that this specific site within the fetal globin promoter is the one where the TALEs optimally compete away the binding by the TF ZBTB7A.

FIG. 1, panel B shows that a TALE protein (HBB-90-TLJC4) that binds to a region bound by a transcriptional activator for expression of adult globin gene (HBB) reduces expression of HBB to a small extent. However, when coupled with A11 TALE (which blocks binding by the fetal globin transcriptional repressor ZBTB7A), a synergistic boost in fetal globin expression is observed. In other words, globin switching is coordinately potentiated by blocking adult globin expression, and blocking fetal globin repression.

BCL11A is a transcriptional repressor of fetal globin expression. A TALE that binds to a region of enhancer element for the BCL11A which region is bound by a transcriptional activator of BCL11A gene was designed. This TALE (DRbce) blocks a GATA transcription factor activator site. This results in downregulation of BCL11A and upregulation of fetal globin, the gene which it represses. This is demonstrated in FIG. 2, panel B in CD34+ hematopoietic stem cells.

**EXAMPLE 2: TALES ENCODED USING DIVERSIFIED NUCLEIC ACID SEQUENCES**

Nucleic acid sequences encoding the repeat units of the TALEs were diversified to reduce recombination and elimination of the encoding sequence in vivo. For example, when lentivirus is used to deliver the TALE encoding nucleic acid into cells, some of the nucleic acid sequence can be recombined resulting in decreased expression of the encoded TALE. The diversified repeat (DR) nucleic acid encoding a TALE can be delivered by lentivirus and result in fetal globin upregulation (FIG. 3, panel B) and sustained expression (panel C).

Sequence of DRA11 nucleic acid encoding HBG-A11 TALE:

CTTACGCCTGACCAGGTGGTAGCGATAGCCAGCAACATTGGGGGGAA
ACAAGCACTCGAAACTGTTCAGCGACTGCTCCCTGTTTTGTGCCAAGACCACGGATT

GACTCCCGACCAAGTGGTTGCTATCGCCAGCAACGGAGGGGGTAAGCAGGCACTGG
AAACCGTTCAAAGGCTGTTGCCAGTTCTCTGCCAGGATCATGGATTGACACCGGAC
CAAGTTGTAGCAATCGCCAGCCACGATGGCGGCAAGCAAGCACTGGAAACAGTGC
AAAGATTGCTGCCCGTTCTTTGCCAGGACCACGGTCTTACACCAGACCAAGTAGTG
GCAATCGCGAGTCACGATGGTGGTAAGCAGGCTCTTGAAACTGTACAGCGGCTGTT
GCCTGTGTTGTGTCAAGATCATGGACTTACGCCAGATCAGGTGGTGGCCATCGCGTC
AAATGGTGGGGGAAAGCAAGCGCTCGAAACGGTTCAGAGGCTCCTCCCGGTTCTGT
GTCAGGATCATGGGCTGACACCGGATCAAGTGGTAGCAATTGCCTCACACGACGGG
GGGAAACAGGCGCTTGAAACGGTCCAACGGCTGCTCCCTGTGCTTTGCCAAGATCA
CGGCCTGACCCCGGATCAAGTAGTGGCTATTGCTAGCAATGGTGGGGGTAAGCAAG
CTCTGGAGACtGTGCAGAGACTTCTCCCTGTTCTTTGTCAAGATCACGGACTTACACC
GGACCAGGTAGTTGCAATCGCGAGCAACGGAGGCGGGAAACAAGCTCTCGAAACT
GTACAAAGGCTTCTCCCAGTACTTTGTCAGGATCACGGCcttACGCCCGACCAGGTTG
TAGCCATTGCCAGCAACCACGGGGGGAAGCAAGCCCTCGAAACCGTTCAGAGGCTT
CTGCCTGTGCTTTGTCAGGACCACGGACTCACCCCTGATCAAGTCGTGGCTATCGCC
AGTAACCACGGGGGAAAACAAGCCCTTGAAACGGTGCAAAGACTTCTTCCGGTGCT
GTGTCAGGACCATGGGCTTACGCCAGACCAGGTGGTGGCGATTGCCAGTAATCATG
GCGGTAAGCAGGCGCTCGAAACTGTGCAGCGGCTGCTGCCGGTTCTTTGCCAAGAC
CATGGACTTACCCCCGATCAAGTGGTTGCCATCGCGAGTAATCACGGCGGCAAACA
GGCGCTGGAAACGGTACAACGGCTGTTGCCGGTCCTTTGCCAGGATCACGGGcttACG
CCTGATCAAGTTGTTGCGATCGCCAGCAATCACGGGGGGCAAGCAAGCTCTTGAAAC
GGTTCAAAGACTGCTCCCGGTTTTGTGTCAAGACCATGGTTTGACCCCTGACCAAGT
TGTTGCAATTGCCAGCCACGACGGTGGTAAACAGGCTCTCGAAACAGTCCAAAGGC
TTTTGCCGGTACTCTGTCAAGACCACGGCCTTACTCCGGACCAGGTGGTTGCCATTG
CGAGTCACGACGGGGGCAAACAGGCACTCGAAACGGTCCAGAGACTTTTGCCTGTG
CTCTGCCAAGATCATGGTCTGACTCCTGACCAAGTGGTGGCAATCGCCTCACACGAT
GGTGGGAAGCAGGCCCTCGAAACGGTACAGCGACTGTTGCCCGTATTGTGCCAGGA
CCATGGCCTGACGCCGGACCAGGTTGTGGCCATAGCTAGCCACGATGGAGGA (SEQ
ID NO: 145)

Sequence of C10D4 nucleic acid encoding HBG-A1 1+2 TALE:

CTTACCCCCGATCAAGTCGTGGCTATCGCTAGTaacattGGAGGTAAACA
GGCCCTTGAAACAGTTCAGAGACTTTTGCCAGTACTTTGCCAGGATCATGGCTTGAC
ACCAGACCAGGTCGTCGCAATAGCTTCTaacggtGGGGGTAAGCAGGCTCTGGAAACA
GTGCAGAGGCTCCTCCCCGTGTTGTGTCAGGACCACGGGTTGACCCCGGATCAGGT

GGTCGCGATTGCCAGTcacgatGGTGGAAAACAAGCACTCGAGACTGTTCAGCGCTTG
CTTCCTGTTTTGTGCCAGGATCATGGGCTTACACCCGATCAGGTAGTTGCTATCGCG
TCCcacgatGGAGGTAAGCAGGCCCTTGAGACTGTGCAACGGCTGTTGCCTGTACTCTG
TCAAGACCACGGTCTTACTCCAGATCAGGTCGTTGCAATCGCGAGTaacggtGGTGGCA
AACAAGCCCTGGAAACCGTGCAGCGCCTCCTCCCCGTGCTTTGTCAAGATCACGGC
CTGACACCCGATCAAGTAGTTGCCATCGCGTCTcacgatGGAGGGAAGCAGGCTCTTG
AAACGGTGCAACGCTTGCTGCCGGTCTTGTGTCAGGACCATGGCCTGACCCCTGACC
AAGTCGTAGCTATCGCTTCAaacggtGGAGGGAAACAAGCCCTTGAGACAGTCCAGCG
GCTCCTGCCAGTGCTCTGCCAAGATCATGGCCTCACCCCTGATCAGGTCGTGGCCAT
CGCAAGCaacggtGGTGGTAAACAGGCTCTGGAGACAGTCCAACGGCTCTTGCCAGTT
CTCTGCCAGGACCATGGGCTTACTCCGGACCAGGTGGTTGCCATCGCCTCAaaccatGG
GGGGAAACAGGCATTGGAGACAGTGCAGAGACTCCTGCCCGTGTTGTGCCAGGATC
ACGGACTTACGCCTGACCAGGTAGTGGCAATTGCCTCCaaccatGGCGGAAAACAGGC
TTTGGAGACTGTCCAGAGACTGTTGCCAGTTCTCTGTCAGGACCACGGTCTGACCCC
AGATCAAGTTGTCGCAATCGCAAGTaaccatGGGGGGGAAGCAAGCCCTCGAAACTGTG
CAGCGCCTGCTGCCAGTCCTGTGCCAGGACCACGGATTGACACCAGATCAGGTGGT
AGCCATTGCAAGTaaccatGGCGGGAAACAAGCCCTCGAAACGGTGCAAAGACTCCTT
CCCGTGCTGTGTCAGGATCATGGCCTTACCCCTGATCAGGTGGTTGCTATCGCCTCTa
accatGGCGGTAAGCAGGCACTCGAAACAGTTCAGCGGCTTCTGCCTGTCTTGTGTCA
AGACCATGGCCTCACTCCAGACCAGGTCGTAGCAATTGCCAGCcacgatGGGGGAAAG
CAAGCGCTCGAGACTGTGCAGCGGCTGCTCCCAGTGCTTTGTCAGGACCATGGACT
GACACCTGATCAGGTTGTTGCAATTGCATCTcacgatGGTGGGAAGCAAGCGTTGGAA
ACCGTCCAGCGCCTGCTCCCCGTTCTCTGCCAGGATCATGGATTGACTCCCGACCAA
GTTGTGGCCATCGCCTCCcacgatGGAGGCAAGCAGGCACTTGAAACTGTTCAGCGGCT
GTTGCCCGTTCTTTGTCAGGATCACGGCCTTACTCCGGATCAAGTGGTAGCTATCGC
ATCCcacgatGGGGGGCAAACAAGCTCTCGAAACAGTGCAGCGCTTGCTGCCCGTGCTCT
GTCAAGATCATGGTCTGACACCCGACCAGGTGGTGGCCATAGCGAGCaacggtGGCGG
CAAGCAGGCCCTGGAAACGGTGCAGAGGCTGCTCCCTGTCCTGTGCCAAGACCACG
GCCTCACACCGGATCAGGTCGTGGCTATAGCTAGCaacggtGGAGGA (SEQ ID NO: 146)

Sequence of DRbce nucleic acid encoding BCL11A enhancer (DRbce):

CTTACCCCGGACCAGGTGGTTGCTATCGCCTCCaacggtGGAGGCAAGCA
GGCTCTCGAAACTGTGCAGCGGCTCCTCCCCGTGCTCTGTCAGGACCATGGCTTGAC
ACCCGATCAAGTAGTCGCTATCGCCTCAaaccatGGTGGCAAGCAGGCCTTGGAGACA
GTGCAACGACTGCTGCCAGTTCTGTGTCAGGACCACGGGCTGACCCCAGACCAGGT

CGTCGCCATAGCGTCAcacgatGGAGGTAAACAAGCACTGGAGACTGTCCAGAGGTTG
CTCCCAGTCCTGTGCCAGGACCATGGATTGACTCCCGACCAGGTTGTTGCCATTGCA
TCCaacggtGGTGGGAAACAGGCTCTTGAGACTGTCCAAAGACTGTTGCCGGTGCTGTG
CCAAGACCACGGTCTTACTCCGGATCAGGTCGTTGCGATAGCAAGTaacggtGGGGGGG
AAACAGGCCCTGGAAACTGTGCAGAGGCTGCTGCCCGTTCTCTGTCAAGATCACGG
GCTGACACCAGATCAGGTAGTTGCCATCGCAAGCaacggtGGGGGCAAGCAGGCGCTC
GAAACGGTGCAACGGCTGCTGCCTGTCCTCTGCCAAGACCATGGCCTGACCCCGGA
TCAGGTAGTGGCTATCGCTTCTaacggtGGAGGCAAACAGGCACTGGAAACAGTCCAG
CGGTTGCTGCCCGTGCTTTGCCAAGATCATGGCTTGACGCCTGACCAAGTCGTGGCA
ATTGCTTCCaacattGGCGGAAAGCAGGCCTTGGAAACCGTGCAGCGACTCCTCCCTGT
GCTCTGCCAGGACCATGGCCTTACTCCCGATCAGGTCGTGGCCATTGCCTCAaacggtG
GCGGCAAGCAGGCACTTGAGACAGTTCAGCGCCTGCTGCCTGTTTTGTGCCAGGAT
CACGGGTTGACCCCTGACCAGGTTGTGGCAATAGCCTCTcacgatGGTGGTAAGCAAG
CCCTCGAGACAGTGCAGCGCCTGTTGCCTGTGCTTTGCCAAGACCACGGATTGACGC
CCGATCAGGTGGTTGCCATAGCCAGCaacattGGAGGGAAGCAGGCCCTGGAGACAGT
GCAGCGGCTGCTTCCTGTCTTGTGCCAAGATCACGGCCTCACCCCCGATCAGGTAGT
CGCAATCGCATCTcacgatGGAGGAAAGCAGGCCCTCGAAACGGTGCAGAGGCTTCTG
CCCGTGTTGTGCCAGGATCATGGCCTTACCCCTGATCAGGTCGTCGCTATCGCGTCC
aacattGGGGGTAAACAGGCTCTCGAGACAGTTCAAAGACTCCTTCCTGTTCTGTGTCA
AGATCATGGCCTGACACCTGATCAAGTGGTCGCAATAGCTTCAaaccatGGTGGAAAG
CAGGCACTCGAAACAGTCCAACGGCTTCTGCCCGTACTCTGTCAGGACCACGGACT
GACACCTGATCAGGTTGTGGCGATTGCGAGTaaccatGGAGGTAAGCAGGCACTTGAA
ACTGTCCAGAGACTCCTTCCGGTCCTTTGCCAGGATCACGGTCTGACGCCTGATCAG
GTGGTGGCTATAGCTAGTcacgatGGCGGGAAGCAGGCATTGGAGACAGTACAGCGGC
TGTTGCCCGTATTGTGTCAAGACCACGGCCTGACTCCGGACCAAGTGGTGGCTATAG
CTAGCaacggtGGAGGA (SEQ ID NO: 147).

## EXAMPLE 3: TALES ACTIVATE HBF EXPRESSION IN PRIMARY HUMAN CELLS

FIGS. 4A and 4B demonstrate that lentiviral transduction of the diversified repeat A11 nucleic acid encoding the HBG-A11 TALE results in fetal globin de-repression in HSCs in vitro. Additional alternatively diversified repeat versions of nucleic acid encoding A11+2 TALE displayed a similar effect.

FIGS. 5A-5B show that the lentiviral transduction of the diversified repeat A11 nucleic acid encoding the HBG-A11 TALE can be used to modify human HSCs and that the modified HSCs engraft successfully in mice, persist in vivo and give rise to erythroid cells with high HbF expression.

**FIG. 6.** Stable expression of TALEs binding to the -200 region of the HBF promoter completely reverse the hemoglobin switch, activating fetal hemoglobin (HbF) expression and concomitantly reducing adult beta-globin expression. **FIG. 6A.** The A11 TALE was stably integrated into the AAVS1 locus of HUDEP-2 cells and single cell clones were generated by sorting GFP+ single cells with FACS. **FIG. 6B.** RT-qPCR of HBG and HBB mRNA expression reveals clones with nearly 100% HBG expression out of total HBG and HBB expression, while control clones show no activation of HBG expression. **FIG. 6C.** Flow cytometry of HbF stained cells reveals activation of HbF expression in nearly 100% of cells, demonstrating HbF activation the protein level. **FIG. 6D.** Reverse phase HPLC chromatogram traces of protein lysate from differentiated HUDEP-2 cells. Cells expressing the A11 TALE show strong G-gamma and A-gamma globin expression and almost no beta globin expression. These results are quantified in **FIG. 6E.**

## EXAMPLE 4: TALES PENETRATE CLOSED CHROMATIN TO EXERT EFFECT

**FIG. 7.** TALEs can penetrate closed chromatin to exert effect. Normalized density traces of DNase1 Sequencing and RNA Sequencing in three A11-expressing clones and three WT clones at the globin locus. Black arrows show the location of the TALE recognition sequence, in which chromatin is closed in WT cells. The TALE catalyzes the formation of an active DNase hypersensitive site in A11-expressing clones.

**FIG. 8.** A11 TALE blocks ZBTB7A binding site and potentiates TF binding to nearby GATA site. DNase1 Sequencing cut counts reveal TF footprints where DNase cleavage is blocked by the presence of a bound TF. DNase1-Seq from A11-expressing clones shows a deep footprint of the A11 TALE, reflective of the factor being bound at every allele with an active DNase1 hypersensitive site. The footprint extends past the binding site of the A11 TALE and into the neighboring GATA1 binding site, demonstrating TF binding to this neighboring site.

## EXAMPLE 5: LENTIVIRAL DELIVERED TALES DRIVE STRONG GLOBIN SWITCHING

**FIG. 9.** Lentiviral delivered TALEs drive strong globin switching. Relative expression of HBG out of total globin (HBG + HBB), in HUDEP-2 cells transduced with lentivirus containing TALEs of varying lengths and binding locations (see table 2) with no effector domain and measured by TaqMan qPCR.

Table 2. Designs tested with lentivirus delivery (stable expression):

| Name | Sequence | ID NO |
|---|---|---|
| HBG-200-NEW-4 | TCTTGGGGGCCCCTTCCCCA | 202 |
| HBG-200-NEW-5 | TCTTGGGGGCCCCTTCCCC | 203 |
| HBG-200-NEW-6 | TCTTGGGGGCCCCTTCCC | 204 |
| HBG-200-NEW-7 | TCTTGGGGGCCCCTTCC | 205 |
| HBG-200-NEW-8 | TCTTGGGGGCCCCTTC | 206 |
| HBG-200-NEW-9 | TCTTGGGGGCCCCTT | 207 |
| HBG-A11+1 | TATCCTCTTGGGGGCCCCT | 163 |
| HBG-A11-1 | TATCCTCTTGGGGGCCC | 165 |
| HBG-A11-3 | TATCCTCTTGGGGGC | 167 |
| HBG-B1-1 | TCCTCTTGGGGGCCCCTTCC | 172 |
| HBG-B1-4 | TCCTCTTGGGGGCCCCT | 175 |
| HBG-B1-5 | TCCTCTTGGGGGCCCC | 176 |
| HBG-B1-6 | TCCTCTTGGGGGCCC | 177 |
| HBG2-u200-1 | TTAAGCAGCAGTATCCTCTT | 208 |
| HBG1-u200-3 | TAGCAGTATCCTCTTGG | 209 |
| HBG1-u200-5 | TAGCAGTATCCTCTTGGGG | 210 |

**FIG. 10.** Long-term survival of modified cells and persistence of hemoglobin switching in mice. **FIG. 10A.** Human CD34+ hematopoietic stem cells transduced with DR A11 or control GFP lentivirus were engrafted into NSGW41 mice. Bone marrow (BM) was harvested 16 weeks post-engraftment for BM staining and ex vivo erythroid differentiation. **FIG. 10B.** BM was stained with various lineage markers and assessed by flow cytometry. No significant differences were observed between DR A11 and GFP control treated groups. **FIG. 10C.** HbF staining and flow cytometry on bone marrow revealed an average of 56% HbF+ cells out of all GlyA+ cells compared to 6.7% HbF+ cells in GFP controls. **FIG. 10D.** 1M BM cells from each mouse differentiated ex vivo into terminal red blood cells (RBCs). **FIG. 10E.** HbF staining and flow cytometry of ex vivo differentiated RBCs shows HbF activation in nearly 100% of cells within both nucleated and enucleated populations. These results are quantified in **FIG. 10F. FIG. 10G.** Reverse phase HPLC chromatogram traces of protein lysate from ex vivo cultured terminally differentiated RBCs. Results are quantified in **FIG. 10I. FIG. 10H.** G-gamma and A-gamma globin proportions, determined by RP-HPLC, reflect the proportions observed in fetal cells.

Although the foregoing invention has been described in some detail by way of illustration and example for purposes of clarity of understanding, it is readily apparent to those of ordinary skill in the art in light of the teachings of this invention that certain changes and modifications may be made thereto without departing from the spirit or scope of the appended claims. It is also to be understood that the terminology used herein is for the purpose of describing particular aspects only, and is not intended to be limiting, since the scope of the present invention will be limited only by the appended claims.

Accordingly, the preceding merely illustrates the principles of the invention. It will be appreciated that those skilled in the art will be able to devise various arrangements which, although not explicitly described or shown herein, embody the principles of the invention and are included within its spirit and scope. Furthermore, all examples and conditional language recited herein are principally intended to aid the reader in understanding the principles of the invention and the concepts contributed by the inventors to furthering the art, and are to be construed as being without limitation to such specifically recited examples and conditions. Moreover, all statements herein reciting principles, aspects, and aspects of the invention as well as specific examples thereof, are intended to encompass both structural and functional equivalents thereof. Additionally, it is intended that such equivalents include both currently known equivalents and equivalents developed in the future, i.e., any elements developed that perform the same function, regardless of structure. The scope of the present invention, therefore, is not intended to be limited to the exemplary aspects shown and described herein. Rather, the scope and spirit of present invention is embodied by the appended claims.

The invention is further disclosed in the following clauses:

1. A method for remodeling chromatin architecture in a regulatory region of a gene of interest in a cell, the method comprising:
providing in the cell a DNA-binding polypeptide (DBP) that binds to a sequence in the regulatory region of the gene.

2. The method of clause 1, wherein the remodeling chromatin architecture comprises localized opening of a closed region of the chromatin and increase in expression of the gene.

3. The method of clause 1 or 2, wherein the DBP comprises a plurality of repeat units (RUs) ordered from N-terminus to C-terminus of the DBP to bind to a sequence in the regulatory region of the gene.

4. The method of any one of clauses 1-3, wherein the sequence bound by the DBP is present at a position at least - 100 nucleotides upstream of the transcription start site (TSS) to up to - 400 nucleotides upstream of the TSS.

5. The method of any one of clauses 1-4, wherein the gene of interest is γ-globin gene (*HBGI* and/or *HBG2*) encoding fetal hemoglobin G (HBG).

6. The method of any one of clauses 1-5, wherein the sequence bound by the DBP is present at a position between -350 to -150; -300 to -175; -250 to -150; -230 to -180; or -230 to - 190 with reference to the TSS.

7. The method of any one of clauses 1-6, wherein the sequence bound by the DBP is present between positions -222 to -193 with reference to the TSS in the *HBG1* and/or *HBG2* gene.

8. The method of any one of clauses 1-6, wherein the sequence bound by the DBP is present between positions -227 to -193 with reference to the TSS in the *HBG2* gene.

9. The method of any one of clauses 1-8, wherein the DBP binds to a sub-sequence in the sequence AGCAGTA TCCTCTTGGGGGCCCCTTCCCCA (SEQ ID NO:3) or a complement thereof in the regulatory region of the HBG1 and/or the HBG2 gene, wherein the sub-sequence is at least 9 nucleotides in length.

10. The method of any one of clauses 1-8, wherein the DBP binds to a sub-sequence in the sequence TAAGCA GCAGTATCCTCTTGGGGGCCCCTTCCCCA (SEQ ID NO:8) or a complement thereof in the regulatory region of the HBG2 gene.

11. The method of any one of clauses 1-10, wherein the DBP binds to the sequence TCTT or a complement thereof and to the sequence up to 16 nt present upstream or downstream of the sequence TCTT.

12. The method of any one of clauses 1-10, wherein the DBP binds to the sequence TCTT or a complement thereof and to the sequence up to 16 nt present downstream of the sequence TCTT.

13. The method of any one of clauses 1-10, wherein the DBP binds to the sequence TCTT or a complement thereof and to the sequence up to 15 nt present upstream of the sequence TCTT.

14. The method of any one of clauses 1-13, wherein providing the DBP comprises expressing the DBP in the cell from a nucleic acid integrated into the genome of the cell.

15. The method of any one of clauses 1-13, wherein providing the DBP comprises introducing in the cell a nucleic acid encoding the DBP.

16. The method of any one of clauses 1-13, wherein the nucleic acid encoding the DBP is codon-optimized for expression in the cell.

17. The method of any one of clauses 1-13, wherein providing the DBP comprises introducing the DBP into the cell.

18. The method of any one of clauses 1-17, wherein the cell is a human hematopoietic stem cell.

19. The method of any one of clauses 1-18, wherein the DBP comprises at least 9 RUs, at least 10 RUs, or at least 11 RUs, wherein RUs each comprise the sequence $X_{1-11}X_{12}X_{13}X_{14-33, 34, or 35}$ (SEQ ID NO: 4), the last RU at the C-terminus is a half-repeat comprising the amino acid sequence $X_{1-11}X_{12}X_{13}X_{14-19, 20, or 21}$ (SEQ ID NO: 5), wherein:

$X_{1-11}$ is a chain of 11 contiguous amino acids,
$X_{14-33 or 34 or 35}$ is a chain of 20, 21 or 22 contiguous amino acids,
$X_{14-20 or 21 or 22}$ is a chain of 7, 8 or 9 contiguous amino acids,
$X_{12}X_{13}$ is selected from:

(a) NH, HH, KH, NK, NQ, RH, RN, SS, NN, SN, KN, GN, VN, LN, DN, QN, EN, AN, or FN for binding to guanine (G);
(b) NI, KI, RI, HI, CI, or SI for binding to adenine (A);
(c) NG, HG, KG, RG, VG, IG, EG, MG, YG, AA, EP, VA, or QG for binding to thymine (T);
(d) HD, RD, SD, ND, KD, AD, or YG for binding to cytosine (C);
(e) NV or HN for binding to A or G; and
(f) H* , HA, KA, N* , NA, NC, NS, RA, or S* for binding to A or T or G or C, wherein

(* ) means that the amino acid at $X_{13}$ is absent.

20. The method of any one of clauses 1-19, wherein the RUs of the DBP are arranged from the N-terminus to the C-terminus of DBP to bind to the sequence:

CCTCTTGGGGGCCCCTTCCCCA (SEQ ID NO:183)
CCTCTTGGGGGCCCCTTCCCC (SEQ ID NO:184)
CCTCTTGGGGGCCCCTTCC (SEQ ID NO:185)
CCTCTTGGGGGCCCCTTC (SEQ ID NO:186)
CCTCTTGGGGGCCCCT (SEQ ID NO:187)
CCTCTTGGGGGCCCC (SEQ ID NO:188)
CCTCTTGGGGGCCC (SEQ ID NO:189)
CTTGGGGGCCCCTTCCCCA (SEQ ID NO:25)
CTTGGGGGCCCCTTCCCC (SEQ ID NO:36)
CTTGGGGGCCCCTTCCC (SEQ ID NO:215)
CTTGGGGGCCCCTTCC (SEQ ID NO:216)
CTTGGGGGCCCCTTC (SEQ ID NO:217)
CTTGGGGGCCCCTT (SEQ ID NO:218)
ATCCTCTTGGGGGCCCCT (SEQ ID NO:192)
ATCCTCTTGGGGGCCCCTT (SEQ ID NO:193)
ATCCTCTTGGGGGCCCCTTC (SEQ ID NO:194)
ATCCTCTTGGGGGCCCCTTCC (SEQ ID NO:195)
ATCCTCTTGGGGGCCCCTTCCC (SEQ ID NO:196)
ATCCTCTTGGGGGCCCCTTCCCC (SEQ ID NO:197)
ATCCTCTTGGGGGCCC (SEQ ID NO:191)
ATCCTCTTGGGGGC (SEQ ID NO:199)
CCTCTTGGGGGCCCCTTCC (SEQ ID NO:185)
CCTCTTGGGGGCCCCT (SEQ ID NO:187)
CCTCTTGGGGGCCCC (SEQ ID NO:188)
CCTCTTGGGGGCCC (SEQ ID NO:189)
TAAGCAGCAGTATCCTCTT (SEQ ID NO:219);
AGCAGTATCCTCTTGG (SEQ ID NO:220); or
AGCAGTATCCTCTTGGGG (SEQ ID NO:221).

21. The method of any one of clauses 1-20, wherein the DBP comprises an N-terminus region that binds to T.
22. A recombinant DNA binding polypeptide (DBP) comprising a plurality of repeat units (RUs) ordered from N-terminus to C-terminus of the DBP to bind to a regulatory region of a gene of interest in a cell, wherein binding of the DBP results in remodeling the chromatin architecture from a closed chromatin into an open chromatin architecture, wherein the RUs each comprise the sequence $X_{1-11}X_{12}X_{13}X_{14-33, 34, or 35}$ (SEQ ID NO: 4), the last RU at the C-terminus is a half-repeat comprising the amino acid sequence $X_{1-11}X_{12}X_{13}X_{14-19, 20, or 21}$ (SEQ ID NO: 5), wherein:

$X_{1-11}$ is a chain of 11 contiguous amino acids,

$X_{14-33 \text{ or } 34 \text{ or } 35}$ is a chain of 20, 21 or 22 contiguous amino acids,

$X_{14-20 \text{ or } 21 \text{ or } 22}$ is a chain of 7, 8 or 9 contiguous amino acids,

$X_{12}X_{13}$ is selected from:

(a) NH, HH, KH, NK, NQ, RH, RN, SS, NN, SN, KN, GN, VN, LN, DN, QN, EN, AN, or FN for binding to guanine (G);

(b) NI, KI, RI, HI, CI, or SI for binding to adenine (A);

(c) NG, HG, KG, RG, VG, IG, EG, MG, YG, AA, EP, VA, or QG for binding to thymine (T);

(d) HD, RD, SD, ND, KD, AD, or YG for binding to cytosine (C);

(e) NV or HN for binding to A or G; and

(f) H* , HA, KA, N* , NA, NC, NS, RA, or S* for binding to A or T or G or C, wherein

(* ) means that the amino acid at $X_{13}$ is absent.

23. The recombinant DBP of clause 22, wherein the DBP binds to a sequence present at a position at least - 100 nucleotides upstream of the transcription start site (TSS) to up to - 400 nucleotides upstream of the TSS of the gene.

24. The recombinant DBP of clause 23, wherein the gene of interest is γ-globin gene (*HBG1* and/or *HBG2*) encoding fetal hemoglobin G (HBG) and the sequence bound by the DBP is present at a position between -350 to -150; -300 to -175; -250 to -150; -230 to -180; or -230 to -190 with reference to the TSS.

25. The recombinant DBP of clause 24, wherein the sequence bound by the DBP is present between positions -222 to -193 with reference to the TSS in the *HBG1* and/or *HBG2* gene.

26. The recombinant DBP of clause 24, wherein the sequence bound by the DBP is present between positions -227 to -193 with reference to the TSS in the *HBG2* gene.

27. The recombinant DBP of any one of clauses 22-26, wherein the DBP binds to a sub-sequence in the sequence AGCAGTATCCTCTTGGGGGCCCCTTCCCCA (SEQ ID NO:3) or a complement thereof in the regulatory region of the HBG1 and/or the HBG2 gene, wherein the sub-sequence is at least 9 nucleotides in length.

28. The recombinant DBP of any one of clauses 22-26, wherein the DBP binds to a sub-sequence in the sequence TAAGCAGCAGTATCCTCTTGGGGGCCCCTTCCCCA (SEQ ID NO:8) or a complement thereof in the regulatory region of the HBG2 gene.

29. The recombinant DBP of any one of clauses 22-28, wherein the DBP binds to the sequence TCTT or a complement thereof and to the sequence up to 16 nt present upstream or downstream of the sequence TCTT.

30. The recombinant DBP of any one of clauses 22-28, wherein the DBP binds to the sequence TCTT or a complement thereof and to the sequence up to 16 nt present downstream of the sequence TCTT.

31. The recombinant DBP of any one of clauses 22-28, wherein the DBP binds to the sequence TCTT or a complement thereof and to the sequence up to 15 nt present upstream of the sequence TCTT.

32. The recombinant DBP of any one of clauses 22-31, wherein the RUs of the DBP are arranged from the N-terminus to the C-terminus of DBP to bind to the sequence:

CCTCTTGGGGGCCCCTTCCCCA (SEQ ID NO:183)
CCTCTTGGGGGCCCCTTCCCC (SEQ ID NO:184)
CCTCTTGGGGGCCCCTTCC (SEQ ID NO:185)
CCTCTTGGGGGCCCCTTC (SEQ ID NO:186)
CCTCTTGGGGGCCCCT (SEQ ID NO:187)
CCTCTTGGGGGCCCC (SEQ ID NO:188)
CCTCTTGGGGGCCC (SEQ ID NO:189)
CTTGGGGGCCCCTTCCCCA (SEQ ID NO:25)
CTTGGGGGCCCCTTCCCC (SEQ ID NO:36)
CTTGGGGGCCCCTTCCC (SEQ ID NO:215)
CTTGGGGGCCCCTTCC (SEQ ID NO:216)
CTTGGGGGCCCCTTC (SEQ ID NO:217)
CTTGGGGGCCCCTT (SEQ ID NO:218)
ATCCTCTTGGGGGCCCCT (SEQ ID NO:192)
ATCCTCTTGGGGGCCCCTT (SEQ ID NO:193)
ATCCTCTTGGGGGCCCCTTC (SEQ ID NO:194)
ATCCTCTTGGGGGCCCCTTCC (SEQ ID NO:195)
ATCCTCTTGGGGGCCCCTTCCC (SEQ ID NO:196)
ATCCTCTTGGGGGCCCCTTCCCC (SEQ ID NO:197)

ATCCTCTTGGGGGCCC (SEQ ID NO:191)
ATCCTCTTGGGGGC (SEQ ID NO:199)
CCTCTTGGGGGCCCCTTCC (SEQ ID NO:185)
CCTCTTGGGGGCCCCT (SEQ ID NO:187)
CCTCTTGGGGGCCCC (SEQ ID NO:188)
CCTCTTGGGGGCCC (SEQ ID NO: 189)
TAAGCAGCAGTATCCTCTT (SEQ ID NO:219);
AGCAGTATCCTCTTGG (SEQ ID NO:220); or
AGCAGTATCCTCTTGGGG (SEQ ID NO:221).

33. A recombinant DNA binding polypeptide (DBP) comprising a plurality of repeat units (RUs) ordered from N-terminus to C-terminus of the DBP to bind to the nucleic acid sequence CCTCTTGGGGGC (SEQ ID NO:201) or a complement thereof present in the fetal $\gamma$-globin gene promoter,
wherein eleven of the RUs each comprise the sequence $X_{1-11}X_{12}X_{13}X_{14-33, 34, \text{ or } 35}$ (SEQ ID NO: 4), the last RU at the C-terminus is a half-repeat comprising the amino acid sequence $X_{1-11}X_{12}X_{13}X_{14-19, 20, \text{ or } 21}$ (SEQ ID NO: 5), wherein:

$X_{1-11}$ is a chain of 11 contiguous amino acids,
$X_{14-33 \text{ or } 34 \text{ or } 35}$ is a chain of 20, 21 or 22 contiguous amino acids,
$X_{14-20 \text{ or } 21 \text{ or } 22}$ is a chain of 7, 8 or 9 contiguous amino acids,
$X_{12}X_{13}$ is selected from:

(a) NH, HH, KH, NK, NQ, RH, RN, SS, NN, SN, KN, GN, VN, LN, DN, QN, EN, AN, or FN for binding to guanine (G);
(b) NI, KI, RI, HI, CI, or SI for binding to adenine (A);
(c) NG, HG, KG, RG, VG, IG, EG, MG, YG, AA, EP, VA, or QG for binding to thymine (T);
(d) HD, RD, SD, ND, KD, AD, or YG for binding to cytosine (C);
(e) NV or HN for binding to A or G; and
(f) H*, HA, KA, N*, NA, NC, NS, RA, or S* for binding to A or T or G or C, wherein (*) means that the amino acid at $X_{13}$ is absent.

34. The recombinant DBP of clause 33, wherein the DBP binds to the nucleic acid sequence CCTCTTGGGGGC (SEQ ID NO:201) and wherein $X_{12}X_{13}$ in the 12 RUs from N-terminus to C-terminus are HD, HD, NG, HD, NG, NG, NH, NH, NH, NH, NH, and HD.

35. The recombinant DBP of clause 34, wherein the DBP comprises at least one additional RU at the N-terminus that binds to T such that the DBP binds to the nucleotide sequence: TCCTCTTGGGGGC (SEQ ID NO:200).

36. The recombinant DBP of clause 35, wherein the DBP comprises at least one additional RU at the N-terminus that binds to A such that the DBP binds to the nucleotide sequence: ATCCTCTTGGGGGC (SEQ ID NO:199).

37. The recombinant DBP of clause 36, wherein the $X_{12}X_{13}$ in the 14 RUs from N-terminus to C-terminus are NI, NG, HD, HD, NG, HD, NG, NG, NH, NH, NH, NH, NH, and HD.

38. The recombinant DBP of clause 36 or 37, wherein the DBP comprises at one additional RU at the C-terminus that binds to C such that the DBP binds to the nucleotide sequence: ATCCTCTTGGGGGCC (SEQ ID NO:198), wherein the one additional RU at the C-terminus that binds to C is positioned immediately before the half-repeat unit.

39. The recombinant DBP of clause 38, wherein the $X_{12}X_{13}$ in the RUs from N-terminus to C-terminus are NI, NG, HD, HD, NG, HD, NG, NG, NH, NH, NH, NH, NH, HD, HD, and HD.

40. The recombinant DBP of any one of clauses 33-39, wherein the plurality of RUs are arranged from N-terminus to C-terminus to bind to the nucleotide sequence: ATCCTCTTGGGGGCCC (SEQ ID NO:191); ATCCTCTTGGGGGCCCCT (SEQ ID NO:192); ATCCTCTTGGGGGCCCCTT (SEQ ID NO:193); ATCCTCTTGGGGGCCCCTTC (SEQ ID NO:194); ATCCTCTTGGGGGCCCCTTCC (SEQ ID NO: 195); ATCCTCTTGGGGGCCCCTTCCC (SEQ ID NO:196); or ATCCTCTTGGGGGCCCCTTCCCC (SEQ ID NO:197).

41. The recombinant DBP of any one of clauses 33-40, wherein the plurality of RUs consist of the RUs of any one of clauses 33-40.

42. The recombinant DBP of clause 33 or 34, wherein the DBP comprises at one additional RU at the C-terminus that binds to C such that the DBP binds to the nucleotide sequence: CCTCTTGGGGGCC (SEQ ID NO:190), wherein the one additional RU at the C-terminus that binds to C is positioned immediately before the half-repeat unit.

43. The recombinant DBP of clause 42, wherein the $X_{12}X_{13}$ in the RUs from N-terminus to C-terminus are HD, HD, NG, HD, NG, NG, NH, NH, NH, NH, NH, HD, and HD.

44. The recombinant DBP of clauses 42 or 43, wherein the plurality of RUs are arranged from N-terminus to C-terminus to bind to the nucleotide sequence: CCTCTTGGGGGCCCCTTCCCCAC (SEQ ID NO:182); CCTCTTG

GGGGCCCCTTCCCCA (SEQ ID NO:183); CCTCTTGGGGGCCCCTTCCCC (SEQ ID NO:184); CCTCTTGGGG GCCCCTTCC (SEQ ID NO:185); CCTCTTGGGGGCCCCTTC (SEQ ID NO:186); CCTCTTGGGGGCCCCT (SEQ ID NO:187); CCTCTTGGGGGCCCC (SEQ ID NO:188); or CCTCTTGGGGGCCC (SEQ ID NO:189).

45. The recombinant DBP of any one of clauses 42-44, wherein the plurality of RUs consist of the RUs of any one of clauses 42-44.

46. The recombinant DBP of any one of clauses 22-45, wherein $X_{1-11}$ is at least 80% identical to LTPEQVVAIAS (SEQ ID NO: 6).

47. The recombinant DBP of any one of clauses 22-46, wherein $X_{1-11}$ is at least 80% identical to LTPDQVVAIAS (SEQ ID NO: 11).

48. The recombinant DBP of any one of clauses 22-47, wherein the chain of 20, 21, or 22 contiguous amino acids is at least 80% identical to GGKQALETVQRLLPVLCQDHG (SEQ ID NO: 15).

49. The recombinant DBP of any one of clauses 22-48, wherein the chain of 7, 8 or 9 contiguous amino acids is at least 80% identical to GGRPALE (SEQ ID NO: 7).

50. The recombinant DBP of any one of clauses 22-49, further comprising an N-terminus region present before the first RU at the N-terminus, wherein the N-terminus region binds to T.

51. The recombinant DBP of clause 50, wherein the N-terminus region comprises an amino acid sequence at least 80%, at least 85%, at least 90%, at least 95%, or 100% identical to the amino acid sequence of SEQ ID NO: 150.

52. A recombinant DNA binding protein (DBP) comprising a plurality of repeat units (RUs) ordered from N-terminus to C-terminus of the DBP to bind to the nucleic acid sequence CACCCTGTGGAGCCACAC (SEQ ID NO:181) or a complement thereof present in the adult β-globin (HBB) gene promoter, wherein seventeen of the RUs each comprise the sequence $X_{1-11}X_{12}X_{13}X_{14-33,\,34,\,or\,35}$ (SEQ ID NO: 4), the last RU at the C-terminus is a half-repeat comprising the amino acid sequence $X_{1-11}X_{12}X_{13}X_{14-19,\,20,\,or\,21}$ (SEQ ID NO: 5), wherein:

$X_{1-11}$ is a chain of 11 contiguous amino acids,
$X_{14-33\,or\,34\,or\,35}$ is a chain of 20, 21 or 22 contiguous amino acids,
$X_{14-20\,or\,21\,or\,22}$ is a chain of 7, 8 or 9 contiguous amino acids,
$X_{12}X_{13}$ is selected from:

(a) NH, HH, KH, NK, NQ, RH, RN, SS, NN, SN, KN, GN, VN, LN, DN, QN, EN, AN, or FN for binding to guanine (G);
(b) NI, KI, RI, HI, CI, or SI for binding to adenine (A);
(c) NG, HG, KG, RG, VG, IG, EG, MG, YG, AA, EP, VA, or QG for binding to thymine (T);
(d) HD, RD, SD, ND, KD, AD, or YG for binding to cytosine (C);
(e) NV or HN for binding to A or G; and
(f) H\*, HA, KA, N\*, NA, NC, NS, RA, or S\* for binding to A or T or G or C, wherein (\*) means that the amino acid at $X_{13}$ is absent.

53. The recombinant DBP of clause 52 wherein the DBP binds to the nucleic acid sequence CACCCTGTGGAG CCACAC (SEQ ID NO: 181) and wherein $X_{12}X_{13}$ in the RUs from N-terminus to C-terminus are HD, NI, HD, HD, HD, NG, NH, NG, NH, NH, NI, NH, HD, HD, NI, HD, NI, and HD.

54. The recombinant DBP of clause 52 or 53, wherein the plurality of RUs consist of the RUs of clause 20 or 21.

55. The recombinant DBP of any one of clauses 52-54, wherein $X_{1-11}$ is at least 80% identical to LTPEQVVAIAS (SEQ ID NO: 6).

56. The recombinant DBP of any one of clauses 52-54, wherein $X_{1-11}$ is at least 80% identical to LTPDQVVAIAS (SEQ ID NO: 11).

57. The recombinant DBP of any one of clauses 52-56, wherein the chain of 20, 21, or 22 contiguous amino acids is at least 80% identical to GGKQALETVQRLLPVLCQDHG (SEQ ID NO: 15).

58. The recombinant DBP of any one of clauses 52-56, wherein the chain of 7, 8 or 9 contiguous amino acids is at least 80% identical to GGRPALE (SEQ ID NO: 7).

59. The recombinant DBP of any one of clauses 52-58, further comprising an N-terminus region present before the first RU at the N-terminus, wherein the N-terminus region binds to T.

60. The recombinant DBP of clause 59, wherein the N-terminus region comprises an amino acid sequence at least 80%, at least 85%, at least 90%, at least 95%, or 100% identical to the amino acid sequence of SEQ ID NO: 150.

61. A recombinant DNA binding protein (DBP) comprising a plurality of repeat units (RUs) ordered from N-terminus to C-terminus of the DBP to bind to the nucleic acid sequence TGCTTTTATCACAGGCT (SEQ ID NO:180) or a complement thereof present in the BCL11A gene promoter, wherein sixteen of the RUs each comprise the sequence $X_{1-11}X_{12}X_{13}X_{14-33,\,34,\,or\,35}$ (SEQ ID NO: 4), the last RU at the C-terminus is a half-repeat comprising the amino acid sequence $X_{1-11}X_{12}X_{13}X_{14-19, 20, or 21}$ (SEQ ID NO: 5), wherein:

$X_{1-11}$ is a chain of 11 contiguous amino acids,
$X_{14-33\ or\ 34\ or\ 35}$ is a chain of 20, 21 or 22 contiguous amino acids,
$X_{14-20\ or\ 21\ or\ 22}$ is a chain of 7, 8 or 9 contiguous amino acids,
$X_{12}X_{13}$ is selected from:

(a) NH, HH, KH, NK, NQ, RH, RN, SS, NN, SN, KN, GN, VN, LN, DN, QN, EN, AN, or FN for binding to guanine (G);
(b) NI, KI, RI, HI, CI, or SI for binding to adenine (A);
(c) NG, HG, KG, RG, VG, IG, EG, MG, YG, AA, EP, VA, or QG for binding to thymine (T);
(d) HD, RD, SD, ND, KD, AD, or YG for binding to cytosine (C);
(e) NV or HN for binding to A or G; and
(f) H*, HA, KA, N*, NA, NC, NS, RA, or S* for binding to A or T or G or C, wherein (*) means that the amino acid at $X_{13}$ is absent.

62. The recombinant DBP of clause 61, wherein the DBP binds to the nucleic acid sequence TGCTTTTATCACA GGCT (SEQ ID NO:180) and wherein $X_{12}X_{13}$ in the RUs from N-terminus to C-terminus are NG, NH, HD, NG, NG, NG, NG, NI, NG, HD, NI, HD, NI, NH, NH, HD, and NG.

63. The recombinant DBP of clause 61 or 62, wherein the plurality of RUs consist of the RUs of clause 61 or 62.

64. The recombinant DBP of any one of clauses 61-63, wherein $X_{1-11}$ is at least 80% identical to LTPEQVVAIAS (SEQ ID NO: 6).

65. The recombinant DBP of any one of clauses 61-63, wherein $X_{1-11}$ is at least 80% identical to LTPDQVVAIAS (SEQ ID NO: 11).

66. The recombinant DBP of any one of clauses 61-65, wherein the chain of 20, 21, or 22 contiguous amino acids is at least 80% identical to GGKQALETVQRLLPVLCQDHG (SEQ ID NO: 15).

67. The recombinant DBP of any one of clauses 61-66, wherein the chain of 7, 8 or 9 contiguous amino acids is at least 80% identical to GGRPALE (SEQ ID NO: 7).

68. The recombinant DBP of any one of clauses 61-67, further comprising an N-terminus region present before the first RU at the N-terminus, wherein the N-terminus region binds to T.

69. The recombinant DBP of clause 68, wherein the N-terminus region comprises an amino acid sequence at least 80%, at least 85%, at least 90%, at least 95%, or 100% identical to the amino acid sequence of SEQ ID NO: 150.

70. A nucleic acid encoding the recombinant DBP of any of clauses 22-69.

71. The nucleic acid of clause 70, wherein the nucleic acid is operably linked to a promoter sequence that confers expression of the DBP.

72. The nucleic acid of clause 70 or 71, wherein the sequence of the nucleic acid is codon optimized for expression of the DBP in a human cell.

73. The nucleic acid of any one of clauses 70-72, wherein the nucleic acid is a deoxyribonucleic acid (DNA).

74. The nucleic acid of any one of clauses 70-72, wherein the nucleic acid is a ribonucleic acid (RNA).

75. A vector comprising the nucleic acid of any of clauses 70-73.

76. The vector of clause 75, wherein the vector is a viral vector.

77. The vector of clause 76, wherein the viral vector is a lentiviral vector.

78. A mammalian cell comprising the nucleic acid of any of clauses 70-74 or the vector of any one of clauses 75-77.

79. A mammalian cell that expresses the DBP of any of clauses 22-69.

80. The mammalian cell of clause 78 or 79, wherein the mammalian cell is a human cell.

81. The mammalian cell of any one of clauses 78-80, wherein the mammalian cell is present in a subject.

82. The mammalian cell of any one of clauses 78-80, wherein the cell is an ex vivo cell.

83. The mammalian cell of any one of clauses 78-82, wherein the cell is a cancer cell.

84. The mammalian cell of any one of clauses 78-82 wherein the mammalian cell is a hematopoietic progenitor cell.

85. The mammalian cell of any one of clauses 78-82, wherein the mammalian cell is an erythroid progenitor.

86. The mammalian cell of any one of clauses 78-82, wherein the cell is a pluripotent stem cell.

87. The mammalian cell of clause 86, wherein the cell is an induced pluripotent stem cell.

88. A pharmaceutical composition comprising the DBP of any of clauses 22-69 and a pharmaceutically acceptable excipient.

89. A pharmaceutical composition comprising the nucleic acid of any of clauses 70-73 or the vector of any of one of clauses 75-77 and a pharmaceutically acceptable excipient.

90. A pharmaceutical composition comprising the mammalian cell of any one of clauses 78-87.

91. A method for increasing expression of fetal hemoglobin-$\gamma$ (HBG) in a subject in need thereof, the method comprising administering to the subject the pharmaceutical composition of any one of clauses 88-90.

92. The method of clause 91, wherein the subject has sickle cell anemia.

93. The method of clause 91, wherein the subject has thalassemia.

94. A nucleic acid comprising a sequence of nucleotides having at least 50%, at least 60%, at least 70%, at least 75%, at least 80%, at least 85% identity to the sequence of SEQ ID NO: 145, 146, or 147.

95. A DNA binding protein comprising an amino acid sequence having at least at least 80%, at least 85%, at least 90%, at least 95%, or 100% identical to the amino acid sequence of SEQ ID NO: 148, 149, 156, or 157.

96. A method for modulating expression of a gene in a cell, the method comprising introducing into the cell a DNA binding polypeptide (DBP), or a nucleic acid encoding the DBP, that binds a sequence in regulatory region of a gene bound by a transcription factor (TF), thereby displacing the TF and modulating expression of the gene, wherein the DBP competes with the TF for binding to a regulatory sequence comprising CACC box or GATA site,

> wherein the DBP binds to the sequence CACC or the complement thereof in the CACC box and at least eight, at least nine, at least ten, at least eleven, at least twelve, at least thirteen, or at least fourteen nucleotides 5' and/or 3' of the CACC sequence or the complement thereof, or
> wherein the DBP binds to the sequence GATA or the complement thereof at the GATA site and at least eight, at least nine, at least ten, at least eleven, at least twelve, at least thirteen, or at least fourteen nucleotides 5' and/or 3' of the GATA sequence or the complement thereof.

97. The method of clause 96, wherein the DBP binds to the CACC box present at position -90 upstream of the transcription start site of the human hemoglobin B (HBB) gene.

98. The method of clause 97, wherein the TF is a transcription activator and wherein the binding of the DBP to the CACC box results in decreased expression of HBB protein.

99. The method of any one of clauses 96-98, wherein the DBP is the DBP according to any one of clauses 52-60.

100. The method of clause 99, wherein the DBP binds to the GATA site in + 58 DHS of BCL11A enhancer.

101. The method of clause 96, wherein the TF is a transcriptional activator and binding of the DBP to the GATA site results in reduced expression of BCL11A protein.

102. The method of clause 100 or 101, wherein the DBP comprises a plurality of repeat units (RUs) arranged from N-terminus to C-terminus to bind to the complement of GATA at the GATA site.

103. The method of any one of clauses 100-102, wherein the DBP comprises a plurality of RUs arranged from N-terminus to C-terminus to bind to the sequence TGCTTTTATCACAGGCT (SEQ ID NO:180) at the GATA site.

104. The method of any one of clauses 100-103, wherein the DBP is the DBP according to any one of clauses 29-37.

105. The method of any one of clauses 96-104, further comprising introducing into the cell the DBP or the nucleic acid encoding the DBP.

106. The method of clause 105, wherein the nucleic acid is a deoxyribonucleic acid (DNA).

107. The method of clause 106, wherein the nucleic acid is a ribonucleic acid (RNA).

108. The method of clause 105 or 106, wherein the sequence of the nucleic acid is codon optimized for expression in a human cell.

109. The method of any one of clauses 96-108, wherein the cell is a human cell.

110. The method of any one of clauses 96-109, wherein the cell is a cancer cell.

111. The method of any one of clauses 96-110, wherein the introducing comprises administering the DBP or the nucleic acid encoding the DBP to a subject.

112. The method of clause 111, wherein the administering comprises parenteral administration.

113. The method of clause 111, wherein the administering comprises intravenous, intramuscular, intrathecal, or subcutaneous administration.

### Claims

1. An *ex vivo* method for modulating expression of a BCL11A gene in a cell, the method comprising introducing into the cell a DNA binding polypeptide (DBP), or a nucleic acid encoding the DBP, that binds a sequence in a regulatory region of the BCL11A gene bound by a transcriptional activator (TA), thereby displacing the TA and reducing expression of the BCL11A gene, wherein the DBP:

> competes with the TA for binding to a regulatory sequence comprising the GATA site;
> binds to the sequence GATA or the complement thereof at the GATA site and at least eight, at least nine, at least ten, at least eleven, at least twelve, at least thirteen, or at least fourteen nucleotides 5' and/or 3' of the GATA sequence or the complement thereof; and

comprises a plurality of repeat units (RUs) derived from a TALE protein, wherein the plurality of RUs are ordered from N-terminus to C-terminus of the DBP to bind to the GATA site in + 58 DHS of BCL11A enhancer or to the complement of GATA at the GATA site,

wherein binding of the DBP to the GATA site results in reduced expression of BCL11A protein.

2. The method of claim 1, wherein the plurality of RUs are arranged from N-terminus to C-terminus to bind to the sequence TGCTTTTATCACAGGCT (SEQ ID NO:180) at the GATA site.

3. The method of claim 1 or 2, comprising introducing into the cell the nucleic acid encoding the DBP.

4. A recombinant DNA binding protein (DBP) comprising a plurality of repeat units (RUs) ordered from N-terminus to C-terminus of the DBP to bind to the nucleic acid sequence TGCTTTTATCACAGGCT (SEQ ID NO: 180) or a complement thereof present in the BCL11A gene promoter,

wherein sixteen of the RUs each comprise the sequence $X_{1-11}X_{12}X_{13}X_{14-33, 34, \text{ or } 35}$ (SEQ ID NO: 4), the last RU at the C-terminus is a half-repeat comprising the amino acid sequence $X_{1-11}X_{12}X_{13}X_{14-19, 20, \text{ or } 21}$ (SEQ ID NO: 5), wherein:

$X_{1-11}$ is a chain of 11 contiguous amino acids,

$X_{14-33 \text{ or } 34 \text{ or } 35}$ is a chain of 20, 21 or 22 contiguous amino acids,

$X_{14-20 \text{ or } 21 \text{ or } 22}$ is a chain of 7, 8 or 9 contiguous amino acids,

$X_{12}X_{13}$ is selected from:

(a) NH, HH, KH, NK, NQ, RH, RN, SS, NN, SN, KN, GN, VN, LN, DN, QN, EN, AN, or FN for binding to guanine (G);

(b) NI, KI, RI, HI, CI, or SI for binding to adenine (A);

(c) NG, HG, KG, RG, VG, IG, EG, MG, YG, AA, EP, VA, or QG for binding to thymine (T);

(d) HD, RD, SD, ND, KD, AD, or YG for binding to cytosine (C);

(e) NV or HN for binding to A or G; and

(f) H*, HA, KA, N*, NA, NC, NS, RA, or S* for binding to A or T or G or C, wherein (*) means that the amino acid at $X_{13}$ is absent.

5. The recombinant DBP of claim 4, wherein the DBP binds to the nucleic acid sequence TGCTTTTATCACAGGCT (SEQ ID NO:180) and wherein $X_{12}X_{13}$ in the RUs from N-terminus to C-terminus are NG, NH, HD, NG, NG, NG, NG, NI, NG, HD, NI, HD, NI, NH, NH, HD, and NG.

6. The recombinant DBP of claim 4 or 5, wherein $X_{1-11}$ is at least 80% identical to LTPEQVVAIAS (SEQ ID NO: 6) or LTPDQVVAIAS (SEQ ID NO: 11), wherein the chain of 20, 21, or 22 contiguous amino acids is at least 80% identical to GGKQALETVQRLLPVLCQDHG (SEQ ID NO: 15), and wherein the chain of 7, 8 or 9 contiguous amino acids is at least 80% identical to GGRPALE (SEQ ID NO: 7).

7. The recombinant DBP of any one of claims 4-6, further comprising an N-terminus region present before the first RU at the N-terminus, wherein the N-terminus region binds to T.

8. The recombinant DBP of claim 7, wherein the N-terminus region comprises an amino acid sequence at least 80%, at least 85%, at least 90%, at least 95%, or 100% identical to the amino acid sequence of SEQ ID NO: 150.

9. A nucleic acid encoding the recombinant DBP of any one of claims 4-8.

10. The nucleic acid of claim 9, comprising a sequence of nucleotides having at least 70%, at least 75%, at least 80%, at least 85% identity to the sequence of SEQ ID NO: 147.

11. A vector comprising the nucleic acid of claim 9 or 10, wherein the vector is a viral vector.

12. A mammalian cell comprising the nucleic acid of claim 9 or 10 or the vector of claim 11.

13. The mammalian cell of claim 12, wherein the mammalian cell is a human cell and is a hematopoietic progenitor cell, an erythroid progenitor, a pluripotent stem cell, or an induced pluripotent stem cell.

14. A pharmaceutical composition comprising:

a) the recombinant DBP of any one of claims 4-8 and a pharmaceutically acceptable excipient;
b) the nucleic acid of claim 9 or 10 or the vector of claim 11 and a pharmaceutically acceptable excipient; or
c) the mammalian cell of claim 12 or 13.

15. The pharmaceutical composition of claim 14 for use in a method of treating sickle cell anemia or thalassemia in a subject in need thereof by increasing expression of fetal hemoglobin-$\gamma$ (HBG) in the subject, the method comprising administering the pharmaceutical composition to the subject.

FIG. 1

A  BCL11A enhancer

10 kb ⊢────────────────────────────────⊣ hg38

chr2:60,490,000⌐     60,495,000⌐     60,500,000⌐

DNase1

+58 DHS

AAACCCTTCCTGGAGCCTGT**GATAA**AAGCAACTGTTAGCTTGCACTAG
TTTGGGAAGGACCTCGGACA**CTATT**TTCGTTGACAATCGAACGTGATC

DRbce

B  % HbF+

80
70
60
50
40

DRbce  Control

FIG. 2

FIG. 3

FIG. 4A

FIG. 4B

FIG. 5A

FIG. 5B

FIG. 6A

FIG. 6B

FIG. 6C

FIG. 6D

FIG. 6E

FIG. 7

FIG. 8

FIG. 9

FIG. 10A

FIG. 10B

FIG. 10C

FIG. 10D

FIG. 10E

FIG. 10F

FIG. 10G

FIG. 10H

FIG. 10I

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• US 62175467

**Non-patent literature cited in the description**

• *ALTI-732WO Seq List_ST25.txt*, 15 April 2022